# EUROPEAN PATENT SPECIFICATION

(11) **EP 2 082 045 B1**
(45) Date of publication and mention of the grant of the patent: **18.03.2015**
(21) Application number: 07821516.7
(22) Date of filing: 18.10.2007
(51) Int. Cl.: C12N 15/77

(54) **METHOD OF REDUCING GENE EXPRESSION USING MODIFIED CODON USAGE**
VERFAHREN ZUR REDUZIERUNG DER GENEXPRESSION MITTELS MODIFIZIERTER CODONVERWENDUNG
PROCÉDÉ PERMETTANT DE RÉDUIRE L'EXPRESSION GÉNIQUE PAR UNE UTILISATION DE CODONS MODIFIÉE

(30) Priority: 24.10.2006 EP 06122882
(43) Date of publication of application: 29.07.2009
(73) Proprietor: BASF SE, 67056 Ludwigshafen (DE)
(72) Inventor: HEROLD, Andrea, 68775 Ketsch (DE); KLOPPROGGE, Corinna, 68239 Mannheim (DE); SCHRÖDER, Hartwig, 69226 Nussloch (DE); ZELDER, Oskar, 67346 Speyer (DE); JEONG, Weol Kyu, Gunsan 573-730 (KR)
(86) International application number: PCT/EP2007/061151
(87) International publication number: WO 2008/049781

(56) References cited:
- EP-A- 1 375 664
- KINNAIRD J H ET AL: "AN APPARENT RARE-CODON EFFECT ON THE RATE OF TRANSLATION OF A NEUROSPORA GENE" JOURNAL OF MOLECULAR BIOLOGY, vol. 221, no. 3, 1991, pages 733-736, XP009097410 ISSN: 0022-2836
- MALUMBRES MARCOS ET AL: "Codon preference in corynebacteria" GENE (AMSTERDAM), vol. 134, no. 1, 1993, pages 15-24, XP002473048 ISSN: 0378-1119
- LI ET AL: "Whole genome analysis of non-optimal codon usage in secretory signal sequences of Streptomyces coelicolor" BIOSYSTEMS, NORTH-HOLLAND, AMSTERDAM, NL, vol. 85, no. 3, September 2006 (2006-09), pages 225-230, XP005601721 ISSN: 0303-2647
- EIKMANNS B J ET AL: "CLONING, SEQUENCE ANALYSIS, EXPRESSION, AND INACTIVATION OF THE CORYNEBACTERIUM GLUTAMICUM ICD GENE ENCODING ISOCITRATE DEHYDROGENASE AND BIOCHEMICAL CHARACTERIZATION OF THE ENZYME" JOURNAL OF BACTERIOLOGY, WASHINGTON, DC, US, vol. 177, no. 3, February 1995 (1995-02), pages 774-782, XP002935154 ISSN: 0021-9193
- EGGELING L ET AL: "Improved L-lysine yield with Corynebacterium glutamicum: Use of dapA resulting in increased flux combined with growth limitation" APPLIED MICROBIOLOGY AND BIOTECHNOLOGY, SPRINGER VERLAG, BERLIN, DE, vol. 49, no. 1, January 1998 (1998-01), pages 24-30, XP002248116 ISSN: 0175-7598

## Description

### OBJECT OF THE INVENTION

The present invention is directed to a method of reducing the amount of at least one polypeptide in a *Corynebacterium glutamicum* cell, comprising the step of expressing in a *C*. *glutamicum* cell a modified nucleotide sequence instead of a non-modified nucleotide sequence encoding for said polypeptide having the same amino acid sequence and/or function wherein said modified nucleotide sequence is derived from the non-modified nucleotide sequence such that at least one codon of the non-modified nucleotide sequence is replaced in the modified nucleotide sequence by a less frequently used codon according to the codon usage of *C*. *glutamicum,* wherein said at least one codon is the start codon being replaced by a start codon which is less frequently used in *C*. *glutamicum,* wherein said less frequently used start codon is GTG or TTG.

Further described are nucleotide sequences encoding for a polypeptide with a codon usage that has been adjusted to use codons that are only rarely used according to the codon usage of the host organism.

Also described are the use of such sequences and methods for producing fine chemicals such as amino acids, sugars, lipids, oils, carbohydrates, vitamins, cofactors etc.

### BACKGROUND

In a lot of biotechnological processes it is necessary to modulate gene expression. Thus, for some applications it is necessary to increase the expression of a certain gene product and to thereby increase the amount and/or activity of e.g. a protein in the host cell in which the gene of interest is (over) expressed. Similarly, it may be desirable to reduce the amount of expression of an endogenous gene in a host cell. Furthermore, it may be desireable to fine-tune the expression level of endogenous or heterologous genes.

The fermentative production of so-called fine chemicals is today typically carried out in microorganisms such as *Corynebacterium glutamicum* (C. *glutamicum), Escherichia coli (E.coli), Saccharomyces cerevisiae (S. cerevisiae), Schizzosaccharomycs pombe (S. pombe), Pichia pastoris (P. pastoris), Aspergillus niger, Bacillus subtilis, Ashbya gossypii* or *Gluconobacter oxydans.*

Fine chemicals which include e.g. organic acids such as lactic acid, proteogenic or non-proteogenic amino acids, purine and pyrimidine bases, carbohydrates, aromatic compounds, vitamins and cofactors, lipids, saturated and unsaturated fatty acids are typically used and needed in the pharmaceutical, agriculture, cosmetic as well as food and feed industry.

As regards for example the amino acid methionine, currently worldwide annual production amounts to about 500,000 tons. The current industrial production process is not by fermentation but a multi-step chemical process. Methionine is the first limiting amino acid in livestock of poultry feed and due to this mainly applied as a feed supplement. Various attempts have been published in the prior art to produce methionine e.g. using microorganisms such as *E. coli.*

Other amino acids such as glutamate, lysine, threonine and threonine, are produced by e.g. fermentation methods. For these purposes, certain microorganisms such as *C*. *glutamicum* have been proven to be particularly suited. The production of amino acids by fermentation has the particular advantage that only L-amino acids are produced and that environmentally problematic chemicals such as solvents as they are typically used in chemical synthesis are avoided.

Some of the attempts in the prior art to produce fine chemicals such as amino acids, lipids, vitamins or carbohydrates in microorganisms such as *E*. *coli* and *C*. *glutamicum* have tried to achieve this goal by e.g. increasing the expression of genes involved in the biosynthetic pathways of the respective fine chemicals. If e.g. a certain step in the biosynthetic pathway of an amino acid such as methionine or lysine is known to be rate-limiting, over-expression of the respective enzyme may allow obtaining a microorganism that yields more product of the catalysed reaction and therefore will ultimately lead to an enhanced production of the respective amino acid. Similarly, if a certain enzymatic step in the biosynthetic pathway of an e.g. desired amino acid is known to be non-desirable as it channels a lot of metabolic energy into formation of undesired by-products it may be contemplated to down-regulate expression of the respective enzymatic activity in order to favour only such metabolic reactions that ultimately lead to the formation of the amino acid in question.

Attempts to increase production of e.g. methionine and lysine by up-and/or down regulating the expression of genes being involved in the biosynthetic pathway of methionine or lysine production are e.g. described in WO 02/10209, WO 2006008097, or WO2005059093.

Typically, overexpression of a certain gene in a microorganism such as *E. coli* or *C*. *glutamicum* or other host cells such as *P. pastoris, A. niger* or even mammalian cell culture systems may be achieved by transforming the respective cell with a vector that comprises a nucleotide sequence encoding for the desired protein and which further comprises elements that allow the vector to drive expression of the nucleotide sequence encoding, e.g. for a certain enzyme. Using this approach foreign proteins, i.e. proteins that are encoded by sequences that are not naturally found in the host cell that is used for expression as well as endogenous host cell-specific proteins may be overexpressed. Other typical methods include increasing of copy number of the respective genes in the chromosome, inserting strong promoters for regulating the transcription of the chromosomal copy of the respective genes and enhancing translational initiation by optimization of the ribosomal binding site (RBS).

Kinnaird and Bums, 1991, Journal of Molecular Biology, 221(3), 733-736 discloses a rare-codon effect on the rate of translation of a Neurospora gene. The document describes that, as the result of two mutually compensating frameshift mutations, three successive codons with third-position A were generated in the *Neurospora crassa* am (NADP-specific glutamate dehydrogenase: CDH) gene.

Malumbres et al., 1993, Gene, 134, 15-24 discloses codon preferences in Corynebacteria. In particular, the document describes the analysis of the codon usage (CU) of 34 genes from the closely related species *Brevibacterium lactofermentum* and *Corynebacterium glutamicum* (BLCG) and the comparison with that of 23 genes from other Brevibacterium and Corynebacterium species. The G+C content of the BLCG genes was found to range from 50 to 62%.

Romero and Garcia, 1991, FEMS Microbiology Letters, 84, 325-330 discloses initiation of translation at AUC, AUA and AUU codons in *Escherichia coli.*

Charlet et al., 2005, Molecular Microbiology, 56(5), 1302-1313 discloses a reduced expression of antigenic proteins MPB70 and MPB83 in *Mycobacterium bovis* BCG strains due to a start codon mutation in sigK.

For down-regulating expression of certain factors within e.g. microorganisms, a multitude of technologies such as gene knockout approaches, antisense technology, RNAi technology etc. are available. Some of the technologies for down-regulation of genes lead to a complete loss-of-function for the respective factors due to the absence of e.g. any enzyme being produced. This can be problematic where only a certain degree of reduction in protein amount is the overall goal. In such cases, one may delete the wild type copy of the respective gene and replace it with a mutant version that shows decreased activity or express it from a weak promoter. Such approaches are however rather cumbersome and other means of reducing partially expression of e.g. a protein without changing its amino acid sequence are highly desirable not only for producing fine chemicals in e.g. microorganisms, but also for other purposes.

In view of this situation, it is one object of the present invention to provide methods and nucleotide sequences encoding for polypeptides which can be used to reduce expression of polypeptides in *C*. *glutamicum.* It is a further object to provide such methods and sequences for producing fine chemicals.

These and other objectives as they will become apparent from the ensuing description of the invention are solved by the present invention as described in the independent claims. The dependent claims relate to preferred embodiments.

### SUMMARY OF THE INVENTION

In one embodiment the invention relates to a method of reducing the amount of at least one polypeptide in a *Corynebacterium glutamicum* cell. This method of reducing the amount of a polypeptide comprises the step of expressing in a *C*. *glutamicum* cell a modified nucleotide sequence instead of a non-modified nucleotide sequence encoding for said polypeptide having the same amino acid sequence and/or function wherein said modified nucleotide sequence is derived from the non-modified nucleotide sequence such that at least one codon of the non-modified nucleotide sequence is replaced in the modified nucleotide sequence by a less frequently used codon according to the codon usage of *C*. *glutamicum,* wherein said at least one codon is the start codon being replaced by a start codon which is less frequently used in *C. glutamicum,* wherein said less frequently used start codon is GTG or TTGIn a preferred embodiment the reference codon usage will be determined on the basis of the group of abundant proteins of the *C*. *glutamicum* cell. The nucleotide sequence which will thus have been optimised for reduced expression of a polypeptide may also be designated as modified nucleotide sequence while the endogenous sequence may be designated as starting or non-modified nucleotide sequence.

In a preferred embodiment frequent, very frequent or extremely frequent codons are exchanged for rare, preferably for very rare and most preferably for extremely rare codons. The reference codon usage will be based on the codon usage of *C*. *glutamicum* and preferably on the codon usage of abundant proteins of *C*. *glutamicum.*

This method of reducing the amount of a polypeptide thus comprises the step of expressing in *C*. *glutamicum* a modified nucleotide sequence which encodes for said polypeptide instead of the endogenous nucleotide sequence which encodes for a polypeptide of substantially the same amino acid sequence and/or function. The modified nucleotide sequence is derived from the (non-modified) endogenous nucleotide sequence such that codons of the non-modified nucleotide sequence are exchanged in the modified nucleotide sequence with less frequently used and preferably the least frequently used codons. The reference codon usage will be based in the codon usage of *C*. *glutamicum* and preferably on the codon usage of abundant proteins of *C*. *glutamicum.* In a preferred embodiment frequent, very frequent or extremely frequent codons are thus exchanged for rare, very rare or extremely rare codons. In a particularly preferred embodiment, one, some or all codons are replaced by the least frequently used codons.

In one embodiment at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the codons of the non-modified nucleotide sequences may be replaced in the modified nucleotide sequence by less frequently used codons for the respective amino acid. In an even more preferred embodiment the afore-mentioned number of codons to be replaced refers to frequent, very frequent or extremely frequent codons. In a particularly preferred embodiment these codons are replaced by rare, preferably very rare and most preferably extremely rare codons. In another particularly preferred embodiment, the above numbers of codons are replaced by the least frequently used codons respectively. In all these case is the reference codon usage based in the codon usage of the host organism and preferably on the codon usage of abundant proteins of the *C*. *glutamicum* cell.

In a preferred embodiment one will use modified nucleotide sequence which uses only rare, very rare, extremely rare or preferably the least frequently used codons for each replaced amino acid as determined for the group of abundant proteins of the *C*. *glutamicum* cell.

The polypeptides that are to be expressed by this method of reducing the amount of a polypeptide in a *C*. *glutamicum* cell are endogenous polypeptides with the proviso that the modified nucleotide sequence must not be identical with the starting nucleotide sequences of the respective polypeptides of the host cell.

Using this method, expression of the respective polypeptide may be reduced in the host cell by at least 5%, 10%, 20%, 30%, 40%, 50%, 60%, 70%, 80%, 90% or 95%. The extent of reduction of expression is determined in comparison to the level of expression of the endogenous polypeptide that is expressed from the endogenous non-modified nucleotide sequence under comparable conditions.

A preferred embodiment of this method of reducing the amount of a polypeptide in a host cell relates to methods wherein the amount of a polypeptide being expressed in *C*. *glutamicum* is reduced.

Such a method comprises the step expressing in *C*. *glutamicum* a modified nucleotide sequence encoding for a polypeptide instead of the endogenous nucleotide sequence encoding for the respective polypeptide of the same amino acid sequence and/or function. In one embodiment at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the codons of the non-modified nucleotide sequences may be replaced in the modified nucleotide sequence by less frequently used codons for the respective amino acid. In an even more preferred embodiment the afore-mentioned number of codons to be replaced refers to frequent, very frequent or extremely frequent codons. In a particularly preferred embodiment these codons are replaced by rare, preferably very rare and most preferably extremely rare codons. In another particularly preferred embodiment, the above numbers of codons are replaced by the least frequently used codons respectively. In all these cases will the reference codon usage based on the codon usage of *C*. *glutamicum.* Preferably the reference codon usage will be based on the codon usage of abundant proteins of *C*. *glutamicum.*

For reducing expression of polypeptides in the genus of *C*. *glutamicum* based on altered codon usage, in another embodiment the non-modified nucleotide sequence encoding for the polypeptide may be modified such that at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the codons of the non-modified nucleotide sequence are replaced in the resulting modified nucleotide sequence by less frequently used codons for the respective amino acid according to Table 1 and preferably according to Table 2. In an even more preferred embodiment the afore-mentioned number of codons to be replaced refers to frequent, very frequent and extremely frequent codons. In another particularly preferred embodiment, the above numbers of codons are replaced by one of the two least frequently used codons encoding for the respective amino acid(s) as set forth in Table 1 and preferably in Table 2.

In another embodiment of the invention which relates to a method of decreasing the amount of polypeptide in *C*. *glutamicum,* said modified nucleotide sequence uses the codons ACG for threonine, GAT for aspartic acid, GAA for glutamic acid, AGA and AGG for arginine and/or TTG for the start codon encoding methionine.

In yet another embodiment of the invention which relates to a method of decreasing the amount of polypeptide in *C. glutamicum,* at least one codon of the modified nucleotide sequences may be selected from the codon usage of Table 3 and preferably of Table 4.

Thus, another particularly preferred embodiment of the invention relates to methods of decreasing the amount of a polypeptide in *C*. *glutamicum* wherein at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the codons of the non-modified nucleotide sequence are replaced in the resulting modified nucleotide sequence by the codons for the respective amino acid according to Table 3 and preferably according to Table 4. In an even more preferred embodiment the afore-mentioned number of codons to be replaced refers to frequent, very frequent, extremely frequent or the most frequent codons.

Another particularly preferred embodiment of the invention relates to methods of decreasing the amount of a polypeptide in in *C*. *glutamicum* wherein all codons of the non-modified nucleotide sequence are replaced in the resulting modified nucleotide sequence by the codons for the respective amino acid according to Table 3 and preferably according to Table 4.

The modified nucleotide sequences as they may be used in the above-described methods of reducing the amount of a polypeptide in the host organism *C*. *glutamicum,.*

Such modified nucleotide sequences are derived from the endogenous nucleotide sequences encoding for a polypeptide of substantially the same amino acid sequence and/or function with the codon usage of the modified nucleotide sequence being adjusted such that codons of the non-modified (wild-type) sequences are replaced by less frequently used codons. The reference codon usage will be based on the codon usage of the host organism and preferably on the codon usage of abundant proteins of the host organism *C*. *glutamicum.*

The application further describes modified nucleotide sequences that have been derived for a specific polypeptide by replacing at least one, at least two, at least three, at least four, at least five, at least six, at least seven, at least eight, at least nine, at least ten, at least 1%, at least 2%, at least 4%, at least 6%, at least 8%, at least 10%, at least 20%, at least 40%, at least 60%, at least 80%, e at least 90% or least 95% or all of the codons of the non-modified nucleotide sequences are replaced in the modified nucleotide sequence by less frequently used codons for the respective amino acid. The afore-mentioned number of codons to be replaced refers to frequent, very frequent, extremely frequent or the most frequent codons. The above numbers of codons may be replaced by the least frequently used codons. In all cases will the reference codon usage be based on the codon usage of the host organism *C*. *glutamicum,* and on the codon usage of abundant proteins of the host organism *C*. *glutamicum.*

It is further described that the modified nucleotide sequences will use for each replaced amino acid the least frequently used codon according to the codon usage of the host cell and according to the codon usage of the abundant proteins of the host cell.

In case of modified nucleotide sequences that are to be expressed in *C*. *glutamicum* for reducing the amount of the respective encoded polypeptide, at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the codons of the non-modified nucleotide sequences may be replaced in the modified nucleotide sequence by less frequently used codons for the respective amino acid. In an even more preferred embodiment the afore-mentioned number of codons to be replaced refers to frequent, very frequent, extremely frequent or the most frequent codons. In another particularly preferred embodiment, the above number of codons are replaced by the least frequently used codons. In all these cases will the reference codon usage based on the codon usage of the *C*. *glutamicum* and preferably on the codon usage of abundant proteins of *C*. *glutamicum.*

In case of modified nucleotide sequences that are to be expressed in *C*. *glutamicum* for reducing the amount of the respective encoded polypeptide, at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the codons of the non-modified nucleotide sequence are replaced in the resulting modified nucleotide sequence by less frequently used codons for the respective amino acid according to Table 1 and preferably according to Table 2. In an even more preferred embodiment the afore-mentioned number of codons to be replaced refers to frequent, very frequent, extremely frequent or the most frequent codons. In another particularly preferred embodiment, the afore-mentioned numbers of codons are replaced by one of the two least frequently used codons encoding for the respective amino acid(s) as set forth in Table 1 and preferably in Table 2.

In another embodiment the modified nucleotide sequence which is used for reducing expression of a polypeptide in *C*. *glutamicum* uses the codons ACG for threonine, GAT for aspartic acid, GAA for glutamic acid, AGA and AGG for arginine and/or TTG for the start codon encoding methionine.

In yet another embodiment of the invention the modified nucleotide sequence which is used for reducing expression of a polypeptide in *C*. *glutamicum* may comprise at least one codon being selected from Table 3 and preferably from Table 4.

The application further describes modified nucleotide sequences for reducing expression of a polypeptide in Corynebacterium and particularly preferred in *C*. *glutamicum* wherein at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the codons of the non-modified nucleotide sequence are replaced in the resulting modified nucleotide sequence by the codons for the respective amino acid according to Table 3 and preferably according to Table 4. The afore-mentioned number of codons to be replaced refers to frequent, very frequent, extremely frequent and most frequent codons.

The application further describes modified nucleotide sequences for reducing expression of a polypeptide in *C*. *glutamicum* wherein all codons of the non-modified nucleotide sequence are replaced in the resulting modified nucleotide sequence by the codons for the respective amino acid according to Table 3 and preferably according to Table 4.

In the situation where they are used for reducing expression of polypeptides in *C*. *glutamicum* these modified nucleotide sequences will preferably be selected from the group comprising nucleotide sequences encoding genes of biosynthetic pathways of fine chemicals such as amino acids, sugars, carbohydrates, lipids, oils, vitamins, cofactors etc. if down-regulation of such genes is known to favour production of the fine chemical(s). They may particularly be selected from the group comprising the sequences coding for genes of biosynthetic pathways being involved in the synthesis of amino acids such as glycine, lysine, cysteine, tryptophane or methionine.

A particularly preferred embodiment of the invention relates to a method of decreasing the expression of isocitrate dehydrogenase in a microorganism by adapting the codon usage as described herein. The microorganism can be *C. glutamicum .* These methods may be used to improve synthesis of amino acids and particularly of methionine and/or lysine.

A host cell which comprises a modified nucleotide sequence that will lead to a reduced expression of a polypeptide in the host cell as described above is also described herein. Such a *C*. *glutamicum* cell may comprise a modified nucleotide sequence that will lead to a reduced expression of isocitrate dehydrogenase

Further described herein is the use of the aforementioned modified nucleotide sequences and/or host cells for producing fine chemicals such as amino acids, sugars, lipids, oils, vitamins, cofactors, carbohydrates etc. They may be particularly used for production of amino acids such as glycine, lysine, threonine, cysteine or methionine.

### FIGURES

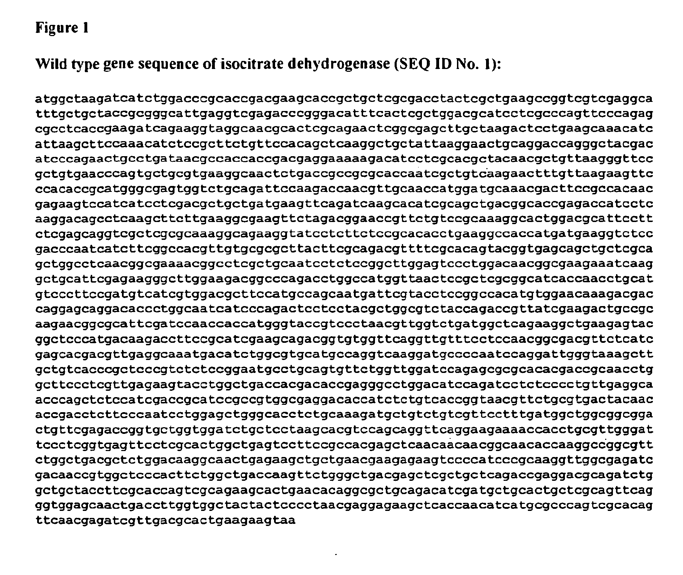

Figure 1 shows the wild type sequence of isocitrate dehydrogenase (SEQ ID No. 1)
Figure 2 a) shows the codon usage amended isocitrate dehydrogenase (icd) carrying an ATG-GTG mutation (SEQ ID No. 2). The mutation is shadowed in grey. Figure 2 b) shows the vector insert that was used to replace the endogenous gene (SEQ ID No. 3). The mutation is shadowed in grey. The restriction sites are underlined.
Figure 3 a) shows the codon usage amended isocitrate dehydrogenase (icd) CA2 (SEQ ID No. 4). The mutation is shadowed in grey. Figure 3 b) shows the vector insert that was used to replace the endogenous gene (SEQ ID No. 5). The mutation is shadowed in grey. The restriction sites are underlined.

### DETAILED DESCRIPTION OF THE INVENTION

As has been set out in the introductory part, it can be desirable in some case to overexpress e.g. foreign genes in a certain host cell as this approach allows to confer novel and unique characteristics to a host cell if e.g. a gene encoding for a certain enzymatic activity is introduced which naturally is not found in the host cell.

However, overexpression of foreign genes having no counterpart in the host all by using e.g. expression vectors such as plasmids has encountered problems. The same has been observed for overexpression of genes which have a counterpart in the host organism as regards their function but which use a nucleotide sequence that is typically not found within the host organism. The failure of host cells such as *E. coli* or *C*. *glutamicum* to express certain foreign (heterologous) sequences may be due to altered codon usage (see e.g. WO 2004/042059).

The genetic code is degenerate which means that a certain amino acid may be encoded by a number of different base triplets. Codon usage refers to the observation that a certain organism will typically not use every possible codon for a certain amino acid with the same frequency. Instead an organism will typically show certain preferences, i.e. a bias for specific codons meaning that these codons are found more frequently in the transcribed genes of an organism.

However, codon usage manipulation has been used in the past for increasing expression of foreign genes. For reducing expression of genes, most commonly a part or the complete coding region has been removed from the genome (so called "knock out" approach). Similarly, mutants with reduced activity or the modulation of transcriptional activity e.g. by weak promoters have been applied in this context.

The present invention relies partly on the surprising finding that, if codons of a nucleotide sequence encoding for an endogenous polypeptide are exchanged against codons which are less frequently found and used in the host cell, one will observe a reduced expression level for this polypeptide due to the amended codon usage.

The use of codons which are less frequently used in the host organism for reducing expression of a polypeptide has numerous advantages. As the modified nucleotide sequence can be integrated into the native genomic locus, the genomic integrity of the organism will be largely preserved. Moreover depending on the number of codons that are exchanged and the frequency of the codons that are introduced it may be possible to fine tune the reduction of the specific factor.

The present invention relies further on the surprising finding that determination of the codon usage of an organism may give different results depending on whether the codon usage is determined for abundant proteins of the host cell or for the host organism as a whole.

Typically, codon usage tables in the prior art for organisms such as *E.coli* etc. have been based on an analysis of the complete genome. The inventors of the present invention have found for the case of *C*. *glutamicum* that codon usage analysis of abundant proteins will give quite different results compared to codon usage frequencies as determined for the complete organism of *C*. *glutamicum.* Without being wanted to be bound to a theory, it is assumed that the specific codon usage frequency of abundant proteins in an organism such as *C*. *glutamicum* reflects certain requirements as to the codon composition of a highly expressed nucleotide sequence.

The specific codon usage distribution of highly expressed genes may e.g. reflect preferences for codons that are recognised by tRNAs that are also frequently and abundantly available in the host organisms' cells. Similarly such codons may reflect transcript RNA structures that for their spatial arrangement can be more efficiently translated.

Identifying codon usage frequencies not on the basis of the whole organism, but for abundant proteins only thus opens the intriguing possibility of defining codons that will likely have a tendency to drive strong expression of a polypeptide. If such frequent codons are specifically selected and replaced by a less frequently used codon as described above, this should additionally help to reduce expression of the respective polypeptide. Moreover, if a codon is used only very rarely in abundant proteins, this may indicate that such a codon is not translated efficiently in the host organism. This presumed inability can be used for a further preferred embodiment of the present invention in which codons are purposively chosen for the modified nucleotide sequence that are only rarely used in abundant proteins of the host cell.

It seems reasonable to assume that the finding that highly expressed proteins in a host cell have a different codon usage compared to the situation where codon usage for all genes of an organism is determined will not be limited to *C*. *glutamicum* but also be observed for other organisms such as *E. coli,* yeast cells, plant cells, insect cells or mammalian cell culture cells.

Thus, the invention is concerned with reducing expression of polypeptides in a *C*. *glutamicum* cell by using modified nucleotide sequences instead of the endogenous sequences wherein codons of the endogenous sequences are exchanged for less frequently used codons of the host organism. This reference codon usage will preferably be determined. In a preferred embodiment codons are exchanged for the least frequently used codons of the host organism. In a further preferred embodiment, one will particularly replace those codons of the endogenous sequences which use codons that are known to be found frequently in the abundant proteins of the host organism.

The term "host cell" or "organism" for the purposes of the present invention refers to *C*. *glutamicum.*

In preferred embodiments of the invention the host cells may be selected from the group comprising *Corynebacterium glutamicum* ATCC13032, , *Corynebacterium glutamicum* KFCC10065 and *Corynebacterium glutamicum* ATCC21608 as well as strains that are derived thereof by e.g. classical mutagenesis and selection or by directed mutagenesis.

Other particularly preferred strains of *C*. *glutamicum* may be selected from the group comprising ATCC13058, ATCC13059, ATCC13060, ATCC21492, ATCC21513, ATCC21526, ATCC21543, ATCC13287, ATCC21851, ATCC21253, ATCC21514, ATCC21516, ATCC21299, ATCC21300, ATCC39684, ATCC21488, ATCC21649, ATCC21650, ATCC19223, ATCC13869, ATCC21157, ATCC21158, ATCC21159, ATCC21355, ATCC31808, ATCC21674, ATCC21562, ATCC21563, ATCC21564, ATCC21565, ATCC21566, ATCC21567, ATCC21568, ATCC21569, ATCC21570, ATCC21571, ATCC21572, ATCC21573, ATCC21579, ATCC19049, ATCC19050, ATCC19051, ATCC19052, ATCC19053, ATCC19054, ATCC19055, ATCC19056, ATCC19057, ATCC19058, ATCC19059, ATCC19060, ATCC19185, ATCC 13286, ATCC21515, ATCC21527, ATCC21544, ATCC21492, NRRL B8183, NRRL W8182, B12NRRLB12416, NRRLB12417, NRRLB12418 and NRRLB11476.

The abbreviation KFCC stands for Korean Federation of Culture Collection, ATCC stands for American-Type Strain Culture Collection and the abbreviation DSM stands for Deutsche Sammlung von Mikroorganismen. The abbreviation NRRL stands for ARS cultures collection Northern Regional Research Laboratory, Peorea, IL, USA.

Particularly preferred are microorganisms of *Corynebacterium glutamicum* that are already capable of producing fine chemicals such as L-lysine, L-methionine and/or L-threonine. Therefore the strain Corynebacterium glutamicum ATCC13032 and derivatives of this strain are particularly preferred.

The term "reducing the amount of at least one polypeptide in a host cell" refers to the situation that if one replaces an endogenous nucleotide sequence coding for a polypeptide with a modified nucleotide sequence in accordance with the invention that encodes for a polypeptide of substantially the same amino acid sequence and/or function, a reduced amount of the encoded polypeptide will be expressed within host cells. This, of course, assumes that the comparison is made for comparable host cell types, comparable genetic background situations etc.

The term "nucleotide sequence" for the purposes of the present invention relates to any nucleic acid molecule that encodes for polypeptides such as peptides, proteins etc. These nucleic acid molecules may be made of DNA, RNA or analogues thereof. However, nucleic acid molecules being made of DNA are preferred.

The terms "non-modified nucleotide sequence" or "starting nucleotide sequence" for the purposes of the present invention will typically relate to an endogenous nucleotide sequence encoding for a polypeptide the expression of which is intended to be reduced. These non-modified or starting nucleotide sequences have not been amended with respect to their codon usage, i.e. no codons have been replaced by less frequently used codons.

The terms "non-modified nucleotide sequence" and "starting nucleotide sequence" do not necessarily have to be equivalent to an endogenous nucleotide sequence. One may for example envisage the situation that an endogenous gene encoding for factor X has been deleted and replaced by a mutated version of factor X carrying a point mutation which leads to a reduced activity of this factor. However, aside from the point mutation the coding sequence of factor X may not have been amended. Starting from such a nucleotide sequence one can still further reduce expression of the mutated factor X by replacing codons of the coding sequence with less frequently used codons. Therefore, the starting nucleotide sequence will not be identical to an endogenous sequence. Nevertheless, the starting nucleotide sequence is characterized in that it has not been amended on the basis of codon usage information to lead to a reduced expression.

Thus, the terms "non-modified" or "starting nucleotide sequence" relate to a nucleotide sequence encoding for an endogenous protein or mutated versions the codon usage of which has not been amended for reducing expression of the encoded polypeptide by replacing codons with less frequently codons as determined for the respective host organism and preferably for the group of abundant proteins thereof.

The term "modified nucleotide sequence" for the purposes of the present invention relates to a sequence that has been modified with the intention to reduce expression of the encoded respective polypeptide in a host cell by adjusting the sequence of the originally different non-modified/starting nucleotide sequence. Thus, codons of the non-modified/starting sequence are replaced by less frequently used codons. The reference codon usage is based on the codon usage of the host organism.

The person skilled in the art is clearly aware that modification of the starting nucleotide sequence describes the process of optimization with respect to codon usage.

If, for example, the coding sequence of an endogenous enzyme is adjusted in order reduce expression of this factor, the changes introduced can be easily identified by comparing the modified sequence and the starting sequence which in such a case is the wild type sequence. Moreover, both sequences will encode in this case for the same amino acid sequence.

If, however, the coding sequence of e.g. an endogenous wild type enzyme is adjusted as described and if the resulting sequence is simultaneously or subsequently further amended by e.g. deleting amino acids, inserting additional amino acids or introducing point mutations in order to convey e.g. new properties to the enzyme (such as reduced feed back inhibition), the resulting modified nucleotide sequence and modified nucleotide sequence may not encode for identical amino acid sequences. In such a situation, no starting sequence in the sense that the starting sequence and the modified sequence encode for the same amino acid sequence may be present simply because the mutation which has been introduced had not been described before. Moreover, the function of the encoded may or may not be affected by the introduced mutations. If for example a point mutation is inserted in a feed back regulated enzyme, the enzyme may still catalyze the respective reaction, but it may be resistant to feed back inhibition. If e.g. amino acids are introduced at the N- or C-terminus, this may have no impact at all on the function. A skilled person will realize that despite the differences between the modified and non-modified nucleotide sequences the inventive method has been used because the starting sequence without the introduced mutation will be known in the form of wild type sequence and the differences of the modified and the starting sequence for those codons which do not code for the introduced mutation will clearly indicate that codon usage optimisation as described above has been carried out. Thus, codon usage optimisation will be clear from a comparison of the starting and the modified sequence for those codons which code for the amino acids at the same or equivalent positions.

This is meant when it is stated in the context of the present invention that the modified and starting nucleotide sequences encode for proteins of substantially identical amino acid sequence and/or function. The modified and starting nucleotide sequence will typically be at least 60%, 65%, preferably at least 70%, 75%, 80%, 85% and more preferably at least 90%, 95 or at least 98% identical as regards the amino acid sequence.

It has been set out above that in a preferred embodiment, one will not only replace codons with less frequently used codons according to the codon usage of the host organism, but also select the codons to be replaced according to certain criteria. The replacement of codons which are frequently used in the host organism may have an additional positive effect because the increased usage frequency indicates the selection of such codons by an organism if expression is to be increased. The replacement of such frequent codons by less frequently used codons and preferably by the least frequently codons for reducing expression thus not only establishes "brakes" in the sequence but also removes elements which usually drive expression. The replacement of codons that are frequently used in abundant or ubiquitous proteins is particularly preferred.

The term "abundant proteins" for the purposes of the present invention relates to the group of highly expressed proteins within a host cell or organism.

The person skilled in the art is familiar with identifying the group of abundant proteins in a host cell or organism. This may be achieved e.g. by 2D gel electrophoresis. In 2D gel electrophoresis, a protein mixture such as a crude cellular extract is separated on protein gels by e.g. size and isoelectric point. Subsequently these gels are stained and the intensity of the various spots is an indication of the overall amount of protein present in the cell.

Using standard software packages one will select a group of proteins whose signal intensities are above a certain threshold background level and will define this group of protein as abundant proteins. Typical software packages used for this purpose include e.g. Melanie3 (Geneva Bioinformatics SA).

The person skilled in the art is well aware that different host cells such as microorganisms, plant cells, insect cells etc. will differ with respect to the number and kind of abundant proteins in a cell. Even within the same organism, different strains may show a somewhat heterogeneous expression profile on the protein level. One will therefore typically analyse different strains and consider such proteins that are found for all strains to be abundant.

A good selection parameter for defining a group of abundant proteins for the purposes of the present invention is to consider only the 10 to 300 and preferably 10 to 30 most abundant proteins as detected in the above described 2D gel electrophoresis procedure. Preferably one will only consider cytosolic proteins for the group of abundant proteins, only.

Thus, in a preferred embodiment the term "abundant proteins" refers to the group of the approximately 13, 14 or 15 abundant proteins in whole cell cytosolic extracts of host organisms as identified by 2D gel electrophoresis.

Once one has identified the abundant proteins, one may use software tools such as the "Cusp" function of the EMBOSS toolbox version 2.2.0 that can be downloaded at HTTP://EMBOSS.sourceforge.net/download/. Other software packages that may be used are available at www.entelechon.com (e.g. Leto I.O).

As has been explained above, the principle of the invention is to reduce the amount at least one polypeptide in a *Corynebacterium glutamicum* cell, comprising the step of expressing in a *C*. *glutamicum* cell a modified nucleotide sequence instead of a non-modified nucleotide sequence encoding for said polypeptide having the same amino acid sequence and/or function wherein said modified nucleotide sequence is derived from the non-modified nucleotide sequence such that at least one codon of the non-modified nucleotide sequence is replaced in the modified nucleotide sequence by a less frequently used codon according to the codon usage of *C*. *glutamicum,* wherein said at least one codon is the start codon being replaced by a start codon which is less frequently used in *C*. *glutamicum,* wherein said less frequently used start codon is GTG or TTG.

Unless otherwise indicated the term "frequent codons" refers to the relative frequency by which a certain codon of all possible codons encoding a specific amino acid is used by the proteins of the host cell and preferably by the abundant proteins of the host cell.

A codon will be considered to be "frequent" if it is used at a relative frequency of more than 40%. It is "very frequent" if it is used at relative frequency of more than 60% and a relative frequency of more than 80% is indicative of an "extremely frequent" codon. Again the relative frequencies are based on the codon usage of proteins of the host cell unless otherwise indicated.

Unless otherwise indicated the term "rare codons" refers to the relative frequency by which a certain codon of all possible codons encoding a specific amino acid is used by the proteins of the host cell.

A codon will be considered to be "rare" if it is used less than 20% for the specific amino acid. A "very rare" codon will be used at a frequency of less than 10% and an "extremely rare" codon will be used at a frequency of less than 5%.

As the amino acids methionine and tryptophane are encoded by one codon only, the respective codon frequency is always 100%. However the amino acid threonine is encoded by four codons, namely ACU, ACC, ACA and ACG. For the whole organism of *C*. *glutamicum* these codons are used at a relative frequency of 20.4 %, 52.9% , 12.5 % and 14.3% (see table 1, experiment 1). In view of the above explanations the codons ACC is thus a frequent codon and the codons ACA and ACG are rare codons.

The amino acid alanine is encoded by four codons, namely GCU, GCC, GCA and GCG. For the whole organism of *C*. *glutamicum* these codons are used at a relative frequency of 23.7 %, 25.4% , 29.3 % and 21.6% (see table 1, experiment 1). However, in the group of abundant proteins, these codons are used at relative frequencies of 46.8%, 9.9%, 35.9% and 7.4% (see table 2, experiment 1) Thus GCU for the group of abundant proteins turns out to be a frequent codon. On the other side GCG is particularly avoided in abundant proteins (see table 2, experiment 1). Thus, one may consider to specifically replace the codon GCU in the starting sequence with GCG in the modified sequence if reduction of expression in *C*. *glutamicum* is the goal.

In some embodiments it may be sufficient and preferred to replace codons with less frequently used codons. In a preferred embodiment the modified nucleotide sequence may use for each of the most frequently used codon the least frequently used codon.

The terms "express", "expressing," "expressed" and "expression" refer to expression of a gene product (e.g., a biosynthetic enzyme of a gene of a pathway) in a host organism. The expression can be done by genetic alteration of the microorganism that is used as a starting organism. In some embodiments, a microorganism can be genetically altered (e.g., genetically engineered) to express a gene product at an increased level relative to that produced by the starting microorganism or in a comparable microorganism which has not been altered. Genetic alteration includes, but is not limited to, altering or modifying regulatory sequences or sites associated with expression of a particular gene (e.g. by adding strong promoters, inducible promoters or multiple promoters or by removing regulatory sequences such that expression is constitutive), modifying the chromosomal location of a particular gene, altering nucleic acid sequences adjacent to a particular gene such as a ribosome binding site or transcription terminator, increasing the copy number of a particular gene, modifying proteins (e.g., regulatory proteins, suppressors, enhancers, transcriptional activators and the like) involved in transcription of a particular gene and/or translation of a particular gene product, or any other conventional means of deregulating expression of a particular gene using routine in the art (including but not limited to use of antisense nucleic acid molecules, for example, to block expression of repressor proteins).

The method of reducing the amount of a polypeptide in accordance with the invention comprises the step of expressing a modified nucleotide sequence which encodes for a polypeptide in *Corynebacterium glutamicum* instead of a starting nucleotide sequence which encodes for a polypeptide of substantially the same amino acid sequence and/or function. The modified nucleotide sequence is derived from the starting nucleotide sequence such that codons of the non-modified nucleotide sequence are exchanged in the modified nucleotide sequence with less frequently used and preferably the least frequently used codons, as defined herein above. In a preferred embodiment frequent and preferably very or extremely frequent codons are thus exchanged for rare and preferably for very rare and extremely rare codons. In a further preferred embodiment the most frequently used codons are replaced by the least frequently used codons. In all these case is the reference codon usage based in the codon usage of *Corynebacterium glutamicum* and preferably on the codon usage of abundant proteins of *Corynebacterium glutamicum.*

In a preferred embodiment one will use a modified nucleotide sequence which uses only rare and preferably very rare and extremely rare codons for each replaced amino acid as determined for the proteins of the host cell. In a further preferred embodiment of this latter aspect of the invention the modified nucleotide sequence uses for each amino acid the least frequently used codon.

In all these cases, the start codon may be a preferred "hot spot" for introducing less frequently used codons, namely GTG instead of ATG and even more preferably TTG.

The polypeptides that are to be expressed by this method of reducing the amount of a polypeptide in a host cell can be endogenous polypeptides with the proviso that the modified nucleotide sequence must not be identical with non-modified nucleotide wild type sequences of the respective endogenous polypeptides of *Corynebacterium glutamicum.*

Using these methods and the afore-mentioned organisms, expression of the respective polypeptide may be reduced in the host cell by at least 5%, 10%, 20%, 30%, 40%, 50%, 60%, 70%, 80%, 90% or 95%. The extent of reduction of expression is determined in comparison to the level of expression of the starting nucleotide sequence under comparable conditions.

It is understood that it is not always desirable to reduce expression as much as possible. In certain cases a repression of e.g. 25% may be sufficient and desirable. The present invention offers the possibility to fine tune repression by e.g. not replacing all codons by the least frequently used codons, but by e.g. introducing only two or three rare codons at selected positions.

Further described are modified nucleotide sequences that can be used to reduce expression of a polypeptide in a host cell.

Such modified nucleotide sequences are derived from the starting nucleotide sequences encoding for a polypeptide of substantially the same amino acid sequence and/or function with the codon usage of the modified nucleotide sequence being adjusted such that codons of the non-modified (wild-type) sequences are replaced by less frequently used codons. The reference codon usage will be based on the codon usage of the host organism and preferably on the codon usage of abundant proteins of the host organism.

Further described are such modified nucleotide sequences that have been derived for a specific polypeptide by replacing at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the codons of the non-modified nucleotide sequences are replaced in the modified nucleotide sequence by less frequently used codons for the respective amino acid.The afore-mentioned number of codons to be replaced refers to frequent, very frequent, extremely frequent or the most frequent codons which are replaced by rare, very rare or extremely rare conditions. The above numbers of codons may be replaced by the least frequently used codons. In all cases will the reference codon usage be based on the codon usage of the host organism and preferably on the codon usage of abundant proteins of the host organism.

The modified nucleotide sequences may use for each replaced amino acid the least frequently used codon as determined for the proteins of the host cell.

Further described are vectors comprising such modified nucleotide sequences. These vectors can be used to express such sequences if for example the endogenous sequences have been deleted on the chromosomal level. The vectors can also be used to replace the endogenous sequences with the modified nucleotide sequences.

Also described are host cells comprising the aforementioned modified nucleotide sequences and/or the aforementioned vectors.

A particularly preferred microorganism for performing the above method of reducing expression is *Corynebacterium glutamicum..*

Further described is the use of host cells which are selected from Coryneform bacteria such as bacteria of the genus Corynebacterium. Particularly preferred are the species *Corynebacterium glutamicum, Corynebacterium acetoglutamicum, Corynebacterium acetoacidophilum, Corynebacterium thermoaminogenes, Corynebacterium callunae, Corynebacterium ammoniagenes, Corynebacterium melassecola* and *Corynebacterium effiziens,* Brevibacteria or the species *Brevibacterium flavum, Brevibacterium lactofermentum* and *Brevibacterium divarecatum.*

Also described are host cells selected from the group comprising *Corynebacterium glutamicum* ATCC13032, *C*. *acetoglutamicum* ATCC15806, *C. acetoacidophilum* ATCC13870, *Corynebacterium thermoaminogenes* FERMBP-1539, *Corynebacterium melassecola* ATCC 17965, *Corynebacterium effiziens* DSM 44547, *Corynebacterium effiziens* DSM 44549, *Brevibacterium flavum* ATCC14067, *Brevibacterium lactoformentum* ATCC13869, *Brevibacterium divarecatum* ATCC 14020, *Corynebacterium glutamicum* KFCC10065 and *Corynebacterium glutamicum* ATCC21608 as well as strains that are derived thereof by e.g. classical mutagenesis and selection or by directed mutagenesis.

Also described is the use of the aforementioned methods, modified nucleotide sequences, vectors and/or host cells to produce fine chemicals. One may use modified nucleotide sequences which are selected from the group comprising nucleotide sequences encoding genes of biosynthetic pathways of fine chemicals for which repression is known to enhance production of the fine chemicals.

The term "fine chemical" is well known to the person skilled in the art and comprises compounds which can be used in different parts of the pharmaceutical industry, agricultural industry as well as in the cosmetics, food and feed industry. Fine chemicals can be the final products or intermediates which are needed for further synthesis steps. Fine chemicals also include monomers for polymer synthesis.

Fine chemicals are defined as all molecules which contain at least two carbon atoms and additionally at least one heteroatom which is not a carbon or hydrogen atom. Preferably fine chemicals relate to molecules that comprise at least two carbon atoms and additionally at least one functional group, such as hydroxy-, amino-, thiol-, carbonyl-, carboxy-, methoxy-, ether-, ester-, amido-, phosphoester-, thioether- or thioester-group.

Fine chemicals thus preferably comprise organic acids such as lactic acid, succinic acid, tartaric acid, itaconic acid etc. Fine chemicals further comprise amino acids, purine and pyrimidine bases, nucleotides, lipids, saturated and unsaturated fatty acids such as arachidonic acid, alcohols, e.g. diols such as propandiol and butandiol, carbohydrates such as hyaluronic acid and trehalose, aromatic compounds such as vanillin, vitamins and cofactors etc.

A particularly preferred group of fine chemicals for the purposes of the present invention are biosynthetic products being selected from the group comprising organic acids, proteins, amino acids, lipids etc. Other particularly preferred fine chemicals are selected from the group of sulphur containing compounds such as thionine, cysteine, homocysteine, cystathionine, glutathione, biotine, thiamine and/or lipoic acid..

The group of most preferred fine chemical products include amino acids among which glycine, lysine, methionine, cysteine and threonine are particularly preferred.

A preferred method in accordance with the present invention relates to a method of reducing the amount of at least one polypeptide in *C*. *glutamicum* wherein the above principles are used. Thus one will express a modified nucleotide sequence instead of the starting nucleotide sequence both of which encode for substantially the same amino acid sequence and/or function wherein at least one codon of the starting nucleotide sequence is replaced in the modified nucleotide sequence by a less frequently used codon. The reference codon usage is determined for *C*. *glutamicum.* Preferably the reference codon usage is determined for the abundant proteins of *C*. *glutamicum.*

Of course, the definitions as provided above for the meaning of the terms "modified nucleotide sequences", "non-modified nucleotide sequences", "rare codons", "frequent codons" etc. apply equally for these preferred embodiments of the invention.

A particularly preferred embodiment of the invention relates to a method of decreasing the expression of isocitrate dehydrogenase in a microorganism by adapting the codon usage as described herein. The microorganism can be *C*. *glutamicum .* These methods may be used to improve synthesis of amino acids and particularly of methionine and/or lysine.

Further described is a host cell, which may be *C*. *glutamicum,* comprising a modified nucleotide sequence that will lead to a reduced expression of isocitrate dehydrogenase

As mentioned above, it can be preferred to replace codons which are frequently found in the group of abundant proteins. The abundant proteins of e.g. *C. glutamicum* can be determined as described above by 2D protein gel electrophoresis. To this purpose, *C. glutamicum* strains may be cultivated under standard conditions. Then, cell extracts may be prepared using common lysis protocols. After lysis, the cell extracts are centrifuged and approximately 25-50 µg are analyzed by standard 2D-PAGE. An example of the approach can be found below in example 1 as well as in the material and methods part of Hansmeier et al. (Proteomics 2006, 6, 233-250)

Following this approach abundant proteins in *C*. *glutamicum* can be identified by either selecting the most abundant 10 to 300 cytosolic proteins or by identifying 10 to 30 cytosolic proteins that are observed to be present in elevated amounts in various strains. These results are assumed to be representative also for the group of abundant proteins in other Corynebacterium species.

For the purposes of the present invention, the term "abundant proteins of *C*. *glutamicum"* can relate to the group comprising the following protein factors (accession number of nucleotide sequence shown in brackets):
Elongation Factor Tu (Genbank accession no: X77034)
Glycerin-aldehyde-3-phosphate-dehydrogonase (Genbank accession no: BX927152, ±, nt. 289401-288397)
Fructose bisphosphate aldolase (Genbank accession no: BX927156, ±, nt. 134992-133958)
Elongation Factor Ts (Genbank accession no: BX927154, ±, nt. 14902-14075)
Hypothetical protein (Genbank accession no: BX927155, ±, nt. 213489-214325)
Enolase (Genbank accession no: BX927150, nt. 338561-339838)
Peptidyl-prolyl-Cis-trans isomerase (Genbank accession no: BX927148, nt. 34330-34902)
Superoxide dismutase (Genbank accession no: AB055218)
Phosphoglycerate dehydrogenase (Genbank accession no: BX927151, nt. 306039-307631)
SSU Rib protein S1P (Genbank accession no: BX927152, ±, nt. 26874-28334)
Triose phosphate-isomerase (Genbank accession no: BX927152, ±, nt. 286884-286105)
Isopropylmalat-synthase (Genbank accession no: X70959)
Butane-2,3-dioldehydrogenase (Genbank accession no: BX927156, nt. 20798-21574)
Fumarate-hydratase (Genbank accession no: BX927151, ±, nt. 18803-17394)

On the basis of these aforementioned fourteen proteins, a codon usage table can be created using the aforementioned "CUSP" function of the EMBOSS toolbox

The above described group of fourteen proteins may particularly be used for determining or for defining the group of abundant proteins in *C. glutamicum* if the *C*. *glutamicum* strain ATCC 13032 and/or derivatives (obtained e.g. by classical mutagenesis and selection or genetic engineering) are used in the 2D-gel electrophoresis analysis.

Using the CUSP function of the EMBOSS toolbox version one can thus create a Codon Usage Table that reflects codon usage of abundant proteins of Corynebacterium in general and preferably of *C*. *glutamicum.*

Surprisingly the codon usage of these abundant proteins differs significantly from the codon usage as determined for the whole genome of *C*. *glutamicum* as becomes clear from a comparison of tables 1 and 2 (see Experiment 1 below). Codon usage of the whole genome of *C*. *glutamicum* can e.g. be determined from strains that are completely sequenced such as strain ATCC13032 and Codon Usage Tables may e.g. generated by the CUSP function of the aforementioned EMBOSS toolbox or are available at e.g. HTTP://www.kazusa.or.jp. Highly comparable results are obtained if one uses the most abundant cytosolic proteins as mentioned in Table 4 of Hansmeier et al. *(vide supra*).

A preferred embodiment of the aspect of the invention that relates to methods of reducing the amount of polypeptides in a host cell derived from the species *Corynebacterium glutamicum* comprises the step of expressing in *C. glutamicum* a modified nucleotide sequence encoding for at least one polypeptide instead of an endogenous non-modified nucleotide sequence encoding for a polypeptide of substantially the same amino acid sequence and/or function wherein said modified nucleotide sequence is derived from the starting nucleotide sequence such that one, some or preferably all of the codons of the starting nucleotide sequences are replaced in the modified nucleotide sequence by less frequently used codons for the respective amino acid. In an even more preferred embodiment the codons to be replaced are frequent, very frequent or extremely frequent codons. In a particularly preferred embodiment these codons are replaced by rare, very rare or extremely rare codons. In another particularly preferred embodiment, the above numbers of codons are replaced by the least frequently used codons, respectively. In all these cases will the reference codon usage be based on the codon usage of *C*. *glutamicum.* Preferably, the reference codon usage is determined for the abundant proteins of *C. glutamicum.*

For reducing expression of polypeptides in the species of *C*. *glutamicum* by optimised codon usage, in another embodiment the non-modified nucleotide sequence encoding for the polypeptide may be modified such that at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the codons of the non-modified nucleotide sequence are replaced in the resulting modified nucleotide sequence by less frequently used codons for the respective amino acid according to Table 1 and preferably according to Table 2.

In yet another embodiment of the invention wherein the method is used to reduce the amount of polypeptides in *C. glutamicum* the codons of the modified nucleotide sequence are selected from one of the two least frequently used codons encoding for the respective amino acid(s) as set forth in Table 1 and preferably in Table 2.

In yet another embodiment of the invention which relates to a method of decreasing the amount of polypeptides in *C*. *glutamicum,* at least one, some or all codons of the modified nucleotide sequences may be selected from the codon usage of Table 3.

In another embodiment of the invention, the method may rely on modified nucleotide sequences which use the codons ACG for threonine, GAT for aspartic acid, GAA for glutamic acid, AGA and AGG for arginine and/or TTG for the start codon.

Another particularly preferred embodiment of the invention relates to methods of decreasing the amount of a polypeptide in *C*. *glutamicum* wherein at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the codons of the non-modified nucleotide sequence are replaced in the resulting modified nucleotide sequence by the codons for the respective amino acid according to Table 3 and preferably according to Table 4. In an even more preferred embodiment the afore-mentioned number of codons to be replaced refers to frequent, very frequent, extremely frequent or the most frequent codons.

Another particularly preferred embodiment of the invention relate to methods of decreasing the amount of a polypeptide in *C*. *glutamicum* wherein all codons of the non-modified nucleotide sequence are replaced in the resulting modified nucleotide sequence by the codons for the respective amino acid according to Table 3 and preferably according to Table 4.

The start codon may be a preferred "hot spot" for introducing less frequently used codons, namely GTG instead of ATG and even more preferably TTG.

The host organism may be selected also from *Corynebacterium glutamicum.* Also preferred are the above-mentioned *C*. *glutamicum strain* and particularly preferred is the strain *Corynebacterium glutamicum* ATCC13032 and all its derivatives. The strains ATCC 13286, ATCC 13287, ATCC 21086, ATCC 21127, ATCC 21128, ATCC 21129, ATCC 21253, ATCC 21299, ATCC 21300, ATCC 21474, ATCC 21475, ATCC 21488, ATCC 21492, ATCC 21513, ATCC 21514, ATCC 21515, ATCC 21516, ATCC 21517, ATCC 21518, ATCC 21528, ATCC 21543, ATCC 21544, ATCC 21649, ATCC 21650, ATCC 21792, ATCC 21793, ATCC 21798, ATCC 21799, ATCC 21800, ATCC 21801, ATCC 700239, ATCC 21529, ATCC 21527, ATCC 31269 and ATCC 21526 which are known to produce lysine can also preferably be used. The other aforementioned strains can also be used.

The extent of reduction of protein expression may be the same as mentioned above.

Further described are (modified) nucleotide sequences as they may be used in the above-described methods of reducing the amount of a polypeptide in *C*. *glutamicum.*

In case of modified nucleotide sequences that are to be expressed in *C*. *glutamicum* for reducing the amount of the respective encoded polypeptide, at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the codons of the non-modified nucleotide sequences may be replaced in the modified nucleotide sequence by less frequently used codons for the respective amino acid. The afore-mentioned number of codons to be replaced may also refer to frequent, very frequent, extremely frequent or the most frequent codons which are replaced by rare, very rare or extremely rare codons. The above number of codons may also be replaced by the least frequently used codons. In all these cases will the reference codon usage be based on the codon usage of *C*. *glutamicum.* Preferably, the reference codon usage is based on the codon usage of abundant proteins of *C*. *glutamicum.*

In case of modified nucleotide sequences that are to be expressed in *C*. *glutamicum* for reducing the amount of the respective encoded polypeptide, at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the codons of the non-modified nucleotide sequence are replaced in the resulting modified nucleotide sequence by less frequently used codons for the respective amino acid according to Table 1 and preferably according to Table 2. The afore-mentioned number of codons to be replaced may also refer to frequent, very frequent, extremely frequent or the most frequent codonsThe afore-mentioned numbers of codons may also be replaced by the least frequently used codons of Table 1 and preferably of Table 2.

As far as nucleotide sequences for reducing expression in *C*. *glutamicum* are concerned, at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the codons of the non-modified nucleotide sequence are replaced in the resulting modified nucleotide sequence by one of the two least frequently used codons for the respective amino acid according to Table 1 and preferably according to Table 2. The afore-mentioned number of codons to be replaced may refer to frequent, very frequent, extremely frequent or the most frequent codons. The estimation of whether a codon is frequent, very frequent or extremely frequent will preferably be based on the codons usage of abundant proteins of *C*. *glutamicum* as depicted in Table 2.

It is further described that the modified nucleotide sequences uses the codons ACG for threonine, GAT for aspartic acid, GAA for glutamic acid, AGA and AGG for arginine and/or TTG for the start codon.

The modified nucleotide sequence which is used for reducing expression of a polypeptide in *C. glutamicum* may comprise at least one, some or all codons being selected from Table 3 and preferably from Table 4.

Further described are modified nucleotide sequences for reducing expression of a polypeptide in *C*. *glutamicum* wherein at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the codons of the non-modified nucleotide sequence are replaced in the resulting modified nucleotide sequence by the codons for the respective amino acid according to Table 3 and preferably according to Table 4. The afore-mentioned number of codons to be replaced refers to frequent, very frequent, extremely frequent and most frequent codons.

Also described are modified nucleotide sequences for reducing expression of a polypeptide in *C*. *glutamicum* wherein all codons of the non-modified nucleotide sequence are replaced in the resulting modified nucleotide sequence by the codons for the respective amino acid according to Table 3 and preferably according to Table 4.

In all these cases the start codon may be a preferred "hot spot" for replacing less frequently used codons, namely GTG instead of ATG and even more preferably TTG. This aspect particularly relates to decreasing the amount and/or activity of isocitrate dehydrogenase e.g. to improve methionine synthesis.

Further described are vectors comprising the aforementioned modified nucleotide sequences which can be used to lower protein expression in Corynebacterium and preferably in *C*. *glutamicum.*

A vector that comprises the aforementioned nucleotide sequences is used to drive expression of a modified nucleotide sequence in *C*. *glutamicum* for increasing the amount of a polypeptide in these host cells. Such vectors may e.g. be plasmid vectors which are autonomously replicable in coryneform bacteria. Exemples are pZ1 (Menkel et al. (1989), Applied and Environmental Microbiology 64: 549-554), pEKEx1 (Eikmanns et al.(1991), Gene 102: 93-98), pHS2-1 (Sonnen et al. (1991), Gene 107: 69-74) These vectors are based on the cryptic plasmids pHM1519, pBL1 oder pGA1. Other vectors are pCLiK5MCS (WO2005059093), or vectors based on pCG4 (US-A 4,489,160) or pNG2 (Serwold-Davis et al. (1990), FEMS Microbiology Letters 66, 119-124) or pAG1 (US-A 5,158,891). Examples for other suitable vectors can be found in the Handbook of Corynebacterium (edited by Eggeling and Bott, ISBN 0-8493-1821-1, 2005).

When optimizing the codon usage, other influencing factors, like the resulting mRNA structure, should also be considered.

Also described are host cells comprising the aforementioned vectors and modified nucleotide sequences which can be used to lower protein expression in *C*. *glutamicum.*

A person skilled in the art is familiar with how to replace e.g. a gene or endogenous nucleotide sequence that encode for a certain polypeptide with a modified nucleotide sequence. This may e.g. be achieved by introduction of a suitable construct (plasmid without origin of replication, linear DNA fragment without origin of replication) by electroporation, chemical transformation, conjugation or other suitable transformation methods. This is followed by e.g. homologous recombination using selectable markers which ensure that only such cells are identified that carry the modified nucleotide sequence instead of the endogenous naturally occurring sequence. Other methods include gene disruption of the endogenous chromosomal locus and expression of the modified sequences from e.g. plasmids. Yet other methods include e.g. transposition. Further information as to vectors and host cells that may be used will be given below.

The above described methods of reducing the amount of a polypeptide in a host cell and particularly for reducing the amount of a polypeptide in *C*. *glutamicum* may be performed with the modified nucleotide sequence being selected from the group comprising nucleotide sequences encoding genes of biosynthetic pathways of fine chemicals if repression of expression of these genes is known to enhance fine chemical production. This is especially useful when the polypeptide is responsible for the synthesis of unwanted by-products/side products. Lowering the amount of a polypeptide such as an enzyme that forms part of a biosynthetic pathway of the aforementioned fine chemicals may allow increasing synthesis of the fine chemicals by e.g. shutting off production of by-products and by channelling metabolic flux into a preferred direction.

Similarly the aforementioned modified nucleotide sequences, vectors and host cells can be used for producing fine chemicals in *C*. *glutamicum.*

Also described is a vector that can be used to replace an endogenous sequence with the modified sequence. One may of course also use a vector that is suitable for expression of such nucleotide sequences in the respective host cell. This may e.g. be done if the modified sequence is expressed from a vector and the endogenous sequence has been silenced before by e.g. gene disruption.

Strains of *Corynebacterium glutamicum* that are already capable of producing fine chemicals such as L-lysine, L-methionine and/or L-threonine are preferred in this context. Such a strain is e.g. *Corynebacterium glutamicum* ATCC13032 and derivatives thereof. The strains ATCC 13286, ATCC 13287, ATCC 21086, ATCC 21127, ATCC 21128, ATCC 21129, ATCC 21253, ATCC 21299, ATCC 21300, ATCC 21474, ATCC 21475, ATCC 21488, ATCC 21492, ATCC 21513, ATCC 21514, ATCC 21515, ATCC 21516, ATCC 21517, ATCC 21518, ATCC 21528, ATCC 21543, ATCC 21544, ATCC 21649, ATCC 21650, ATCC 21792, ATCC 21793, ATCC 21798, ATCC 21799, ATCC 21800, ATCC 21801, ATCC 700239, ATCC 21529, ATCC 21527, ATCC 31269 and ATCC 21526 which are known to produce lysine can also preferably be used. The other aforementioned strains can also be used.

These modified nucleotide sequences may again be preferably selected from the group comprising nucleotide sequences encoding genes of biosynthetic pathways of fine chemicals. The definitions and preferences as to the meaning and desirability of fine chemicals given above equally apply. Thus, the group of most preferred fine chemical products includes amino acids among which glycine, lysine, methionine, cysteine and threonine are particularly preferred.

In a preferred embodiment of the methods of reducing the amount of a polypeptide in *C*. *glutamicum,* the modified nucleotide sequences may thus be selected from the group comprising sequences encoding threonine-dehydratase, homoserine-O-acetyltransferase, O-acetylhomoserine-sulfhydrylase, phosphoenolpyruvate-carboxykinase (pepCK), pyruvate-oxidase (poxB), homoserine-kinase, homoserine-dehydrogenase, threonine-exporter, threonine-efflux, asparaginase, aspartate-decarboxylase and threonine-synthase, citrate synthase, aconitase, isocitrate-dehydrogenase, alpha-ketoglutarate dehydrogenase, succinyl-CoA-synthase, succinate-dehydrogenase, fumarase, malate-quinone oxidoreductase, malate dehydrogenase, pyruvate kinase, malic enzyme, alr (alanine racemase), atr43 (ABC transporter), ccpA1 (catabolite control protein a), ccpA2 (catabolite control protein), chrA (two component response regulator), chrS (histidine kinase), citB (transcriptional regulator), citE (citratlyase E), citE (citrat lyase E), clpC (protease), csp1, ctaF (4. subunit of cytochrom aa3 oxidase), dctA (C4-dicarboxylat transport protein), dctQ sodit (C4-dicarboxylat transport protein), dead (DNA/RNA helicase), def (peptide deformylase), dep33 (multi drug resistance protein B), dep34 (efflux protein), fda (fructose bisphosphate ldolase), gorA (glutathion reductase), gpi/pgi (glucose-6-P-isomerase), hisC2 (histidinol phosphate aminotransferase), hom (homoserin dehydrogenase), lipA (lipoate synthase), lipB (lipoprotein-ligase B), lrp (leucine resonse regulator), luxR (transcriptional regulator), luxS (sensory transduction protein kinase), lysR1 (transcriptional regulator), lysR2 (transcriptional regulator, lysR3 (transcriptional regulator), mdhA (malate dehydrogenase), menE (O-succinylbenzoic acid CoA ligase), mikE17 (transcription factor), mqo (malate-quinon oxodoreductase), mtrA mtrB(sensor protein cpxA, regulatory component of sensory), nadA (quinolinate synthase A), nadC (niocotinate nucleotide pyrophosphase), otsA (trehalose-6-P-synthase), otsB, treY, treZ (trehalose phosphatase, maltooligosyl-trehalose synthase maltooligosyl-trehalose trehalohydrolase), pepC (aminopeptidase I), , pfKA pfkB (1 und 6-phosphofructokinase), poxB (pyruvate oxidase),, pstC2 (membrane bound phosphate transport protein), rplK (PS1-protein), sucC sucD (succinyl CoA synthetase), sugA (sugar transport protein), tmk (thymidylate kinase), zwa2, metK metZ, glyA (serinhydroxymethyltransferase), sdhC sdhA sdhB (succinat DH), smtB (transcriptional regulator), cgl1 (transcriptional regulator), hspR (transcriptional regulator), cgl2 (transcriptional regulator), cebR (transcriptional regulator), cgl3 (transcriptional regulator), gatR (transcriptional regulator), glcR (transcriptional regulator), tcmR (transcriptional regulator), smtB2 (transcriptional regulator), dtxR (transcriptional regulator), degA (transcriptional regulator), galR (transcriptional regulator), tipA2 (transcriptional regulator), malI (transcriptional regulator), cgl4 (transcriptional regulator), arsR (transcriptional regulator), merR (transcriptional regulator), hrcA (transcriptional regulator), glpR2 (transcriptional regulator), lexA (transcriptional regulator), ccpA3 (transcriptional regulator), degA2 (transcriptional regulator) in the case of lysine.

In a further preferred embodiment of the methods of reducing the amount of a polypeptide in *C*. *glutamicum,* the modified nucleotide sequences may thus be selected from the group comprising sequences encoding homoserine-kinase, threonine-dehydratase, threonine-synthase, meso-diaminopimelat D-dehydrogenase, phosphoenolpyruvate-carboxykinase, pyruvat-oxidase, dihydrodipicolinate-synthase, dihydrodipicolinate-reductase, and diaminopicolinate-decarboxylase in the case of methionine. Isocitrate dehydrogenase may be preferred particulalry when production of methionine is concerned.

In a preferred embodiment of the methods of reducing the amount of a polypeptide in *C*. *glutamicum,* the modified nucleotide sequences may thus be selected from the group comprising sequences encoding threonine-dehydratase, homoserine O-acetyltransferase, serine-hydroxymethyltransferase, O-acetylhomoserine-sulfhydrylase, meso-diaminopimelate D-dehydrogenase, phosphoenolpyruvate-carboxykinase, pyruvate-oxidase, dihydrodipicolinate-synthetase, dihydrodipicolinate-reductase, asparaginase, aspartate-decarboxylase, lysin-exporter, acetolactate-synthase, ketol-aid-reductoisomerase, branched chain aminotransferase, coenzym B12-dependent methionine-synthase, coenzym B12-independent methione-synthase, dihydroxy acid dehydratase and diaminopicolinate-decarboxylase in the case of threonine.

In general, the person skilled in the art is familiar with designing constructs such as vectors for driving expression of a polypeptide in microorganisms such as *E. coli* and *C*. *glutamicum.* The person skilled in the art is also well acquainted with culture conditions of microorganisms such as *C*. *glutamicum* and *E. coli* as well as with procedures for harvesting and purifying fine chemicals such as amino acids and particularly lysine, methionine and threonine from the aforementioned microorganisms. Some of these aspects will be set out in further detail below.

The person skilled in the art is also well familiar with techniques that allow to change the original non-modified nucleotide sequence into a modified nucleotide sequence encoding for polypeptides of identical amino acid but with different codon usage. This may e.g. be achieved by polymerase chain reaction based mutagenesis techniques, by commonly known cloning procedures, by chemical synthesis etc. Some of these procedures are set out in the examples.

In the following, it will be described and set out in detail how genetic manipulations in microorgansims such as *E. coli* and particularly *Corynebacterium glutamicum* can be performed.

### Vectors and Host Cells

Described herein are vectors, preferably expression vectors, containing a modified nucleotide sequences as mentioned above. As used herein, the term "vector" refers to a nucleic acid molecule capable of transporting another nucleic acid to which it has been linked.

One type of vector is a "plasmid", which refers to a circular double stranded DNA loop into which additional DNA segments can be ligated. Another type of vector is a viral vector, wherein additional DNA segments can be ligated into the viral genome.

Certain vectors are capable of autonomous replication in a host cell into which they are introduced (e.g., bacterial vectors having a bacterial origin of replication and episomal mammalian vectors). Other vectors (e. g., non-episomal mammalian vectors) are integrated into the genome of a host cell upon introduction into the host cell, and thereby are replicated along with the host genome. Moreover, certain vectors are capable of directing the expression of genes to which they are operatively linked.

Such vectors are referred to herein as "expression vectors".

In general, expression vectors of utility in recombinant DNA techniques are often in the form of plasmids. In the present specification, "plasmid" and "vector" can be used interchangeably as the plasmid is the most commonly used form of vector. Further included are other forms of expression vectors, such as viral vectors (e. g., replication defective retroviruses, adenoviruses and adeno-associated viruses), which serve equivalent functions.

The recombinant expression vectors described herein may comprise a modified nucleic acid as mentioned above in a form suitable for expression of the respective nucleic acid in a host cell, which means that the recombinant expression vectors include one or more regulatory sequences, selected on the basis of the host cells to be used for expression, which is operatively linked to the nucleic acid sequence to be expressed.

Within a recombinant expression vector, "operably linked" is intended to mean that the nucleotide sequence of interest is linked to the regulatory sequence (s) in a manner which allows for expression of the nucleotide sequence (e.g., in an in vitro transcription/translation system or in a host cell when the vector is introduced into the host cell). The term "regulatory sequence" is intended to include promoters, repressor binding sites, activator binding sites, enhancers and other expression control elements (e.g., terminators, polyadenylation signals, or other elements of mRNA secondary structure). Such regulatory sequences are described, for example, in Goeddel; Gene Expression Technology: Methods in Enzymology 185, Academic Press, San Diego, CA (1990). Regulatory sequences include those which direct constitutive expression of a nucleotide sequence in many types of host cell and those which direct expression of the nucleotide sequence only in certain host cells. Preferred regulatory sequences are, for example, promoters such as cos-, tac-, trp-, tet-, trp-, tet-, lpp-, lac-, lpp-lac-, lacIq-, T7-, T5-, T3-, gal-, trc-, ara-, SP6-, amy, SP02, e-Pp- ore PL, SOD, EFTu, EFTs, GroEL, MetZ (last 5 from *C*. *glutamicum*), which are used preferably in bacteria. Additional regulatory sequences are, for example, promoters from yeasts and fungi, such as ADC 1, MFa, AC, P-60, CYC1, GAPDH, TEF, rp28, ADH, promoters from plants such as CaMV/35S, SSU, OCS, lib4, usp, STLS1, B33, nos or ubiquitin-or phaseolin-promoters. It is also possible to use artificial promoters. It will be appreciated by one of ordinary skill in the art that the design of the expression vector can depend on such factors as the choice of the host cell to be transformed, the level of expression of protein desired, etc. The expression vectors described herein can be introduced into host cells to thereby produce proteins or peptides, including fusion proteins or peptides, encoded by the above-mentioned modified nucleotide sequences.

The recombinant expression vectors described herein can be designed for expression of the modified nucleotide sequences as mentioned above in prokaryotic or eukaryotic cells. For example, the modified nucleotide sequences as mentioned above can be expressed in bacterial cells such as *C*. *glutamicum* and *E. coli,* insect cells (using baculovirus expression vectors), yeast and other fungal cells (see Romanos, M. A. et al. (1992), Yeast 8: 423-488; van den Hondel, C. A. M.J. J. et al.(1991) in: More Gene Manipulations in Fungi,J. W.Bennet & L. L. Lasure, eds.,p. 396-428: Academic Press: San Diego; and van den Hondel, C. A. M. J. J. & Punt, P. J.(1991) in: Applied Molecular Genetics of Fungi, Peberdy, J. F. et al., eds., p. 1-28, Cambridge University Press: Cambridge), algae and multicellular plant cells (see Schmidt, R. and Willmitzer, L. (1988) Plant Cell Rep.: 583-586). Suitable host cells are discussed further in Goeddel, Gene Expression Technology : Methods in Enzymology 185, Academic Press, San Diego, CA (1990). Alternatively, the recombinant expression vector can be transcribed and translated in vitro, for example using T7 promoter regulatory sequences and T7 polymerase.

Expression of proteins in prokaryotes is most often carried out with vectors containing constitutive or inducible promoters directing the expression of either fusion or non-fusion proteins.

Fusion vectors add a number of amino acids to a protein encoded therein, usually to the amino terminus of the recombinant protein but also to the C-terminus or fused within suitable regions in the proteins. Such fusion vectors typically serve four purposes: 1) to increase expression of recombinant protein; 2) to increase the solubility of the recombinant protein; and 3) to aid in the purification of the recombinant protein by acting as a ligand in affinity purification 4) to provide a "tag" for later detection of the protein. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase.

Typical fusion expression vectors include pGEX (Pharmacia Biotech Inc; Smith, D. B. and Johnson, K. S. (1988) Gene 67: 31-40), pMAL (New England Biolabs, Beverly, MA) and pRIT5 (Pharmacia, Piscataway, NJ) which fuse glutathione S-transferase (GST), maltose E binding protein, or protein A, respectively.

Examples of suitable inducible non-fusion *E. coli* expression vectors include pTrc (Amann et al., (1988) Gene 69: 301-315), pLG338, pACYC184, pBR322,pUC18, pUC19, pKC30, pRep4,pHSl, pHS2, pPLc236, pMBL24, pLG200, pUR290,pIN-III113-Bl, egtll, pBdCl, and pET lld (Studier etal., Gene Expression Technology : Methods in Enzymology 185, Academic Press, San Diego, California (1990) 60-89; and Pouwels et al., eds. (1985) Cloning Vectors. Elsevier: New York IBSN 0 444 904018). Target gene expression from the pTrc vector relies on host RNA polymerase transcription from a hybrid trp-lac fusion promoter. Target gene expression from the pET lld vector relies on transcription from a T7 gnlO-lac fusion promoter mediated by a coexpressed viral RNA polymerase (T7gnl). This viral polymerase is supplied by host strains BL21 (DE3) or HMS174 (DE3) from a resident X prophage harboring a T7gnl gene under the transcriptional control of the lacUV 5 promoter. For transformation of other varieties of bacteria, appropriate vectors may be selected. For example, the plasmids pIJ101, pIJ364, pIJ702 and pIJ361 are known to be useful in transforming Streptomyces, while plasmidspUB110, pC194 or pBD214 are suited for transformation of Bacillus species. Several plasmids of use in the transfer of genetic information into Corynebacterium include pHM1519, pBL1, pSA77 or pAJ667 (Pouwels et al., eds. (1985) Cloning Vectors. Elsevier: New York IBSN 0 444 904018).

Examples of suitable *C*. *glutamicum* and *E coli* shuttle vectors are e.g. pClik5aMCS (WO2005059093) or can be found in Eikmanns et al (Gene. (1991) 102, 93-8).

Examples for suitable vectors to manipulate Corynebacteria can be found in the Handbook of Corynebacterium (edited by Eggeling and Bott, ISBN 0-8493-1821-1, 2005). One can find a list of *E. coli - C*. *glutamicum* shuttle vectors (table 23.1), a list of *E*. *coli - C*. *glutamicum* shuttle expression vectors (table 23.2), a list of vectors which can be used for the integration of DNA into the *C*. *glutamicum* chromosome (table 23.3), a list of expression vectors for integration into the *C*. *glutamicum* chromosome (table 23.4.) as well as a list of vectors for site-specific integration into the *C*. *glutamicum* chromosome (table 23.6).

The protein expression vector described herein may be a yeast expression vector. Examples of vectors for expression in yeast S. cerevisiae include pYepSecl (Baldari, et al., (1987) Embo J. 6: 229-234)" 2i, pAG-1, Yep6, Yep13, pEMBLYe23, pMFa (Kurjan and Herskowitz, (1982) Cell 30: 933-943), pJRY88 (Schultz et al., (1987) Gene 54: 113-123), and pYES2 (Invitrogen Corporation, San Diego, CA). Vectors and methods for the construction of vectors appropriate for use in other fungi, such as the filamentous fungi, include those detailed in: van den Hondel, C. A. M. J. J. & Punt,P. J. (1991) in: Applied Molecular Genetics of Fungi, J. F. Peberdy, et al., eds., p. 1-28, Cambridge University Press: Cambridge, and Pouwels et al., eds. (1985) Cloning Vectors. Elsevier: New York (IBSN 0 444 904018).

For the purposes of the present invention, an operative link is understood to be the sequential arrangement of promoter, coding sequence, terminator and, optionally, further regulatory elements in such a way that each of the regulatory elements can fulfill its function, according to its determination, when expressing the coding sequence.

The modified nucleotide sequences as mentioned above may be expressed in unicellular plant cells (such as algae) or in plant cells from higher plants (e. g., the spermatophytes, such as crop plants). Examples of plant expression vectors include those detailed in: Becker, D., Kemper, E., Schell, J. and Masterson, R. (1992) Plant Mol. Biol. 20: 1195-1197; and Bevan, M. W. (1984) Nucl. Acid. Res. 12: 8711-8721, and include pLGV23, pGHlac+, pBIN19, pAK2004, and pDH51 (Pouwels et al., eds. (1985) Cloning Vectors. Elsevier: New York IBSN 0 444 904018).

For other suitable expression systems for both prokaryotic and eukaryotic cells see chapters 16 and 17 of Sambrook, J. et al. Molecular Cloning: A Laboratory Manual. 3rd ed., Cold Spring Harbor Laboratory, Cold Spring Harbor Laboratory Press, Cold Spring Harbor, NY, 2003.

The recombinant mammalian expression vector may be capable of directing expression of the nucleic acid preferentially in a particular cell type, e.g. in plant cells (e. g., tissue-specific regulatory elements are used to express the nucleic acid). Tissue-specific regulatory elements are known in the art.

Further described are organisms or host cells into which a recombinant expression vector as mentioned herein has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

Vector DNA can be introduced into prokaryotic or eukaryotic cells via conventional transformation or transfection techniques. As used herein, the terms "transformation" and "transfection", "conjugation" and "transduction" are intended to refer to a variety of art-recognized techniques for introducing foreign nucleic acid (e.g., linear DNA or RNA (e. g., a linearized vector or a gene construct alone without a vector) or nucleic acid in the form of a vector (e.g., a plasmid, phage, phasmid, phagemid, transposon or other DNA) into a host cell, including calcium phosphate or calcium chloride co-precipitation, DEAE-dextran-mediated transfection, lipofection, natural competence, conjugation chemical-mediated transfer, or electroporation. Suitable methods for transforming or transfecting host cells can be found in Sambrook, et al. (Molecular Cloning : A Laboratory Manual. 3rd ed., Cold Spring Harbor Laboratory, Cold Spring Harbor Laboratory Press, Cold Spring Harbor, NY, 2003), and other laboratory manuals.

In order to identify and select these integrants, a gene that encodes a selectable marker (e.g., resistance to antibiotics) is generally introduced into the host cells along with the gene of interest. Preferred selectable markers include those which confer resistance to drugs, such as G418, hygromycin , kanamycine, tratracycleine, ampicillin and methotrexate. Nucleic acid encoding a selectable marker can be introduced into a host cell on the same vector as that encoding the above-mentioned modified nucleotide sequences or can be introduced on a separate vector. Cells stably transfected with the introduced nucleic acid can be identified by drug selection (e. g., cells that have incorporated the selectable marker gene will survive, while the other cells die).

When plasmids without an origin of replication and two different marker genes are used (e.g. pClik int sacB), it is also possible to generate marker-free strains which have part of the insert inserted into the genome. This is achieved by two consecutive events of homologous recombination (see also Becker et al., APPLIED AND ENVIRONMENTAL MICROBIOLOGY, 71 (12), p. 8587-8596). The sequence of plasmid pClik int sacB can be found in WO2005059093; SEQ ID 24; the plasmid is called pCIS in this document).

Recombinant microorganisms can be produced which contain selected systems which allow for regulated expression of the introduced gene. For example, inclusion of one of the above-mentioned optimized nucleotide sequences on a vector placing it under control of the lac operon permits expression of the gene only in the presence of IPTG. Such regulatory systems are well known in the art.

In one embodiment, the method comprises culturing the organisms as described herein (into which a recombinant expression vector or into which genome has been introduced a gene comprising the modified nucleotide sequences as mentioned above) in a suitable medium for fine chemical production. In another embodiment, the method further comprises isolating the fine chemical from the medium or the host cell.

### Growth of Escherichia coli and Corynebacterium glutamicum-Media and Culture Conditions

The person skilled in the art is familiar with the cultivation of common microorganisms such as *C.glutamicum* and *E.coli.* Thus, a general teaching will be given below as to the cultivation of *C.glutamicum.* Corresponding information may be retrieved from standard textbooks for cultivation of *E.coli.*

*E. coli* strains are routinely grown in MB and LB broth, respectively (Follettie et al. (1993) J. Bacteriol. 175, 4096-4103). Minimal media for E. coli is M9 and modified MCGC (Yoshihama et al. (1985) J. Bacteriol. 162,591-507), respectively. Glucose may be added at a final concentration of 1%. Antibiotics may be added in the following amounts (micrograms per millilitre): ampicillin, 50; kanamycin, 25; nalidixic acid, 25. Amino acids, vitamins, and other supplements may be added in the following amounts: methionine, 9.3 mM; arginine, 9.3 mM; histidine, 9.3 mM; thiamine, 0.05 mM. *E. coli* cells are routinely grown at 37 C, respectively.

Genetically modified *Corynebacteria* are typically cultured in synthetic or natural growth media. A number of different growth media for *Corynebacteria* are both well-known and readily available (Lieb et al. (1989) Appl.Microbiol. Biotechnol., 32: 205-210; von der Osten et al. (1998) Biotechnology Letters, 11: 11-16; Patent DE 4,120,867; Liebl(1992) "The Genus Corynebacterium, in: The Procaryotes, Volume II, Balows, A. et al., eds. Springer-Verlag). Instructions can also be found in the Handbook of Corynebacterium (edited by Eggeling and Bott, ISBN 0-8493-1821-1, 2005).

These media consist of one or more carbon sources, nitrogen sources, inorganic salts, vitamins and trace elements. Preferred carbon sources are sugars, such as mono-, di-, or polysaccharides. For example, glucose, fructose, mannose, galactose, ribose, sorbose, ribose, lactose, maltose, sucrose, glycerol, raffinose, starch or cellulose serve as very good carbon sources.

It is also possible to supply sugar to the media via complex compounds such as molasses or other by-products from sugar refinement. It can also be advantageous to supply mixtures of different carbon sources. Other possible carbon sources are alcohols and organic acids, such as methanol, ethanol, acetic acid or lactic acid. Nitrogen sources are usually organic or inorganic nitrogen compounds, or materials which contain these compounds. Exemplary nitrogen sources include ammonia gas or ammonia salts, such as NH₄Cl or (NH₄)₂S0₄, NH₄0H, nitrates, urea, amino acids or complex nitrogen sources like corn steep liquor, soy bean flour, soy bean protein, yeast extract, meat extract and others.

The overproduction of methionine is possible using different sulfur sources. Sulfates, thiosulfates, sulfites and also more reduced sulfur sources like H₂S and sulfides and derivatives can be used. Also organic sulfur sources like methyl mercaptan, thioglycolates, thiocyanates, and thiourea, sulfur containing amino acids like cysteine and other sulfur containing compounds can be used to achieve efficient methionine production. Formate may also be possible as a supplement as are other C1 sources such as methanol or formaldehyde.

Inorganic salt compounds which may be included in the media include the chloride-, phosphorous-or sulfate-salts of calcium, magnesium, sodium, cobalt, molybdenum, potassium, manganese, zinc, copper and iron. Chelating compounds can be added to the medium to keep the metal ions in solution. Particularly useful chelating compounds include dihydroxyphenols, like catechol or protocatechuate, or organic acids, such as citric acid. It is typical for the media to also contain other growth factors, such as vitamins or growth promoters, examples of which include biotin, riboflavin, thiamine, folic acid, nicotinic acid, pantothenate and pyridoxine. Growth factors and salts frequently originate from complex media components such as yeast extract, molasses, corn steep liquor and others. The exact composition of the media compounds depends strongly on the immediate experiment and is individually decided for each specific case. Information about media optimization is available in the textbook "Applied Microbiol. Physiology, A Practical Approach (Eds. P. M. Rhodes, P.F. Stanbury, IRL Press (1997) pp. 53-73, ISBN 0 19 963577 3). It is also possible to select growth media from commercial suppliers, like standard 1 (Merck) or BHI (grain heart infusion, DIFCO) or others.

All medium components should be sterilized, either by heat (20 minutes at 1.5 bar and 121 C) or by sterile filtration. The components can either be sterilized together or, if necessary, separately.

All media components may be present at the beginning of growth, or they can optionally be added continuously or batch wise. Culture conditions are defined separately for each experiment.

The temperature should be in a range between 15°C and 45°C. The temperature can be kept constant or can be altered during the experiment. The pH of the medium may be in the range of 5 to 8.5, preferably around 7.0, and can be maintained by the addition of buffers to the media. An exemplary buffer for this purpose is a potassium phosphate buffer. Synthetic buffers such as MOPS, HEPES, ACES and others can alternatively or simultaneously be used. It is also possible to maintain a constant culture pH through the addition of NaOH or NH₄OH during growth. If complex medium components such as yeast extract are utilized, the necessity for additional buffers may be reduced, due to the fact that many complex compounds have high buffer capacities. If a fermentor is utilized for culturing the microorganisms, the pH can also be controlled using gaseous ammonia.

The incubation time is usually in a range from several hours to several days. This time is selected in order to permit the maximal amount of product to accumulate in the broth. The disclosed growth experiments can be carried out in a variety of vessels, such as microtiter plates, glass tubes, glass flasks or glass or metal fermentors of different sizes. For screening a large number of clones, the microorganisms should be cultured in microtiter plates, glass tubes or shake flasks, either with or without baffles. Preferably 100 ml shake flasks are used, filled with 10% (by volume) of the required growth medium. The flasks should be shaken on a rotary shaker (amplitude 25 mm) using a speed-range of 100-300'rpm. Evaporation losses can be diminished by the maintenance of a humid atmosphere; alternatively, a mathematical correction for evaporation losses should be performed.

If genetically modified clones are tested, an unmodified control clone or a control clone containing the basic plasmid without any insert should also be tested. The medium is inoculated to an OD600 of 0.5-1.5 using cells grown on agar plates, such as CM plates (10g/l glucose, 2,5g/l NaCl, 2g/l urea, 10g/l polypeptone, 5g/l yeast extract, 5g/l meat extract, 22g/l NaCl, 2g/l urea, 10g/l polypeptone, 5g/l yeast extract, 5g/l meat extract, 22g/l agar, pH 6.8 with 2M NaOH) that had been incubated at30 C.
Inoculation of the media is accomplished by either introduction of a saline suspension of C. *glutamicum* cells from CM plates or addition of a liquid preculture of this bacterium.

### Quantification of amino acids and methionine intermediates

The analysis is done by HPLC (Agilent 1100, Agilent, Waldbronn, Germany) with a guard cartridge and a Synergi 4µm column (MAX-RP 80 Å, 150 * 4.6 mm) (Phenomenex, Aschaffenburg, Germany). Prior to injection the analytes are derivatized using o-phthaldialdehyde (OPA) and mercaptoethanol as reducing agent (2-MCE). Additionally sulfhydryl groups are blocked with iodoacetic acid. Separation is carried out at a flow rate of 1 ml/min using 40 mM NaH₂PO₄ (eluent A, pH=7.8, adjusted with NaOH) as polar and a methanol water mixture (100 / 1) as non-polar phase (eluent B). The following gradient is applied: Start 0% B; 39 min 39 % B; 70 min 64 % B; 100 % B for 3.5 min; 2 min 0 % B for equilibration. Derivatization at room temperature is automated as described below. Initially 0.5 µl of 0.5% 2-MCE in bicine (0.5M, pH 8.5) are mixed with 0.5 µl cell extract. Subsequently 1.5 µl of 50 mg/ml iodoacetic acid in bicine (0.5M, pH 8.5) are added, followed by addition of 2.5 µl bicine buffer (0.5M, pH 8.5). Derivatization is done by adding 0.5 µl of 10mg/ml OPA reagent dissolved in 1/45/54 v/v/v of 2-MCE/MeOH/bicine (0.5M, pH 8.5). Finally the mixture is diluted with 32 µl H₂O. Between each of the above pipetting steps there is a waiting time of 1 min. A total volume of 37.5 µl is then injected onto the column. Note, that the analytical results can be significantly improved, if the auto sampler needle is periodically cleaned during (e.g. within waiting time) and after sample preparation. Detection is performed by a fluorescence detector (340 nm excitation, emission 450 nm, Agilent, Waldbronn, Germany). For quantification α-amino butyric acid (ABA) is used as internal standard

### Definition of recombination protocol

In the following it will be described how a strain of *C*. *glutamicum* with increased efficiency of methionine production can be constructed implementing the findings of the above predictions. Before the construction of the strain is described, a definition of a recombination event/protocol is given that will be used in the following.

"Campbell in," as used herein, refers to a transformant of an original host cell in which an entire circular double stranded DNA molecule (for example a plasmid being based on pCLIK int sacB has integrated into a chromosome by a single homologous recombination event (a cross-in event), and that effectively results in the insertion of a linearized version of said circular DNA molecule into a first DNA sequence of the chromosome that is homologous to a first DNA sequence of the said circular DNA molecule. "Campbelled in" refers to the linearized DNA sequence that has been integrated into the chromosome of a "Campbell in" transformant. A "Campbell in" contains a duplication of the first homologous DNA sequence, each copy of which includes and surrounds a copy of the homologous recombination crossover point. The name comes from Professor Alan Campbell, who first proposed this kind of recombination.

"Campbell out," as used herein, refers to a cell descending from a "Campbell in" transformant, in which a second homologous recombination event (a cross out event) has occurred between a second DNA sequence that is contained on the linearized inserted DNA of the "Campbelled in" DNA, and a second DNA sequence of chromosomal origin, which is homologous to the second DNA sequence of said linearized insert, the second recombination event resulting in the deletion (jettisoning) of a portion of the integrated DNA sequence, but, importantly, also resulting in a portion (this can be as little as a single base) of the integrated Campbelled in DNA remaining in the chromosome, such that compared to the original host cell, the "Campbell out" cell contains one or more intentional changes in the chromosome (for example, a single base substitution, multiple base substitutions, insertion of a heterologous gene or DNA sequence, insertion of an additional copy or copies of a homologous gene or a modified homologous gene, or insertion of a DNA sequence comprising more than one of these aforementioned examples listed above).

A "Campbell out" cell or strain is usually, but not necessarily, obtained by a counter-selection against a gene that is contained in a portion (the portion that is desired to be jettisoned) of the "Campbelled in" DNA sequence, for example the *Bacillus subtilis sacB* gene, which is lethal when expressed in a cell that is grown in the presence of about 5% to 10% sucrose. Either with or without a counter-selection, a desired "Campbell out" cell can be obtained or identified by screening for the desired cell, using any screenable phenotype, such as, but not limited to, colony morphology, colony color, presence or absence of antibiotic resistance, presence or absence of a given DNA sequence by polymerase chain reaction, presence or absence of an auxotrophy, presence or absence of an enzyme, colony nucleic acid hybridization, antibody screening, *etc.* The term "Campbell in" and "Campbell out" can also be used as verbs in various tenses to refer to the method or process described above.

It is understood that the homologous recombination events that leads to a "Campbell in" or "Campbell out" can occur over a range of DNA bases within the homologous DNA sequence, and since the homologous sequences will be identical to each other for at least part of this range, it is not usually possible to specify exactly where the crossover event occurred. In other words, it is not possible to specify precisely which sequence was originally from the inserted DNA, and which was originally from the chromosomal DNA. Moreover, the first homologous DNA sequence and the second homologous DNA sequence are usually separated by a region of partial non-homology, and it is this region of non-homology that remains deposited in a chromosome of the "Campbell out" cell.

For practicality, in *C*. *glutamicum,* typical first and second homologous DNA sequence are at least about 200 base pairs in length, and can be up to several thousand base pairs in length, however, the procedure can be made to work with shorter or longer sequences. For example, a length for the first and second homologous sequences can range from about 500 to 2000 bases, and the obtaining of a "Campbell out" from a "Campbell in" is facilitated by arranging the first and second homologous sequences to be approximately the same length, preferably with a difference of less than 200 base pairs and most preferably with the shorter of the two being at least 70% of the length of the longer in base pairs. The "Campbell In and -Out-method" is described in WO2007012078

The invention will now be illustrated by means of various examples.

### EXAMPLES

In the following it will be shown how the codon usage of abundant proteins in *C*. *glutamicum* was identified. Furthermore, examples are presented which show that usage of modified nucleotide sequences which have been optimized with regard to either the codon usage of abundant proteins or the organism of *C*. *glutamicum* can be used to either increase or reduce the amount of a protein in *C*. *glutamicum.* This is shown for foreign genes as well as endogenous genes.

### 1. Identification of abundant proteins in C. glutamicum

Cellular extracts were prepared from the *C. glutamicum* strain ATCC 13032 and of some derivatives. For this purpose, 250 mg of cell grown under standard conditions were pelleted and suspended in 750 µl lysis buffer (20 mM TRIS, 5 mM EDTA, pH 7.5) containing a protease inhibitor mix (Complete, Roche). Cell disruption was carried out at 4 °C in a mixer mill (Retsch, MM 2000) using 0.25 - 0.5 mm glass beads. Cell debris was removed by centrifugation at 22.000 rpm for 1 hour at 4 °C. Protein concentrations were determined by the Popov (Popov et a. (1975) Acta. Biol. Med. Germ, 34, 1441-1446). Cell extracts were used immediately or frozen in aliquots at -80 °C.

For 2D polyacrylamide gel electrophoresis of proteins 30 µg of crude protein extract was resuspended 450 µl of rehydration buffer (8M urea, 2M thiourea, 1% CHAPS, 20 mM DTT, 1% Ampholines 3.5 - 10) and a few grains of bromophenol blue. For isoelectric focussing precast 24 cm-IPG strips with a linear pH gradient of 4.5 to 5.5 were used in a Multiphor II isoelectric focussing unit (Amersham Biosciences). Proteins were focused using a gradient programme up to 3500 V resulting 65.000 Vh in total. Focused IPG gels were equilibrated twice for 15 minutes in a buffer containing 1.5 M Tris-HCl (pH 8.8), 6M urea, 30 % (vol/vol) glycerol, 2 % (wt/vol) sodium dodecyl sulfate, and 1 % (wt/vol) DTT. For the second equilibration step DDT was replaced by 5 % (wt/vol) iodoacetamide, and a few grains of bromophenol blue were added. The second dimension was run in sodium dodecyl sulfate-12.5 % polyacrylamide gels in an Ettan Dalt apparatus (Amersham Biosciences) as recommended by the manufacturer, and gels were subsequently silver stained (Blum et al. (1987), Electrophoresis, 8, 93-99) in a home made staining automat.

Protein spots were excised from preparative Coomassie-stained gels (300 µg total protein load each) and digested with modified trypsin (Roche, Mannheim) as described by Hermann et al. (Electrophoresis (2001), 22, 1712-1723). Mass spectrometrical identifications were performed on an LCQ advantage (Thermo Electron) after nano-HPLC separation of the peptides (LC Packings, RP18 column, length 15 cm, i.d. 75 µm), using the MASCOT software (David et al. (1999) Electrophoresis, 20,3551-3567).

Based on the 2D gel electrophoresis results of different gels 14 proteins were identified as being abundant in C. *glutamicum* as these proteins could be observed at elevated amounts in all gels. These proteins are: Elongation Factor Tu (Genbank accession no: X77034), glycerine-aldehyde-3-phosphate-dehydrogenase (Genbank accession no: BX927152, ±, nt. 289401-288397), fructose bisphosphate aldolase (Genbank accession no: BX927156, ±, nt. 134992-133958), Elongation Factor Ts (Genbank accession no: BX927154, ±, nt. 14902-14075), hypothetical protein (Genbank accession no: BX927155, ±, nt. 213489-214325) enolase (Genbank accession no: BX927150, nt. 338561-339838) peptidyl-prolyl cis-trans isomerase (Genbank accession no: BX927148, nt. 34330-34902), superoxide dismutase (Genbank accession no: AB055218) phospho-glycerate dehydrogenase (Genbank accession no: BX927151, nt. 306039-307631) SSU Rib protein S1P(Genbank accession no: BX927152, ±, nt. 26874-28334) triosephosphate-isomerase (Genbank accession no: BX927152, ±, nt. 286884-286105) isopropyl malate synthase (Genbank accession no: X70959) butan-2,3-dioldehydrogenase (Genbank accession no: BX927156, nt. 20798-21574) and fumarate hydratase (Genbank accession no: BX927151, ±, nt. 18803-17394).

The coding sequences of these genes were then fed into the "Cusp" function of the EMBOSS tool box using standard parameters In an independent approach the genomic sequence of the complete *C*. *glutamicum* strain ATCC 13032 was used to generate a codon usage table for the organism as a whole.

The codon usage frequencies as determined for the aforementioned 14 abundant proteins were used to calculate codon usage frequencies for abundant proteins in C. *glutamicum.* The codon relative codon usage frequencies of abundant proteins in C. *glutamicum* are found in table 2, while the relative codon usage frequencies of the organism as a whole are found in table 1.

**Table 1 - Relative codon usage frequencies of Corynebacterium glutamicum ATCC 13032.**

| | | | |
|---|---|---|---|
| UUU 37.1 | UCU 17.3 | UAU 33.8 | UGU 36.5 |
| UUC 62.9 | UCC 33.6 | UAC 66.2 | UGC 63.5 |
| UUA 5.3 | UCA 13.0 | UAA 53.1 | UGA 16.7 |
| UUG 20.3 | UCG 11.9 | UAG 30.2 | UGG 100 |
| | | | |
| CUU 17,2 | CCU 23.3 | CAU 32.1 | CGU 24.5 |
| CUC 22.5 | CCC 20.2 | CAC 67.9 | CGC 44.7 |
| CUA 6.1 | CCA 34.9 | CAA 38.5 | CGA 11.8 |
| CUG 28.6 | CCG 21.6 | CAG 61.5 | CGG 8.8 |
| | | | |
| AUU 37.7 | ACU 20.4 | AAU 33.4 | AGU 7.8 |
| AUC 59.2 | ACC 52.9 | AAC 66.4 | AGC 16.4 |
| AUA 3.1 | ACA 12.5 | AAA 39.9 | AGA 4.1 |
| AUG 100 | ACG 14.2 | AAG 60.1 | AGG 6.1 |
| | | | |
| GUU 26.0 | GCU 23.7 | GAU 55.6 | GGU 30.3 |
| GUC 27.7 | GCC 25.4 | GAC 44.4 | GGC 42.4 |
| GUA 10.1 | GCA 29.3 | GAA 56.3 | GGA 18.9 |
| GUG 36.2 | GCG 21.6 | GAG 43.7 | GGG 8.4 |
| | | | |
| ATG* 72.5 | | | |
| GTG* 20.5 | | | |
| TTG* 7.0 | | | |

| | | | |
|---|---|---|---|
| *designates start codons; relative Frequencies are in percentage. | | | |

**Table 2 - Relative codon usage frequencies of 14 abundant proteins in Corynebacterium glutamicum ATCC 13032.**

| | | | |
|---|---|---|---|
| UUU 10.6 | UCU 20.2 | UAU 3.6 | UGU 25.0 |
| UUC 89.4 | UCC 65.4 | UAC 96.4 | UGC 75.0 |
| UUA 0.8 | UCA 3.3 | UAA 92.9 | UGA 0.0 |
| UUG 7.5 | UCG 2.2 | UAG 7.1 | UGG 100 |
| | | | |
| CUU 20.8 | CCU 37.6 | CAU 5.8 | CGU 39.6 |
| CUC 25.4 | CCC 4.4 | CAC 94.2 | CGC 57.6 |
| CUA 2.6 | CCA 51.4 | CAA 7.7 | CGA 2.2 |
| CUG 42.9 | CCG 6.6 | CAG 92.3 | CGG 0.6 |
| | | | |
| AUU 17.1 | ACU 18.9 | AAU 9.7 | AGU 0.8 |
| AUC 82.6 | ACC 78.9 | AAC 90.3 | AGC 8.1 |
| AUA 0.3 | ACA 1.4 | AAA 7.1 | AGA 0.0 |
| AUG 100 | ACG 0.8 | AAG 92.9 | AGG 0.0 |
| | | | |
| GUU 47.9 | GCU 46.8 | GAU 34.9 | GGU 32.3 |
| GUC 34.0 | GCC 9.9 | GAC 65.1 | GGC 59.0 |
| GUA 6.5 | GCA 35.9 | GAA 32.5 | GGA 8.2 |
| GUG 11.6 | GCG 7.4 | GAG 67.5 | GGG 0.5 |
| | | | |
| ATG* 78.6 | | | |
| GTG* 21.4 | | | |
| TTG* 0.0 | | | |

| | | | |
|---|---|---|---|
| * indicates start codons; relative Frequencies are in percentage. | | | |

Table 1 was then used to determine the codons that are least frequently for each amino acid in *C*. *glutamicum.* This information is displayed in table 3 below.

**Table 3 - The least frequently used codons in Corynebacterium glutamicum ATCC 13032.**

| | |
|---|---|
| UUU | F |
| AGU | S |
| UAU | Y |
| UGU | C |
| UGA | Stop |
| AUG | M |
| UGG | W |
| UUA | L |
| CCC | P |
| CAU | H |
| AGA | R |
| CAA | Q |
| AUA | I |
| ACA | T |
| AAU | N |
| TTG (if start codon) | M |
| AAA | K |
| GUA | V |
| GCG | A |
| GAC | D |
| GGG | G |
| GAG | E |

Table 2 was then used to determine the codons that are least frequently for each amino acid in abundant proteins of C. *glutamicum.* This information is displayed in table 4 below.

**Table 4 - The least frequently used codons in abundant proteins of Corynebacterium glutamicum ATCC 13032.**

| | |
|---|---|
| UUU | F |
| AGU | S |
| UAU | Y |
| UGU | C |
| UGA | Stop |
| AUG | M |
| UGG | W |
| UUA | L |
| CCC | P |
| CAU | H |
| AGA, AGG | R |
| CAA | Q |
| AUA | I |
| ACG | T |
| AAU | N |
| TTG (if start codon) | M |
| AAA | K |
| GUA | V |
| GCG | A |
| GAU | D |
| GGG | G |
| GAA | E |

Table 5 shows the frequencies of codons which are not calculated on the basis of codons encoding a specific amino acid, but on the basis of all codons for all amino acids. The values in brackets indicate the absolute number of the respective codon. The relative frequencies of table 1 were calculated on the basis of these absolute numbers. The values refer to the genome of *C*. *glutamicum.*

**Table 5 - Codon usage of Corynebacterium glutamicum ATCC 13032.**

| | | | | | | | |
|---|---|---|---|---|---|---|---|
| UUU | 13.4( 25821) | UCU | 11.0( 21227) | UAU | 7.5( 14384) | UGU | 2.4( 4605) |
| UUC | 22.8( 43837) | UCC | 21.4( 41118) | UAC | 14.7( 28214) | UGC | 4.2( 8015) |
| UUA | 5.1( 9795) | UCA | 8.3( 15898) | UAA | 1.7( 3272) | UGA | 0.5( 1032) |
| UUG | 19.6( 37762) | UCG | 7.6( 14639) | UAG | 1.0( 1859) | UGG | 14.1( 27072) |
| | | | | | | | |
| CUU | 16.7( 32074) | CCU | 11.3( 21668) | CAU | 6.8( 12991) | CGU | 13.7( 26310) |
| CUC | 21.8( 41988) | CCC | 9.7( 18716) | CAC | 14.3( 27445) | CGC | 24.9( 47939) |
| CUA | 5.9( 11320) | CCA | 16.9( 32429) | CAA | 13.0( 24975) | CGA | 6.6( 12698) |
| CUG | 27.7( 53261) | CCG | 10.4( 20070) | CAG | 20.7( 39864) | CGG | 4.9( 9466) |
| | | | | | | | |
| AUU | 21.7( 41804) | ACU | 12.6( 24184) | AAU | 10.9( 21056) | AGU | 4.9( 9515) |
| AUC | 34.1( 65557) | ACC | 32.5( 62592) | AAC | 21.8( 42037) | AGC | 10.4( 20019) |
| AUA | 1.8( 3483) | ACA | 7.7( 14747) | AAA | 13.9( 26703) | AGA | 2.3( 4445) |
| AUG | 22.1( 42484) | ACG | 8.8( 16879) | AAG | 20.9( 40213) | AGG | 3.3( 6398) |
| | | | | | | | |
| GUU | 20.8( 40069) | GCU | 25.4( 48864) | GAU | 33.0( 63429) | GGU | 24.3( 46678) |
| GUC | 22.2( 42696) | GCC | 27.2( 52264) | GAC | 26.4( 50716) | GGC | 34.0( 65427) |
| GUA | 8.1( 15628) | GCA | 31.3( 60329) | GAA | 35.7( 68737) | GGA | 15.2( 29219) |
| GUG | 28.9( 55708) | GCG | 23.2( 44613) | GAG | 27.7( 53381) | GGG | 6.7( 12923) |

| | | | | | | | |
|---|---|---|---|---|---|---|---|
| Frequencies are indicated after the codons in /1000. | | | | | | | |

Table 6 shows the frequencies of codons which were not calculated on the basis of codons encoding a specific amino acid, but on the basis of all codons for all amino acids. The values in brackets indicate the absolute number of the respective codon. The relative frequencies of table 2 were calculated on the basis of these absolute numbers. The values refer to the group of abundant proteins in *C*. *glutamicum.*

**Table 6 - Codon usage of 14 abundant proteins in Corynebacterium glutamicum ATCC 13032.**

| | | | | | | | |
|---|---|---|---|---|---|---|---|
| UUU | 3.6( 18) | UCU | 10.9( 55) | UAU | 0.8( 4) | UGU | 1.2( 6) |
| UUC | 30.0( 152) | UCC | 35.2( 178) | UAC | 21.1( 107) | UGC | 3.6( 18) |
| UUA | 0.6 ( 3) | UCA | 1.8( 9) | UAA | 2.6( 13) | UGA | 0.0( 0) |
| UUG | 5.7( 29) | UCG | 1.2( 6) | UAG | 0.2( 1) | UGG | 8.3( 42) |
| | | | | | | | |
| CUU | 16.0( 81) | CCU | 13.4( 68) | CAU | 1.2( 6) | CGU | 17.4( 88) |
| CUC | 19.6( 99) | CCC | 1.6( 8) | CAC | 19.4( 98) | CGC | 25.3( 128) |
| CUA | 2.0( 10) | CCA | 18.4( 93) | CAA | 2.6( 13) | CGA | 1.0( 5) |
| CUG | 33.0( 167) | CCG | 2.4( 12) | CAG | 30.6( 155) | CGG | 0.2( 1) |
| | | | | | | | |
| AUU | 9.9( 50) | ACU | 10.5( 53) | AAU | 4.0( 20) | AGU | 0.4( 2) |
| AUC | 47.8( 242) | ACC | 43.7( 221) | AAC | 36.8( 186) | AGC | 4.3( 22) |
| AUA | 0.2( 1) | ACA | 0.8( 4) | AAA | 3.6 ( 18) | AGA | 0.0( 0) |
| AUG | 17.2( 87) | ACG | 0.4( 2) | AAG | 46.4( 235) | AGG | 0.0( 0) |
| | | | | | | | |
| GUU | 43.5( 220) | GCU | 54.9( 278) | GAU | 21.9( 111) | GGU | 28.1( 142) |
| GUC | 30.8( 156) | GCC | 11.7( 59) | GAC | 40.9( 207) | GGC | 51.2( 259) |
| GUA | 5.9( 30) | GCA | 42.1( 213) | GAA | 27.9( 141) | GGA | 7.1( 36) |
| GUG | 10.5( 53) | GCG | 8.7( 44) | GAG | 57.9( 293) | GGG | 0.4( 2) |

| | | | | | | | |
|---|---|---|---|---|---|---|---|
| Frequencies are indicated after the codons in /1000. | | | | | | | |

Surprisingly there are many significant differences in the codon usage between tables generated by using all proteins (whole genome, table 1) compared to the situation where only the above-specified abundant genes are considered (table 2). Some of the examples are shown in table 7 below.

**Table 7 - Relative frequency of codons used**

| Amino acid | Codon | Whole genome | Abundant proteins |
|---|---|---|---|
| q | caa | 38.5% | 7.7 |
| q | cag | 61.5% | 91.3 |
| y | tac | 66.0% | 96.4 |
| y | tat | 33.8% | 3.6 |

### 3. Reduction of expression of an endogenous protein

It has been set out above that codon usage modification may be used to decrease protein expression. This will be illustrated in the following for the proteins isocitrate dehydrogenase (icd) and diaminopimelate synthase (dapA).

### 3.1 Reducing expression of isocitrate dehydrogenase (icd)

### Cloning

To reduce the activity of the isocitrate dehydrogenase (Genbank Accession code X71489), two different changes in codon usage were made. In all cases the codons of the coding sequence were changed without changing the amino acid sequence of the encoded protein. The manipulations were all made on the only chromosomal copy of the icd gene of *Corynebacterium glutamicum.* The subsequent measurement of ICD activity directly allows a readout of the effect, as one can assume that it reflects the expression level given that the enzyme itself is not changed. The modifications are shown in table 8.

**Table 8 - Overview codon exchanges in ICD**

| | name | Description | affected amino acid positions |
|---|---|---|---|
| 1 | ICD ATG → GTG | Change of the start codon from ATG to GTG | 1 (Met) |
| 2 | ICD CA2 | Change of a glycine and an isoleucine codon from GGC ATT to GGG ATA | 32 (Gly), 33 (Ile) |

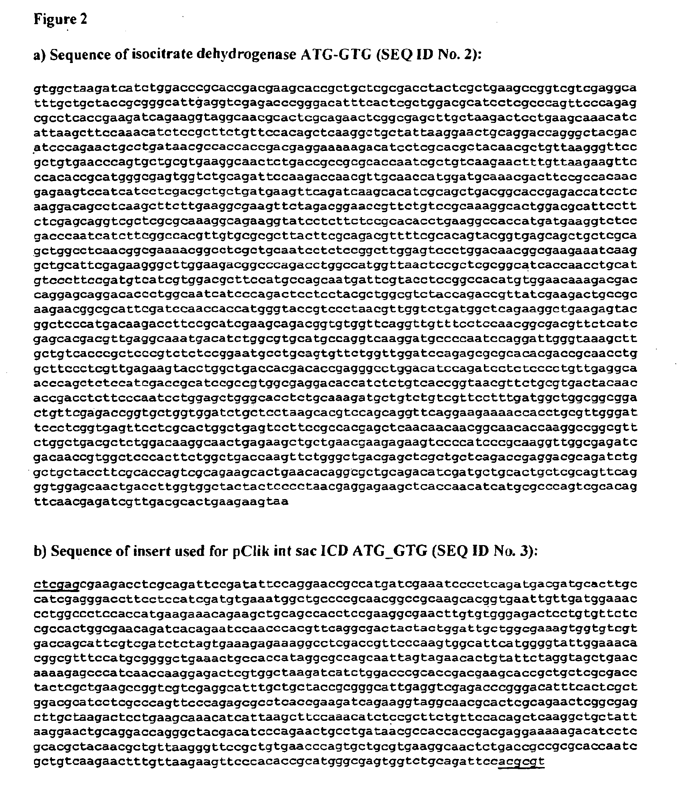

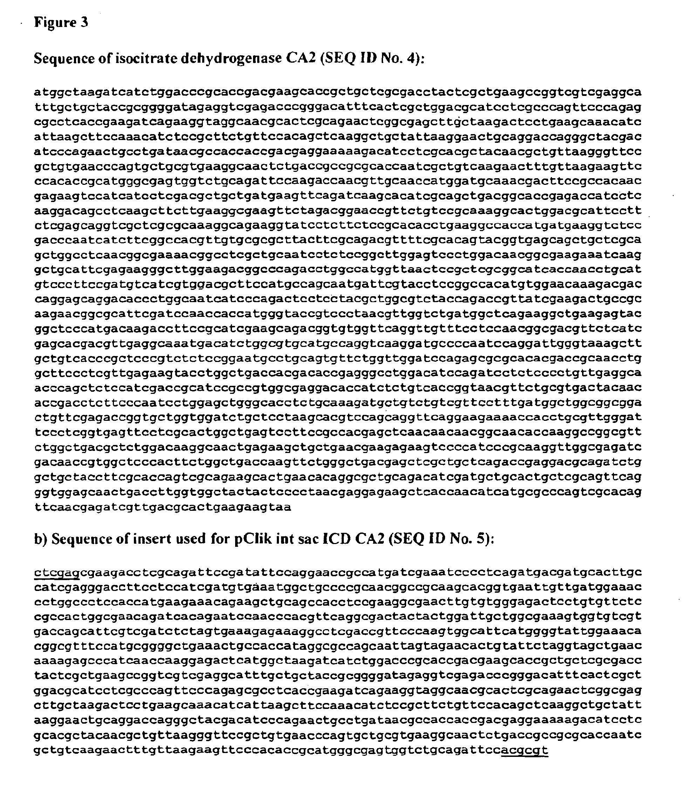

The sequence of ICD ATG-GTG is depicted in figure 2 a). The sequence of ICD CA is depicted in figure 3 a). To introduce these mutations into the chromosomal copy of the icd coding region, 2 different plasmids were constructed which allow the marker-free manipulation by 2 consecutive homologous recombination events.

To this end the sequences of ICD ATG-GTG and ICD CA2 were cloned into the vector pClik int sacB (Becker et al (2005), Applied and Environmental Microbiology, 71 (12), p.8587-8596) being a plasmid containing the following elements:
- Kanamycin-resistance gene
- SacB-gene which can be used as a positive selection marker as cells which carry this gene cannot grow on sucrose containing medium.
- Origin of replication for E. coli
- Multiple Cloning Site (MCS)

This plasmid allows the integration of sequences at the genomic locus of C. *glutamicum.*

### Construction of the plasmids

All inserts were amplified by PCR using genomic DNA of ATCC 13032 as a template. The modification of the coding region was achieved by fusion PCR using the following oligonucleotides: The table shows the primers used as well as the template DNA

**Table 9 - Overview of primers for cloning idh constructs**

| | PCR A | PCR B | Fusion PCR | |
|---|---|---|---|---|
| ICD ATG → GTG | Old 441 | Old 443 | Old 441 | Primer 1 |
| | Old 444 | Old 442 | Old 442 | Primer 2 |
| | Genom. DNA of ATCC 13032 | Genom. DNA of ATCC 13032 | PCR A+B | Template |
| ICD CA2 | Old 441 | Old 447 | Old 441 | Primer 1 |
| | Old 448 | Old 442 | Old 442 | Primer 2 |
| | Genom. DNA of ATCC 13032 | Genom. DNA of ATCC 13032 | PCR A+B | Template |

| | | | | |
|---|---|---|---|---|
| Old 441 GAGTACCTCGAGCGAAGACCTCGCAGATTCCG (SEQ ID No. 6) Old 442 CATGAGACGCGTGGAATCTGCAGACCACTCGC (SEQ ID No. 7) Old 443 GAGACTCGTGGCTAAGATCATCTG (SEQ ID No. 8) Old 444 CAGATGATCTTAGCCACGAGTCTC (SEQ ID No. 9) Old 447 CTACCGCGGGGATAGAGG (SEQ ID No. 10) Old 448 CCTCTATCCCCGCGGTAG (SEQ ID No. 11) | | | | |

In all cases the product of the fusion PCR was purified, digested with XhoI and MluI, purified again and ligated into pClik int sacB which had been linearized with the same restriction enzymes. The integrity of the insert was confirmed by sequencing.

The coding sequence of the optimised sequence ICD ATG → GTG is shown in Figure 2 (SEQ ID 2). The coding sequence of the optimised sequence ICD CA2 is shown in Figure 3 (SEQ ID 4).

### Construction of strains with modified ICD expression levels

The plasmids were then used to replace the native coding region of these genes by the coding regions with the modified coding usage. The strain used was ATCC 13032 lysC fbr.

Two consecutive recombination events one in each of the up- and the downstream region respectively are necessary to change the complete coding sequence. The method of replacing the endogenous genes with the optimized genes is in principle described in the publication by Becker et al. (vide supra). The most important steps are:
- Introduction of the plasmids in the strain by electroporation. The step is e.g. described in DE 10046870.
- Selection of clones that have successfully integrated the plasmid after a first homologous recombination event into the genome. This selection is achieved by growth on kanamycine-containing agar plates. In addition to that selection step, successful recombination can be checked via colony PCR.
   Primers used to confirm the presence of the plasmid in the genome were: BK1776 (AACGGCAGGTATATGTGATG) (SEQ ID No. 12) and OLD 450 (CGAGTAGGTCGCGAGCAG) (SEQ ID No. 13). The positive clones give a band of ca. 600 bp)
- By incubating a positive clone in a kanamycine-free medium a second recombination event is allowed for.
- Clones in which the vector backbone has been successfully removed by way of a second recombination event are identified by growth on sucrose -containing medium. Only those clones will survive that have lost the vector backbone comprising the SacB gene.
- Then, clones in which the two recombination events have led to successful replacement of the native idh-coding region were identified by sequencing of a PCR-product spanning the relevant region. The PCR- product was generated using genomic DNA of individual clones as a template and primers OLD 441 and OLD 442. The PCR-product was purified and sequenced with Old 471 (GAATCCAACCCACGTTCAGGC) (SEQ ID No. 14)

One may use different *C*. *glutamicum* strains for replacing the endogenous copy of ICD. However, it is preferred to use a C. *glutamicum* lysine production strain such as for example ATCC 13032 lysC^{fbr} or other derivatives of ATCC13032 or ATCC 13286

ATCC13032 lysC^{fbr} may be produced starting from ATCC13032. In order to generate such a lysine producing strain, an allelic exchange of the lysC wild type gene was performed in *C*. *glutamicum* ATCC13032. To this end a nucleotide exchange was introduced into the lysC gene such that the resulting protein carries an isoleucine at position 311 instead of threonine. The detailed construction of this strain is described in patent application WO2005059093. The accession no. of the lysC gene is P26512.

To analyze the effect of the codon usage amended IDH ATG-GTG and IDH CA2, the optimized strains are compared to lysine productivity of the parent strain.

### Determination of ICD activity

One to two clones of each mutant strain were tested for ICD activity. Cells were grown in liquid culture over night at 30 °C, harvested in exponential growth phase by centrifugation. The cells were washed twice with 50 mM Tris-HCl, pH 7.0. 200 mg cells were resuspended in 800 µl lysis buffer (50 mM Tris-HCL, pH 7.0, 10 mM MgCl2, 1 mM DTT, 10 % Glycerol) and disrupted by bead beating (Ribolyser, 2x 30s, intensity 6). The cell debris was pelleted by centrifugation (table top centrifuge, 30 min, 13 K). The resulting supernatant is an extract of soluble proteins which was used as the following enzyme assay.

ICD activity was monitored by increase of absorption at 340 nm due to the reduction of NADP in a total volume of 1 ml under the following conditions:
30 mM Triethanolamine-chloride, pH 7.4, 0.4 mM NADP, 8 mM DL-Isocitrate, 2 mM MnSO4, cell lysate corresponding to 0.1-0.2 mg protein

ICD activities were calculated using the molar extinction coefficient of 6.22/mM*cm for NADPH.

### Results

The measured ICD activities were as follows:

**Table 10 - ICD activity**

| Strain | Clone | Specific activity of cell extract |
|---|---|---|
| | | U*µmol/ml*min*mg protein |
| ATCC lysC fbr | | 0,29 |
| ATCC lysC fbr | | 0,26 |
| ICD ATG → GTG | | 0,04 |
| ICD CA2 | 1 | 0,10 |
| ICD CA2 | 2 | 0,10 |

### Effect on lysine productivity

To analyze the effect of the modified expression of ICD on lysine productivity, the optimized strains are compared to lysine productivity of the parent strains.

To this end one the strains were grown on CM-plates (10% sucrose, 10 g/l glucose, 2,5 g/l NaCl, 2 g/l urea, 10 g/l Bacto Pepton, 10 g/l yeast extract, 22 g/l agar) for 2 days at 30°C. Subsequently cells were scraped from the plates and re-suspended in saline. For the main culture 10 ml of medium 1 and 0.5 g autoclave CaCo₃ in a 100 ml Erlenmeyer flask were incubated together with the cell suspension up to an OD₆₀₀ of 1.5. The cells were then grown for 72 hours on a shaker of the type Infors AJ118 (Infors, Bottmingen, Switzerland) at 220 rpm. Medium 1 had the same concentration as mentioned in experiment 3.3:

Subsequently, the concentration of lysine that is segregated into the medium was determined. This was dome using HPLC on an Agilent 1100 Series LC system HPLC. A precolumn derivatisation with ortho-phthalaldehyde allowed to quantify the formed amino acid. The separation of the amino acid mixture can be done on a Hypersil AA-column (Agilent).

The determined lysine concentration values shown are average data from 2 independent cultivations. The deviations from the average was always below 4%

**Table 11 - Lysine productivity**

| Strain | Clone | Relative lysine amount [%] | Relative OD [%] |
|---|---|---|---|
| ATCC lysC fbr | | 100.00 | 100,00 |
| ATCC lysC fbr | | 99,81 | 101,22 |
| ICD ATG → GTG | | 102,34 | 92,77 |
| ICD CA2 | 1 | 101,44 | 99,80 |
| ICD CA2 | 2 | 104,85 | 96,23 |

It can be easily seen that strains with lowered ICD activity have higher lysine productivities. As all carbon source is used after 72 h, one can also directly see that the carbon yield (amount of formed product per sugar consumed) is higher in these strains.

The amount of other amino acids in the medium after another cultivation was also analyzed. Interestingly, the amount of glycine is also influenced by ICD expression.

The table shows the amount of glycine obtained after 48 h of cultivation. Each clone was cultivated in three independent flasks.

**Table 12 - Glycine productivity**

| | | Glycine [µmol/l |
|---|---|---|
| Medium | | 5,72 |
| ATCC lysC fbr | | 78,56 |
| | | 81,33 |
| | | 79,40 |
| ATCC lysC fbr | | 86,29 |
| | | 78,51 |
| | | 81,35 |
| ICD ATG → GTG | | 110,44 |
| | | 108,51 |
| | | 117,36 |
| ICD CA2 | 1 | 103,29 |
| | | 104,56 |
| | | 96,26 |
| ICD CA2 | 2 | 98,83 |
| | | 98,72 |
| | | 99,39 |

It can be seen that strains with lower ICD activity accumulate higher amounts of glycine.

In a further experiment isocitrate dehydrogenase carrying the above mentioned ATG-GTG mutation in the start codon was cloned into pClik as described above leading to pClik int sacB ICD (ATG-GTG) (SEQ ID No. 15). Subsequently, strain M2620 was constructed by campbelling in and campbelling out the plasmid pClik int sacB ICDH (ATG-GTG) (SEQ ID No. 15) into genome of the strain OM469. The strain OM469 has been described in WO2007012078.

The strain was grown as described in WO2007020295. After 48h incubation at 30°C the samples were analyzed for sugar consumption. It was found that the strains had used up all added sugar, meaning that all strains had used the same amount of carbon source. Synthesized methionine was determined by HPLC as described above and in WO2007020295.

**Table 13 - Methionine production**

| Strain | Methionine (mM) |
|---|---|
| OM 469 | 10,2 |
| M2620 | 23,7 |

From the data in table 13 it can be seen that the strain M2620 with an altered start codon of the ICDH gene and therefore altered ICDH activity has higher methionine productivity. Since all carbon source is used up after 48h, one can also directly see, that the carbon yield (amount of formed product per sugar consumed) for the produced methionine is higher in this strain.

### 3.2. Reduced protein expression of dapA in C. glutamicum

In the following, it is described how to decrease the amount of dapA by adapting the codon usage as mentioned above.

The enzyme dihydropicolinate synthase (dapA) is important for lysine biosynthesis. The wild type sequence of *C*. *glutamicum* dapA is given as SEQ ID No. 16. The codon usage of the coding sequence of dapA was determined using the Cusp function of the EMBOSS software package.

Then, the bases corresponding to positions 12 (T to G), 27 (C to A), 30 (A to G), 45 (C to G) and 54 (A to G) were altered using established mutagenesis methods (Quikchange kit, Stratagene La Jolla USA) resulting in altered codons which still coded for the same amino acids (SEQ ID No. 17).

The mutated gene sequence was cloned into a vector for chromosomal integration as mentioned in WO2007020295.

Briefly, The strain M2059 was constructed by campbelling in and out the plasmid pK19 PSOD ask (SEQ ID No. 18) into the strain OM99 to overexpress the aspartokinase gene The strain M2121 was constructed by campbelling in and out the plasmid pCLIK Int Psod Glucose-6-PDH (SEQ ID No. 19) into the strain M2059 to over express glucose-6-phosphate dehydrogenase gene (zwf gene). The strain M2199 was constructed by campbelling in and out the plasmid pK19 sacB Glu-6-P-DH (SEQ ID No. 20) replacement into the strain M2121 to express a glucose-6-phosphate dehydrogenase mutant gene (zwf gene). The strain M2271 was constructed by campbelling in and out the plasmid pK19 PGro PycA (SEQ ID No. 21) into the strain M2199 to express a mutated pycA gene. The strain M2376 was constructed by campbelling in and out the plasmid pCLIK int sacB dapA codon low (SEQ ID No. 22) into the strain M2271 to express the mutated dapA.

The resulting strain was named M2376 and grown as described in 2007020295. Amin oacids were determined after 48h incubation and are described as mM concentration (see table 14).

**Table 14 - lysine and homoserine production**

| Strain | homoserine (mM) |
|---|---|
| M2271 | 78 |
| M2376 (= M2271 + dapA mutated) | 103 |

It was found that strains expressing the mutated dapA gene showed an increased accumulation of homoserine when compared to the isogenic strain showing that the activity of dapA was decreased over the unmutated version of dapA

## Claims

1. Method of reducing the amount at least one polypeptide in a *Corynebacterium glutamicum* cell, comprising the step of expressing in a *C. glutamicum* cell a modified nucleotide sequence instead of a non-modified nucleotide sequence encoding for said polypeptide having the same amino acid sequence and/or function wherein said modified nucleotide sequence is derived from the non-modified nucleotide sequence such that at least one codon of the non-modified nucleotide sequence is replaced in the modified nucleotide sequence by a less frequently used codon according to the codon usage of *C. glutamicum,* wherein said at least one codon is the start codon being replaced by a start codon which is less frequently used in *C. glutamicum,* wherein said less frequently used start codon is GTG or TTG.

2. Method according to claim 1,
wherein said modified nucleotide sequence is derived from the non-modified nucleotide sequence such that at least one further codon of the non-modified nucleotide sequence is replaced in the modified nucleotide sequence by a less frequently used codon according to the codon usage of abundant proteins of *C*. *glutamicum.*

3. Method according to claim 1 or 2,
wherein at least one codon of the non-modified nucleotide sequence is replaced in the modified nucleotide sequence by one of the two least frequently used codons as set forth in table 1.

4. Method according to claim 3,
wherein all codons of said modified nucleotide sequence for each amino acid are selected from the codon usage of table 3.

5. Method according to claim 1 or 2,
wherein at least one codon of the non-modified nucleotide sequence is replaced in the modified nucleotide sequence by one of the two least frequently used codons as set forth in table 2.

6. Method according to claim 5,
wherein said modified nucleotide sequence uses the codons ACG for threonine; or GAT for aspartic acid; or GAA for glutamic acid; or AGA or AGG for arginine and TTG for the start codon.

7. Method according to claim 5 or 6,
wherein all codons of said modified nucleotide sequence for each amino acid are selected from the codon usage of table 4.

8. Method according to any one of claims 1 to 7,
wherein the *C. glutamicum* strain ATCC 13032 or derivatives thereof is used.

9. Method according to any of claims 1 to 8,
wherein the method is used to decrease expression of said polypeptide in the *C. glutamicum* cell by at least 5%, 10%, 20%, 30%, 40%, 50%, 60%, 70%, 80%, 90% or 95%, with the extent of reduction of expression being determined in comparison to the level of expression of the polypeptide that is expressed from the non-modified nucleotide sequence under comparable conditions.

## Patentansprüche

1. Verfahren zur Reduzierung der Menge wenigstens eines Polypeptids in einer *Corynebacterium glutamicum*-Zelle, umfassend den Schritt der Expression einer modifizierten Nukleotidsequenz statt einer nicht modifizierten Nukleotidsequenz, die für das Polypeptid mit der gleichen Aminosäuresequenz und/oder Funktion codiert, in einer *C. glutamicum*-Zelle, wobei die modifizierte Nukleotidsequenz von der nicht modifizierten Nukleotidsequenz so abgeleitet ist, dass wenigstens ein Codon der nicht modifizierten Nukleotidsequenz in der modifizierten Nukleotidsequenz durch ein weniger häufig verwendetes Codon entsprechend der Codonverwendung von *C. glutamicum* ersetzt ist, wobei es sich bei dem wenigstens einen Codon um das Startcodon handelt, das durch ein weniger häufig in *C. glutamicum* verwendetes Startcodon ersetzt ist, wobei es sich bei dem weniger häufig verwendeten Startcodon um GTG oder TTG handelt.

2. Verfahren nach Anspruch 1,
wobei die modifizierte Nukleotidsequenz von der nicht modifizierten Nukleotidsequenz so abgeleitet ist, dass wenigstens ein weiteres Codon der nicht modifizierten Nukleotidsequenz in der modifizierten Nukleotidsequenz durch ein weniger häufig verwendetes Codon entsprechend der Codonverwendung bei häufig vorkommenden Proteinen von *C. glutamicum* ersetzt ist.

3. Verfahren nach Anspruch 1 oder 2,
wobei wenigstens ein Codon der nicht modifizierten Nukleotidsequenz in der modifizierten Nukleotidsequenz durch eines der beiden am wenigsten häufig verwendeten Codons gemäß Tabelle 1 ersetzt ist.

4. Verfahren nach Anspruch 3,
wobei alle Codons der modifizierten Nukleotidsequenz für jede Aminosäure anhand der Codonverwendung in Tabelle 3 ausgewählt sind.

5. Verfahren nach Anspruch 1 oder 2,
wobei wenigstens ein Codon der nicht modifizierten Nukleotidsequenz in der modifizierten Nukleotidsequenz durch eines der beiden am wenigsten häufig verwendeten Codons gemäß Tabelle 2 ersetzt ist.

6. Verfahren nach Anspruch 5,
wobei in der modifizierten Nukleotidsequenz die Codons ACG für Threonin; oder GAT für Asparaginsäure; oder GAA für Glutaminsäure; oder AGA oder AGG für Arginin und TTG für das Startcodon verwendet werden.

7. Verfahren nach Anspruch 5 oder 6,
wobei alle Codons der modifizierten Nukleotidsequenz für jede Aminosäure anhand der Codonverwendung in Tabelle 4 ausgewählt sind.

8. Verfahren nach einem der Ansprüche 1 bis 7,
wobei der *C. glutamicum*-Stamm ATCC 13032 oder Derivate davon verwendet wird bzw. werden.

9. Verfahren nach einem der Ansprüche 1 bis 8,
wobei das Verfahren zur Verringerung der Expression des Polypeptids in der *C. glutamicum-*zelle um wenigstens 5%, 10%, 20%, 30%, 40%, 50%, 60%, 70%, 80%, 90% oder 95% verwendet wird, wobei das Ausmaß der Reduzierung der Expression im Vergleich zum Expressionsniveau des Polypeptids, das von der nicht modifizierten Nukleotidsequenz unter vergleichbaren Bedingungen exprimiert wird, bestimmt wird.

## Revendications

1. Procédé de réduction de la quantité d'au moins un polypeptide dans une cellule de *Corynebacterium glutamicum,* comprenant l'étape consistant à exprimer, dans une cellule de *C*. *glutamicum,* une séquence nucléotidique modifiée à la place d'une séquence nucléotidique non modifiée codant pour ledit polypeptide, ayant la même séquence d'acides aminés et/ou fonction, dans laquelle ladite séquence nucléotidique modifiée est dérivée de la séquence nucléotidique non modifiée de sorte qu'au moins un codon de la séquence nucléotidique non modifiée soit remplacé, dans la séquence nucléotidique modifiée, par un codon moins fréquemment utilisé selon l'utilisation des codons de *C. glutamicum,* dans laquelle ledit au moins un codon est le codon d'initiation, qui est remplacé par un codon d'initiation qui est moins fréquemment utilisé dans *C*. *glutamicum,* dans laquelle ledit codon d'initiation étant moins fréquemment utilisé est un GTG ou TTG.

2. Procédé selon la revendication 1, dans laquelle ladite séquence nucléotidique modifiée est dérivée de la séquence nucléotidique non modifiée de sorte qu'au moins un codon supplémentaire de la séquence nucléotidique non modifiée soit remplacé, dans la séquence nucléotidique modifiée, par un codon moins fréquemment utilisé selon l'utilisation des codons des protéines abondantes de *C. glutamicum.*

3. Procédé selon la revendication 1 ou la 2, dans laquelle au moins un codon de la séquence nucléotidique non modifiée est remplacé, dans la séquence nucléotidique modifiée, par l'un des deux codons les moins fréquemment utilisés, tel qu'indiqué dans le tableau 1.

4. Procédé selon la revendication 3, dans laquelle tous les codons de ladite séquence nucléotidique modifiée, pour chaque acide aminé, sont choisis à partir de l'utilisation des codons du tableau 3.

5. Procédé selon la revendication 1 ou la 2, dans laquelle au moins un codon de la séquence nucléotidique non modifiée est remplacé, dans la séquence nucléotidique modifiée, par l'un des deux codons les moins fréquemment utilisés, tel qu'indiqué dans le tableau 2.

6. Procédé selon la revendication 5, dans laquelle ladite séquence nucléotidique modifiée utilise les codons : ACG pour la thréonine ; ou GAT pour l'acide aspartique ; ou GAA pour l'acide glutamique ; ou AGA ou AGG pour l'arginine et TTG pour le codon d'initiation.

7. Procédé selon la revendication 5 ou la 6, dans laquelle tous les codons de ladite séquence nucléotidique modifiée, pour chaque acide aminé, sont choisis à partir de l'utilisation des codons du tableau 4.

8. Procédé selon l'une quelconque des revendications 1 à 7, dans laquelle est utilisée la souche ATCC 13032 de C. *glutamicum* ou sont utilisés des dérivés de celle-ci.

9. Procédé selon l'une quelconque des revendications 1 à 8, dans laquelle le procédé est utilisé pour diminuer l'expression dudit polypeptide dans la cellule de *C. glutamicum* d'au moins 5%, 10%, 20%, 30%, 40%, 50%, 60%, 70%, 80%, 90% ou 95%, l'étendue de la réduction de l'expression étant déterminée par comparaison avec le niveau d'expression du polypeptide qui est exprimé à partir de la séquence nucléotidique non modifiée, dans des conditions comparables.


### SEQUENCE LISTING

<110> BASF AG
<120> Method of reducing gene expression using modified codon usage
<130> B 8441 / DB
<150> EP 06122882.1
   <151> 2006-10-24
<160> 22
<170> PatentIn version 3.3
<210> 1
   <211> 2217
   <212> DNA
   <213> Corynebacterium glutamicum
<400> 1
   atggctaaga tcatctggac ccgcaccgac gaagcaccgc tgctcgcgac ctactcgctg    60
   aagccggtcg tcgaggcatt tgctgctacc gcgggcattg aggtcgagac ccgggacatt   120
   tcactcgctg gacgcatcct cgcccagttc ccagagcgcc tcaccgaaga tcagaaggta   180
   ggcaacgcac tcgcagaact cggcgagctt gctaagactc ctgaagcaaa catcattaag   240
   cttccaaaca tctccgcttc tgttccacag ctcaaggctg ctattaagga actgcaggac   300
   cagggctacg acatcccaga actgcctgat aacgccacca ccgacgagga aaaagacatc   360
   ctcgcacgct acaacgctgt taagggttcc gctgtgaacc cagtgctgcg tgaaggcaac   420
   tctgaccgcc gcgcaccaat cgctgtcaag aactttgtta agaagttccc acaccgcatg   480
   ggcgagtggt ctgcagattc caagaccaac gttgcaacca tggatgcaaa cgacttccgc   540
   cacaacgaga agtccatcat cctcgacgct gctgatgaag ttcagatcaa gcacatcgca   600
   gctgacggca ccgagaccat cctcaaggac agcctcaagc ttcttgaagg cgaagttcta   660
   gacggaaccg ttctgtccgc aaaggcactg gacgcattcc ttctcgagca ggtcgctcgc   720
   gcaaaggcag aaggtatcct cttctccgca cacctgaagg ccaccatgat gaaggtctcc   780
   gacccaatca tcttcggcca cgttgtgcgc gcttacttcg cagacgtttt cgcacagtac   840
   ggtgagcagc tgctcgcagc tggcctcaac ggcgaaaacg gcctcgctgc aatcctctcc   900
   ggcttggagt ccctggacaa cggcgaagaa atcaaggctg cattcgagaa gggcttggaa   960
   gacggcccag acctggccat ggttaactcc gctcgcggca tcaccaacct gcatgtccct  1020
   tccgatgtca tcgtggacgc ttccatgcca gcaatgattc gtacctccgg ccacatgtgg  1080
   aacaaagacg accaggagca ggacaccctg gcaatcatcc cagactcctc ctacgctggc  1140
   gtctaccaga ccgttatcga agactgccgc aagaacggcg cattcgatcc aaccaccatg  1200
   ggtaccgtcc ctaacgttgg tctgatggct cagaaggctg aagagtacgg ctcccatgac  1260
   aagaccttcc gcatcgaagc agacggtgtg gttcaggttg tttcctccaa cggcgacgtt  1320
   ctcatcgagc acgacgttga ggcaaatgac atctggcgtg catgccaggt caaggatgcc  1380
   ccaatccagg attgggtaaa gcttgctgtc acccgctccc gtctctccgg aatgcctgca  1440
   gtgttctggt tggatccaga gcgcgcacac gaccgcaacc tggcttccct cgttgagaag   1500
   tacctggctg accacgacac cgagggcctg gacatccaga tcctctcccc tgttgaggca   1560
   acccagctct ccatcgaccg catccgccgt ggcgaggaca ccatctctgt caccggtaac   1620
   gttctgcgtg actacaacac cgacctcttc ccaatcctgg agctgggcac ctctgcaaag   1680
   atgctgtctg tcgttccttt gatggctggc ggcggactgt tcgagaccgg tgctggtgga   1740
   tctgctccta agcacgtcca gcaggttcag gaagaaaacc acctgcgttg ggattccctc   1800
   ggtgagttcc tcgcactggc tgagtccttc cgccacgagc tcaacaacaa cggcaacacc   1860
   aaggccggcg ttctggctga cgctctggac aaggcaactg agaagctgct gaacgaagag   1920
   aagtccccat cccgcaaggt tggcgagatc gacaaccgtg gctcccactt ctggctgacc   1980
   aagttctggg ctgacgagct cgctgctcag accgaggacg cagatctggc tgctaccttc   2040
   gcaccagtcg cagaagcact gaacacaggc gctgcagaca tcgatgctgc actgctcgca   2100
   gttcagggtg gagcaactga ccttggtggc tactactccc ctaacgagga gaagctcacc   2160
   aacatcatgc gcccagtcgc acagttcaac gagatcgttg acgcactgaa gaagtaa      2217
<210> 2
   <211> 2217
   <212> DNA
   <213> Artificial
<220>
   <223> Isocitrate Dehydrogenase, Corynebacterium glutamicum, ATG-GTG mutation in the first codon
<400> 2
   gtggctaaga tcatctggac ccgcaccgac gaagcaccgc tgctcgcgac ctactcgctg     60
   aagccggtcg tcgaggcatt tgctgctacc gcgggcattg aggtcgagac ccgggacatt    120
   tcactcgctg gacgcatcct cgcccagttc ccagagcgcc tcaccgaaga tcagaaggta    180
   ggcaacgcac tcgcagaact cggcgagctt gctaagactc ctgaagcaaa catcattaag    240
   cttccaaaca tctccgcttc tgttccacag ctcaaggctg ctattaagga actgcaggac    300
   cagggctacg acatcccaga actgcctgat aacgccacca ccgacgagga aaaagacatc    360
   ctcgcacgct acaacgctgt taagggttcc gctgtgaacc cagtgctgcg tgaaggcaac    420
   tctgaccgcc gcgcaccaat cgctgtcaag aactttgtta agaagttccc acaccgcatg    480
   ggcgagtggt ctgcagattc caagaccaac gttgcaacca tggatgcaaa cgacttccgc    540
   cacaacgaga agtccatcat cctcgacgct gctgatgaag ttcagatcaa gcacatcgca    600
   gctgacggca ccgagaccat cctcaaggac agcctcaagc ttcttgaagg cgaagttcta    660
   gacggaaccg ttctgtccgc aaaggcactg gacgcattcc ttctcgagca ggtcgctcgc    720
   gcaaaggcag aaggtatcct cttctccgca cacctgaagg ccaccatgat gaaggtctcc    780
   gacccaatca tcttcggcca cgttgtgcgc gcttacttcg cagacgtttt cgcacagtac    840
   ggtgagcagc tgctcgcagc tggcctcaac ggcgaaaacg gcctcgctgc aatcctctcc    900
   ggcttggagt ccctggacaa cggcgaagaa atcaaggctg cattcgagaa gggcttggaa    960
   gacggcccag acctggccat ggttaactcc gctcgcggca tcaccaacct gcatgtccct   1020
   tccgatgtca tcgtggacgc ttccatgcca gcaatgattc gtacctccgg ccacatgtgg   1080
   aacaaagacg accaggagca ggacaccctg gcaatcatcc cagactcctc ctacgctggc   1140
   gtctaccaga ccgttatcga agactgccgc aagaacggcg cattcgatcc aaccaccatg   1200
   ggtaccgtcc ctaacgttgg tctgatggct cagaaggctg aagagtacgg ctcccatgac   1260
   aagaccttcc gcatcgaagc agacggtgtg gttcaggttg tttcctccaa cggcgacgtt   1320
   ctcatcgagc acgacgttga ggcaaatgac atctggcgtg catgccaggt caaggatgcc   1380
   ccaatccagg attgggtaaa gcttgctgtc acccgctccc gtctctccgg aatgcctgca   1440
   gtgttctggt tggatccaga gcgcgcacac gaccgcaacc tggcttccct cgttgagaag   1500
   tacctggctg accacgacac cgagggcctg gacatccaga tcctctcccc tgttgaggca   1560
   acccagctct ccatcgaccg catccgccgt ggcgaggaca ccatctctgt caccggtaac   1620
   gttctgcgtg actacaacac cgacctcttc ccaatcctgg agctgggcac ctctgcaaag   1680
   atgctgtctg tcgttccttt gatggctggc ggcggactgt tcgagaccgg tgctggtgga   1740
   tctgctccta agcacgtcca gcaggttcag gaagaaaacc acctgcgttg ggattccctc   1800
   ggtgagttcc tcgcactggc tgagtccttc cgccacgagc tcaacaacaa cggcaacacc   1860
   aaggccggcg ttctggctga cgctctggac aaggcaactg agaagctgct gaacgaagag   1920
   aagtccccat cccgcaaggt tggcgagatc gacaaccgtg gctcccactt ctggctgacc   1980
   aagttctggg ctgacgagct cgctgctcag accgaggacg cagatctggc tgctaccttc   2040
   gcaccagtcg cagaagcact gaacacaggc gctgcagaca tcgatgctgc actgctcgca   2100
   gttcagggtg gagcaactga ccttggtggc tactactccc ctaacgagga gaagctcacc   2160
   aacatcatgc gcccagtcgc acagttcaac gagatcgttg acgcactgaa gaagtaa      2217
<210> 3
   <211> 1002
   <212> DNA
   <213> Artificial
<220>
   <223> Vectorinsert with Isocitrate Dehydrogenase, Corynebacterium glutamicum, ATG-GTG Mutation
<400> 3
   ctcgagcgaa gacctcgcag attccgatat tccaggaacc gccatgatcg aaatcccctc     60
   agatgacgat gcacttgcca tcgagggacc ttcctccatc gatgtgaaat ggctgccccg    120
   caacggccgc aagcacggtg aattgttgat ggaaaccctg gccctccacc atgaagaaac    180
   agaagctgca gccacctccg aaggcgaact tgtgtgggag actcctgtgt tctccgccac    240
   tggcgaacag atcacagaat ccaacccacg ttcaggcgac tactactgga ttgctggcga    300
   aagtggtgtc gtgaccagca ttcgtcgatc tctagtgaaa gagaaaggcc tcgaccgttc    360
   ccaagtggca ttcatggggt attggaaaca cggcgtttcc atgcggggct gaaactgcca    420
   ccataggcgc cagcaattag tagaacactg tattctaggt agctgaacaa aagagcccat    480
   caaccaagga gactcgtggc taagatcatc tggacccgca ccgacgaagc accgctgctc    540
   gcgacctact cgctgaagcc ggtcgtcgag gcatttgctg ctaccgcggg cattgaggtc    600
   gagacccggg acatttcact cgctggacgc atcctcgccc agttcccaga gcgcctcacc    660
   gaagatcaga aggtaggcaa cgcactcgca gaactcggcg agcttgctaa gactcctgaa    720
   gcaaacatca ttaagcttcc aaacatctcc gcttctgttc cacagctcaa ggctgctatt    780
   aaggaactgc aggaccaggg ctacgacatc ccagaactgc ctgataacgc caccaccgac    840
   gaggaaaaag acatcctcgc acgctacaac gctgttaagg gttccgctgt gaacccagtg    900
   ctgcgtgaag gcaactctga ccgccgcgca ccaatcgctg tcaagaactt tgttaagaag    960
   ttcccacacc gcatgggcga gtggtctgca gattccacgc gt                      1002
<210> 4
   <211> 2217
   <212> DNA
   <213> Artificial
<220>
   <223> Isocitrate Dehydrogenase, Corynebacterium glutamicum, GGC ATT--->GGG ATA mutation in positions 32 and 33
<400> 4
   atggctaaga tcatctggac ccgcaccgac gaagcaccgc tgctcgcgac ctactcgctg    60
   aagccggtcg tcgaggcatt tgctgctacc gcggggatag aggtcgagac ccgggacatt   120
   tcactcgctg gacgcatcct cgcccagttc ccagagcgcc tcaccgaaga tcagaaggta   180
   ggcaacgcac tcgcagaact cggcgagctt gctaagactc ctgaagcaaa catcattaag   240
   cttccaaaca tctccgcttc tgttccacag ctcaaggctg ctattaagga actgcaggac   300
   cagggctacg acatcccaga actgcctgat aacgccacca ccgacgagga aaaagacatc   360
   ctcgcacgct acaacgctgt taagggttcc gctgtgaacc cagtgctgcg tgaaggcaac   420
   tctgaccgcc gcgcaccaat cgctgtcaag aactttgtta agaagttccc acaccgcatg   480
   ggcgagtggt ctgcagattc caagaccaac gttgcaacca tggatgcaaa cgacttccgc   540
   cacaacgaga agtccatcat cctcgacgct gctgatgaag ttcagatcaa gcacatcgca   600
   gctgacggca ccgagaccat cctcaaggac agcctcaagc ttcttgaagg cgaagttcta   660
   gacggaaccg ttctgtccgc aaaggcactg gacgcattcc ttctcgagca ggtcgctcgc   720
   gcaaaggcag aaggtatcct cttctccgca cacctgaagg ccaccatgat gaaggtctcc   780
   gacccaatca tcttcggcca cgttgtgcgc gcttacttcg cagacgtttt cgcacagtac   840
   ggtgagcagc tgctcgcagc tggcctcaac ggcgaaaacg gcctcgctgc aatcctctcc   900
   ggcttggagt ccctggacaa cggcgaagaa atcaaggctg cattcgagaa gggcttggaa   960
   gacggcccag acctggccat ggttaactcc gctcgcggca tcaccaacct gcatgtccct  1020
   tccgatgtca tcgtggacgc ttccatgcca gcaatgattc gtacctccgg ccacatgtgg  1080
   aacaaagacg accaggagca ggacaccctg gcaatcatcc cagactcctc ctacgctggc   1140
   gtctaccaga ccgttatcga agactgccgc aagaacggcg cattcgatcc aaccaccatg   1200
   ggtaccgtcc ctaacgttgg tctgatggct cagaaggctg aagagtacgg ctcccatgac   1260
   aagaccttcc gcatcgaagc agacggtgtg gttcaggttg tttcctccaa cggcgacgtt   1320
   ctcatcgagc acgacgttga ggcaaatgac atctggcgtg catgccaggt caaggatgcc   1380
   ccaatccagg attgggtaaa gcttgctgtc acccgctccc gtctctccgg aatgcctgca   1440
   gtgttctggt tggatccaga gcgcgcacac gaccgcaacc tggcttccct cgttgagaag   1500
   tacctggctg accacgacac cgagggcctg gacatccaga tcctctcccc tgttgaggca   1560
   acccagctct ccatcgaccg catccgccgt ggcgaggaca ccatctctgt caccggtaac   1620
   gttctgcgtg actacaacac cgacctcttc ccaatcctgg agctgggcac ctctgcaaag   1680
   atgctgtctg tcgttccttt gatggctggc ggcggactgt tcgagaccgg tgctggtgga   1740
   tctgctccta agcacgtcca gcaggttcag gaagaaaacc acctgcgttg ggattccctc   1800
   ggtgagttcc tcgcactggc tgagtccttc cgccacgagc tcaacaacaa cggcaacacc   1860
   aaggccggcg ttctggctga cgctctggac aaggcaactg agaagctgct gaacgaagag   1920
   aagtccccat cccgcaaggt tggcgagatc gacaaccgtg gctcccactt ctggctgacc   1980
   aagttctggg ctgacgagct cgctgctcag accgaggacg cagatctggc tgctaccttc   2040
   gcaccagtcg cagaagcact gaacacaggc gctgcagaca tcgatgctgc actgctcgca   2100
   gttcagggtg gagcaactga ccttggtggc tactactccc ctaacgagga gaagctcacc   2160
   aacatcatgc gcccagtcgc acagttcaac gagatcgttg acgcactgaa gaagtaa      2217
<210> 5
   <211> 1002
   <212> DNA
   <213> Artificial
<220>
   <223> Vectorinsert with Isocitrate Dehydrogenase, Corynebacterium glutamicum, GGC ATT--->GGG ATA mutation in positions 32 and 33
<400> 5
   ctcgagcgaa gacctcgcag attccgatat tccaggaacc gccatgatcg aaatcccctc 60
   agatgacgat gcacttgcca tcgagggacc ttcctccatc gatgtgaaat ggctgccccg 120
   caacggccgc aagcacggtg aattgttgat ggaaaccctg gccctccacc atgaagaaac 180
   agaagctgca gccacctccg aaggcgaact tgtgtgggag actcctgtgt tctccgccac 240
   tggcgaacag atcacagaat ccaacccacg ttcaggcgac tactactgga ttgctggcga 300
   aagtggtgtc gtgaccagca ttcgtcgatc tctagtgaaa gagaaaggcc tcgaccgttc 360
   ccaagtggca ttcatggggt attggaaaca cggcgtttcc atgcggggct gaaactgcca 420
   ccataggcgc cagcaattag tagaacactg tattctaggt agctgaacaa aagagcccat 480
   caaccaagga gactcatggc taagatcatc tggacccgca ccgacgaagc accgctgctc 540
   gcgacctact cgctgaagcc ggtcgtcgag gcatttgctg ctaccgcggg gatagaggtc 600
   gagacccggg acatttcact cgctggacgc atcctcgccc agttcccaga gcgcctcacc 660
   gaagatcaga aggtaggcaa cgcactcgca gaactcggcg agcttgctaa gactcctgaa 720
   gcaaacatca ttaagcttcc aaacatctcc gcttctgttc cacagctcaa ggctgctatt 780
   aaggaactgc aggaccaggg ctacgacatc ccagaactgc ctgataacgc caccaccgac 840
   gaggaaaaag acatcctcgc acgctacaac gctgttaagg gttccgctgt gaacccagtg 900
   ctgcgtgaag gcaactctga ccgccgcgca ccaatcgctg tcaagaactt tgttaagaag 960
   ttcccacacc gcatgggcga gtggtctgca gattccacgc gt 1002
<210> 6
   <211> 32
   <212> DNA
   <213> Artificial
<220>
   <223> oligonucleotide; Primer old 441
<400> 6
   gagtacctcg agcgaagacc tcgcagattc cg 32
<210> 7
   <211> 32
   <212> DNA
   <213> Artificial
<220>
   <223> Oligonucleotide; Primer old 442
<400> 7
   catgagacgc gtggaatctg cagaccactc gc 32
<210> 8
   <211> 24
   <212> DNA
   <213> Artificial
<220>
   <223> Oligonucleotide; Primer old 443
<400> 8
   gagactcgtg gctaagatca tctg 24
<210> 9
   <211> 24
   <212> DNA
   <213> Artificial
<220>
   <223> Oligonucleotide; Primer old 444
<400> 9
   cagatgatct tagccacgag tctc 24
<210> 10
   <211> 18
   <212> DNA
   <213> Artificial
<220>
   <223> Oligonucleotide; Primer old 447
<400> 10
   ctaccgcggg gatagagg 18
<210> 11
   <211> 18
   <212> DNA
   <213> Artificial
<220>
   <223> oligonucleotide; Primer Old 448
<400> 11
   cctctatccc cgcggtag 18
<210> 12
   <211> 20
   <212> DNA
   <213> Artificial
<220>
   <223> Oligonucleotide; Primer BK1776
<400> 12
   aacggcaggt atatgtgatg 20
<210> 13
   <211> 18
   <212> DNA
   <213> Artificial
<220>
   <223> oligonucleotide; Primer Old 450
<400> 13
   cgagtaggtc gcgagcag 18
<210> 14
   <211> 21
   <212> DNA
   <213> Artificial
<220>
   <223> oligonucleotide; Primer old 471
<400> 14
   gaatccaacc cacgttcagg c 21
<210> 15
   <211> 5293
   <212> DNA
   <213> Artificial
<220>
   <223> plasmid pClik int sacB ICDH (ATG-GTG)
<400> 15
   ctcgagcgaa gacctcgcag attccgatat tccaggaacc gccatgatcg aaatcccctc 60
   agatgacgat gcacttgcca tcgagggacc ttcctccatc gatgtgaaat ggctgccccg 120
   caacggccgc aagcacggtg aattgttgat ggaaaccctg gccctccacc atgaagaaac    180
   agaagctgca gccacctccg aaggcgaact tgtgtgggag actcctgtgt tctccgccac    240
   tggcgaacag atcacagaat ccaacccacg ttcaggcgac tactactgga ttgctggcga    300
   aagtggtgtc gtgaccagca ttcgtcgatc tctagtgaaa gagaaaggcc tcgaccgttc    360
   ccaagtggca ttcatggggt attggaaaca cggcgtttcc atgcggggct gaaactgcca    420
   ccataggcgc cagcaattag tagaacactg tattctaggt agctgaacaa aagagcccat    480
   caaccaagga gactcgtggc taagatcatc tggacccgca ccgacgaagc accgctgctc    540
   gcgacctact cgctgaagcc ggtcgtcgag gcatttgctg ctaccgcggg cattgaggtc    600
   gagacccggg acatttcact cgctggacgc atcctcgccc agttcccaga gcgcctcacc    660
   gaagatcaga aggtaggcaa cgcactcgca gaactcggcg agcttgctaa gactcctgaa    720
   gcaaacatca ttaagcttcc aaacatctcc gcttctgttc cacagctcaa ggctgctatt    780
   aaggaactgc aggaccaggg ctacgacatc ccagaactgc ctgataacgc caccaccgac    840
   gaggaaaaag acatcctcgc acgctacaac gctgttaagg gttccgctgt gaacccagtg    900
   ctgcgtgaag gcaactctga ccgccgcgca ccaatcgctg tcaagaactt tgttaagaag    960
   ttcccacacc gcatgggcga gtggtctgca gattccacgc gtcatatgac tagttcggac   1020
   ctagggatat cgtcgacatc gatgctcttc tgcgttaatt aacaattggg atcctctaga   1080
   cccgggattt aaatgatccg ctagcgggct gctaaaggaa gcggaacacg tagaaagcca   1140
   gtccgcagaa acggtgctga ccccggatga atgtcagcta ctgggctatc tggacaaggg   1200
   aaaacgcaag cgcaaagaga aagcaggtag cttgcagtgg gcttacatgg cgatagctag   1260
   actgggcggt tttatggaca gcaagcgaac cggaattgcc agctggggcg ccctctggta   1320
   aggttgggaa gccctgcaaa gtaaactgga tggctttctt gccgccaagg atctgatggc   1380
   gcaggggatc aagatctgat caagagacag gatgaggatc gtttcgcatg attgaacaag   1440
   atggattgca cgcaggttct ccggccgctt gggtggagag gctattcggc tatgactggg   1500
   cacaacagac aatcggctgc tctgatgccg ccgtgttccg gctgtcagcg caggggcgcc   1560
   cggttctttt tgtcaagacc gacctgtccg gtgccctgaa tgaactgcag gacgaggcag   1620
   cgcggctatc gtggctggcc acgacgggcg ttccttgcgc agctgtgctc gacgttgtca   1680
   ctgaagcggg aagggactgg ctgctattgg gcgaagtgcc ggggcaggat ctcctgtcat   1740
   ctcaccttgc tcctgccgag aaagtatcca tcatggctga tgcaatgcgg cggctgcata   1800
   cgcttgatcc ggctacctgc ccattcgacc accaagcgaa acatcgcatc gagcgagcac   1860
   gtactcggat ggaagccggt cttgtcgatc aggatgatct ggacgaagag catcaggggc   1920
   tcgcgccagc cgaactgttc gccaggctca aggcgcgcat gcccgacggc gaggatctcg   1980
   tcgtgaccca tggcgatgcc tgcttgccga atatcatggt ggaaaatggc cgcttttctg   2040
   gattcatcga ctgtggccgg ctgggtgtgg cggaccgcta tcaggacata gcgttggcta   2100
   cccgtgatat tgctgaagag cttggcggcg aatgggctga ccgcttcctc gtgctttacg   2160
   gtatcgccgc tcccgattcg cagcgcatcg ccttctatcg ccttcttgac gagttcttct 2220
   gagcgggact ctggggttcg aaatgaccga ccaagcgacg cccaacctgc catcacgaga 2280
   tttcgattcc accgccgcct tctatgaaag gttgggcttc ggaatcgttt tccgggacgc 2340
   cggctggatg atcctccagc gcggggatct catgctggag ttcttcgccc acgctagcgg 2400
   cgcgccggcc ggcccggtgt gaaataccgc acagatgcgt aaggagaaaa taccgcatca 2460
   ggcgctcttc cgcttcctcg ctcactgact cgctgcgctc ggtcgttcgg ctgcggcgag 2520
   cggtatcagc tcactcaaag gcggtaatac ggttatccac agaatcaggg gataacgcag 2580
   gaaagaacat gtgagcaaaa ggccagcaaa aggccaggaa ccgtaaaaag gccgcgttgc 2640
   tggcgttttt ccataggctc cgcccccctg acgagcatca caaaaatcga cgctcaagtc 2700
   agaggtggcg aaacccgaca ggactataaa gataccaggc gtttccccct ggaagctccc 2760
   tcgtgcgctc tcctgttccg accctgccgc ttaccggata cctgtccgcc tttctccctt 2820
   cgggaagcgt ggcgctttct catagctcac gctgtaggta tctcagttcg gtgtaggtcg 2880
   ttcgctccaa gctgggctgt gtgcacgaac cccccgttca gcccgaccgc tgcgccttat 2940
   ccggtaacta tcgtcttgag tccaacccgg taagacacga cttatcgcca ctggcagcag 3000
   ccactggtaa caggattagc agagcgaggt atgtaggcgg tgctacagag ttcttgaagt 3060
   ggtggcctaa ctacggctac actagaagga cagtatttgg tatctgcgct ctgctgaagc 3120
   cagttacctt cggaaaaaga gttggtagct cttgatccgg caaacaaacc accgctggta 3180
   gcggtggttt ttttgtttgc aagcagcaga ttacgcgcag aaaaaaagga tctcaagaag 3240
   atcctttgat cttttctacg gggtctgacg ctcagtggaa cgaaaactca cgttaaggga 3300
   ttttggtcat gagattatca aaaaggatct tcacctagat ccttttaaag gccggccgcg 3360
   gccgccatcg gcattttctt ttgcgttttt atttgttaac tgttaattgt ccttgttcaa 3420
   ggatgctgtc tttgacaaca gatgttttct tgcctttgat gttcagcagg aagctcggcg 3480
   caaacgttga ttgtttgtct gcgtagaatc ctctgtttgt catatagctt gtaatcacga 3540
   cattgtttcc tttcgcttga ggtacagcga agtgtgagta agtaaaggtt acatcgttag 3600
   gatcaagatc catttttaac acaaggccag ttttgttcag cggcttgtat gggccagtta 3660
   aagaattaga aacataacca agcatgtaaa tatcgttaga cgtaatgccg tcaatcgtca 3720
   tttttgatcc gcgggagtca gtgaacaggt accatttgcc gttcatttta aagacgttcg 3780
   cgcgttcaat ttcatctgtt actgtgttag atgcaatcag cggtttcatc acttttttca 3840
   gtgtgtaatc atcgtttagc tcaatcatac cgagagcgcc gtttgctaac tcagccgtgc 3900
   gttttttatc gctttgcaga agtttttgac tttcttgacg gaagaatgat gtgcttttgc 3960
   catagtatgc tttgttaaat aaagattctt cgccttggta gccatcttca gttccagtgt 4020
   ttgcttcaaa tactaagtat ttgtggcctt tatcttctac gtagtgagga tctctcagcg 4080
   tatggttgtc gcctgagctg tagttgcctt catcgatgaa ctgctgtaca ttttgatacg 4140
   tttttccgtc accgtcaaag attgatttat aatcctctac accgttgatg ttcaaagagc 4200
   tgtctgatgc tgatacgtta acttgtgcag ttgtcagtgt ttgtttgccg taatgtttac 4260
   cggagaaatc agtgtagaat aaacggattt ttccgtcaga tgtaaatgtg gctgaacctg 4320
   accattcttg tgtttggtct tttaggatag aatcatttgc atcgaatttg tcgctgtctt 4380
   taaagacgcg gccagcgttt ttccagctgt caatagaagt ttcgccgact ttttgataga 4440
   acatgtaaat cgatgtgtca tccgcatttt taggatctcc ggctaatgca aagacgatgt 4500
   ggtagccgtg atagtttgcg acagtgccgt cagcgttttg taatggccag ctgtcccaaa 4560
   cgtccaggcc ttttgcagaa gagatatttt taattgtgga cgaatcaaat tcagaaactt 4620
   gatatttttc atttttttgc tgttcaggga tttgcagcat atcatggcgt gtaatatggg 4680
   aaatgccgta tgtttcctta tatggctttt ggttcgtttc tttcgcaaac gcttgagttg 4740
   cgcctcctgc cagcagtgcg gtagtaaagg ttaatactgt tgcttgtttt gcaaactttt 4800
   tgatgttcat cgttcatgtc tcctttttta tgtactgtgt tagcggtctg cttcttccag 4860
   ccctcctgtt tgaagatggc aagttagtta cgcacaataa aaaaagacct aaaatatgta 4920
   aggggtgacg ccaaagtata cactttgccc tttacacatt ttaggtcttg cctgctttat 4980
   cagtaacaaa cccgcgcgat ttacttttcg acctcattct attagactct cgtttggatt 5040
   gcaactggtc tattttcctc ttttgtttga tagaaaatca taaaaggatt tgcagactac 5100
   gggcctaaag aactaaaaaa tctatctgtt tcttttcatt ctctgtattt tttatagttt 5160
   ctgttgcatg ggcataaagt tgccttttta atcacaattc agaaaatatc ataatatctc 5220
   atttcactaa ataatagtga acggcaggta tatgtgatgg gttaaaaagg atcggcggcc 5280
   gctcgattta aat 5293
<210> 16
   <211> 906
   <212> DNA
   <213> Corynebacterium glutamicum
<400> 16
   atgagcacag gtttaacagc taagaccgga gtagagcact tcggcaccgt tggagtagca 60
   atggttactc cattcacgga atccggagac atcgatatcg ctgctggccg cgaagtcgcg 120
   gcttatttgg ttgataaggg cttggattct ttggttctcg cgggcaccac tggtgaatcc 180
   ccaacgacaa ccgccgctga aaaactagaa ctgctcaagg ccgttcgtga ggaagttggg 240
   gatcgggcga agctcatcgc cggtgtcgga accaacaaca cgcggacatc tgtggaactt 300
   gcggaagctg ctgcttctgc tggcgcagac ggccttttag ttgtaactcc ttattactcc 360
   aagccgagcc aagagggatt gctggcgcac ttcggtgcaa ttgctgcagc aacagaggtt 420
   ccaatttgtc tctatgacat tcctggtcgg tcaggtattc caattgagtc tgataccatg 480
   agacgcctga gtgaattacc tacgattttg gcggtcaagg acgccaaggg tgacctcgtt 540
   gcagccacgt cattgatcaa agaaacggga cttgcctggt attcaggcga tgacccacta 600
   aaccttgttt ggcttgcttt gggcggatca ggtttcattt ccgtaattgg acatgcagcc 660
   cccacagcat tacgtgagtt gtacacaagc ttcgaggaag gcgacctcgt ccgtgcgcgg 720
   gaaatcaacg ccaaactatc accgctggta gctgcccaag gtcgcttggg tggagtcagc 780
   ttggcaaaag ctgctctgcg tctgcagggc atcaacgtag gagatcctcg acttccaatt 840
   atggctccaa atgagcagga acttgaggct ctccgagaag acatgaaaaa agctggagtt 900
   ctataa 906
<210> 17
   <211> 906
   <212> DNA
   <213> Artificial
<220>
   <223> optimized dapA of Corynebacterium glutamicum
<400> 17
   atgagcacag ggttaacagc taagacaggg gtagagcact tcgggaccgt tggggtagca 60
   atggttactc cattcacgga atccggagac atcgatatcg ctgctggccg cgaagtcgcg 120
   gcttatttgg ttgataaggg cttggattct ttggttctcg cgggcaccac tggtgaatcc 180
   ccaacgacaa ccgccgctga aaaactagaa ctgctcaagg ccgttcgtga ggaagttggg 240
   gatcgggcga agctcatcgc cggtgtcgga accaacaaca cgcggacatc tgtggaactt 300
   gcggaagctg ctgcttctgc tggcgcagac ggccttttag ttgtaactcc ttattactcc 360
   aagccgagcc aagagggatt gctggcgcac ttcggtgcaa ttgctgcagc aacagaggtt 420
   ccaatttgtc tctatgacat tcctggtcgg tcaggtattc caattgagtc tgataccatg 480
   agacgcctga gtgaattacc tacgattttg gcggtcaagg acgccaaggg tgacctcgtt 540
   gcagccacgt cattgatcaa agaaacggga cttgcctggt attcaggcga tgacccacta 600
   aaccttgttt ggcttgcttt gggcggatca ggtttcattt ccgtaattgg acatgcagcc 660
   cccacagcat tacgtgagtt gtacacaagc ttcgaggaag gcgacctcgt ccgtgcgcgg 720
   gaaatcaacg ccaaactatc accgctggta gctgcccaag gtcgcttggg tggagtcagc 780
   ttggcaaaag ctgctctgcg tctgcagggc atcaacgtag gagatcctcg acttccaatt 840
   atggctccaa atgagcagga acttgaggct ctccgagaag acatgaaaaa agctggagtt 900
   ctataa 906
<210> 18
   <211> 8438
   <212> DNA
   <213> Artificial
<220>
   <223> plasmid pK19 PSOD ask
<400> 18
   aattcgccct tcaccgcggc tttggacatc actgctacgt agccaaacaa tgcacccgtc 60
   acaagaccaa ggatgagggc tttgtccttc tttaatacgt attccgcaag cagccacatt 120
   ccacccatta ctgcaacgcc gactaaaagt actggaatcc atcgatcgag tgggggtggg 180
   ggtttccggg aagggggcgt cccaaaacga tcatgatgcc cacggctacg gtgaggaggg 240
   tagcccagaa gatttcagtt cggcgtagtc ggtagccatt gaatcgtgct gagagcggca 300
   gcgtgaacat cagcgacagg acaagcactg gttgcactac caagagggtg ccgaaaccaa 360
   gtgctactgt ttgtaagaaa tatgccagca tcgcggtact catgcctgcc caccacatcg 420
   gtgtcatcag agcattgagt aaaggtgagc tccttaggga gccatctttt ggggtgcgga 480
   gcgcgatccg gtgtctgacc acggtgcccc atgcgattgt taatgccgat gctagggcga 540
   aaagcacggc gagcagattg ctttgcactt gattcagggt agttgactaa agagttgctc 600
   gcgaagtagc acctgtcact tttgtctcaa atattaaatc gaatatcaat atatggtctg 660
   tttattggaa cgcgtcccag tggctgagac gcatccgcta aagccccagg aagggcgaat 720
   tctgcagata tccatcacac tggcggccgc tcgagcatgc atctagctta tcgccattcg 780
   ccattcaggc tgcgcaactg ttgggaaggg cgatcggtgc gggcctcttc gctattacgc 840
   cagctggcga aagggggatg tgctgcaagg cgattaagtt gggtaacgcc agggttttcc 900
   cagtcacgac gttgtaaaac gacggccagt gaattccgtg gcacggaaat cgaggtagaa 960
   gacattactc aggcaaccga aagggcgaat tccgtggcac ggaaatcgag gtagaagaca 1020
   ttactcaggc aaccgaaagg gcgaattcgc ccttgcggcg caggattttc taaaacagga 1080
   tgcctgccac ctttaagcgc ctcatcagcg gtaaccatca cgggttcggg tgcgaaaaac 1140
   catgccataa caggaatgtt cctttcgaaa attgaggaag ccttatgccc ttcaacccta 1200
   cttagctgcc aattattccg ggcttgtgac ccgctacccg ataaataggt cggctgaaaa 1260
   atttcgttgc aatataaaca aaaaggccta tcattgggag gtgtcgcacc aagtactttt 1320
   gcgaagcgcc atctgacgga ttttcaaaag atgtatatgc tcggtgcgga aacctacgaa 1380
   aggatttttt acccgtggcc ctggtcgtac agaaatatgg cggttcctcg cttgagagtg 1440
   cggaacgcat tagaaacgtc gctgaacgga tcgttgccac caagaaggct ggaaatgatg 1500
   tcgtggttgt ctgctccgca atgggagaca ccacggatga acttctagaa cttgcagcgg 1560
   cagtgaatcc cgttccgcca gctcgtgaaa tggatatgct cctgactgct ggtgagcgta 1620
   tttctaacgc tctcgtcgcc atggctattg agtcccttgc gcagaagccc aatctttcac 1680
   gggctctcag gctggtgtgc tcaccaccga gcgccacgga aacgcacgca ttgttgatgt 1740
   cactccaggt cgtgtgcgtg aagcactcga tgagggcaag atctgcattg ttgctggttt 1800
   ccagggtgtt aataaagaaa cccgcgatgt caccacgttg ggtcgtggtg gttctgacac 1860
   cactgcagtt gcgttggcag ctgctttgaa cgctgatgtg tgtgagattt actcggacgt 1920
   tgacggtgtg tataccgctg acccgcgcat cgttcctaat gcacagaagc tggaaaagct 1980
   cagcttcgaa gaaatgctgg aacttgctgc tgttggctcc aagattttgg tgctgcgcag 2040
   tgttgaatac gctcgtgcat tcaatgtgcc acttcgcgta cgctcgtctt atagtaatga 2100
   tcccggcact ttgattgccg gctctatgga ggatattcct gtggaagaag cagtccttac 2160
   cggtgtcgca accgacaagt ccgaagccaa agtaaccgtt ctgggtattt ccgataagcc 2220
   aggcgaggct gcgaaggttt tccgtgcgtt ggctgatgca gaaatcaaca ttgacatggt 2280
   tctgcagaac gtctcttctg tagaagacgg caccaccgac atcatcttca cctgccctcg 2340
   ttccgacggc cgccgcgcga tggagatctt gaagaagctt caggttcagg gcaactggac 2400
   caatgtgctt tacgacgacc aggtcggcaa agtctccctc gtgggtgctg gcatgaagtc 2460
   tcacccaggt gttaccgcag agttcatgga agctctgcgc gatgtcaacg tgaacatcga 2520
   attgatttcc acctctgaga ttcgtatttc cgtgctgatc cgtgaagatg atctggatgc 2580
   tgctgcacgt gcattgcatg agcagttcca gctgggcggc gaagacgaag ccgtcgttta 2640
   tgcaggcacc ggacgctaaa gttttaaagg agtagtttta caatgaccac catcgcagtt 2700
   gttggtgcaa ccggccaggt cggccaggtt atgcgcaccc ttttggaaga gcgcaatttc 2760
   ccagctgaca ctgttcgttt ctttgcttcc ccacgttccg caggccgtaa gattgaattc 2820
   gccctttcgg ttgcctgagt aatgtcttct acctcgattt ccgtgccacg gaattcgagc 2880
   tcggtacccg gggatcctct agagtcgacc tgcaggcatg caagcttggc gtaatcatgg 2940
   tcatagctgt ttcctgtgtg aaattgttat ccgctcacaa ttccacacaa catacgagcc 3000
   ggaagcataa agtgtaaagc ctggggtgcc taatgagtga gctaactcac attaattgcg 3060
   ttgcgctcac tgcccgcttt ccagtcggga aacctgtcgt gccagctgca ttaatgaatc 3120
   ggccaacgcg cggggagagg cggtttgcgt attgggcgct cttccgcttc ctcgctcact 3180
   gactcgctgc gctcggtcgt tcggctgcgg cgagcggtat cagctcactc aaaggcggta 3240
   atacggttat ccacagaatc aggggataac gcaggaaaga acatgtgagc aaaaggccag 3300
   caaaaggcca ggaaccgtaa aaaggccgcg ttgctggcgt ttttccatag gctccgcccc 3360
   cctgacgagc atcacaaaaa tcgacgctca agtcagaggt ggcgaaaccc gacaggacta 3420
   taaagatacc aggcgtttcc ccctggaagc tccctcgtgc gctctcctgt tccgaccctg 3480
   ccgcttaccg gatacctgtc cgcctttctc ccttcgggaa gcgtggcgct ttctcatagc 3540
   tcacgctgta ggtatctcag ttcggtgtag gtcgttcgct ccaagctggg ctgtgtgcac 3600
   gaaccccccg ttcagcccga ccgctgcgcc ttatccggta actatcgtct tgagtccaac 3660
   ccggtaagac acgacttatc gccactggca gcagccactg gtaacaggat tagcagagcg 3720
   aggtatgtag gcggtgctac agagttcttg aagtggtggc ctaactacgg ctacactaga 3780
   agaacagtat ttggtatctg cgctctgctg aagccagtta ccttcggaaa aagagttggt 3840
   agctcttgat ccggcaaaca aaccaccgct ggtagcggtg gtttttttgt ttgcaagcag 3900
   cagattacgc gcagaaaaaa aggatctcaa gaagatcctt tgatcttttc tacggggtct 3960
   gacgctcagt ggaacgaaaa ctcacgttaa gggattttgg tcatgagatt atcaaaaagg 4020
   atcttcacct agatcctttt ggggtgggcg aagaactcca gcatgagatc cccgcgctgg 4080
   aggatcatcc agccctgata gaaacagaag ccactggagc acctcaaaaa caccatcata 4140
   cactaaatca gtaagttggc agcatcaccc gacgcacttt gcgccgaata aatacctgtg 4200
   acggaagatc acttcgcaga ataaataaat cctggtgtcc ctgttgatac cgggaagccc 4260
   tgggccaact tttggcgaaa atgagacgtt gatcggcacg taagaggttc caactttcac 4320
   cataatgaaa taagatcact accgggcgta ttttttgagt tatcgagatt ttcaggagct 4380
   gatagaaaca gaagccactg gagcacctca aaaacaccat catacactaa atcagtaagt 4440
   tggcagcatc acccgacgca ctttgcgccg aataaatacc tgtgacggaa gatcacttcg 4500
   cagaataaat aaatcctggt gtccctgttg ataccgggaa gccctgggcc aacttttggc 4560
   gaaaatgaga cgttgatcgg cacgtaagag gttccaactt tcaccataat gaaataagat 4620
   cactaccggg cgtatttttt gagttatcga gattttcagg agctctttgg catcgtctct 4680
   cgcctgtccc ctcagttcag taatttcctg catttgcctg tttccagtcg gtagatattc 4740
   cacaaaacag cagggaagca gcgcttttcc gctgcataac cctgcttcgg ggtcattata 4800
   gcgatttttt cggtatatcc atcctttttc gcacgatata caggattttg ccaaagggtt 4860
   cgtgtagact ttccttggtg tatccaacgg cgtcagccgg gcaggatagg tgaagtaggc 4920
   ccacccgcga gcgggtgttc cttcttcact gtcccttatt cgcacctggc ggtgctcaac 4980
   gggaatcctg ctctgcgagg ctggccggct accgccggcg taacagatga gggcaagcgg 5040
   atggctgatg aaaccaagcc aaccaggaag ggcagcccac ctatcaaggt gtactgcctt 5100
   ccagacgaac gaagagcgat tgaggaaaag gcggcggcgg ccggcatgag cctgtcggcc 5160
   tacctgctgg ccgtcggcca gggctacaaa atcacgggcg tcgtggacta tgagcacgtc 5220
   cgcgagggcg tcccggaaaa cgattccgaa gcccaacctt tcatagaagg cggcggtgga 5280
   atcgaaatct cgtgatggca ggttgggcgt cgcttggtcg gtcatttcgc tcggtaccca 5340
   tcggcatttt cttttgcgtt taactgttaa ttgtccttgt tcaaggatgc tgtctttgac 5400
   aacagatgtt ttcttgcctt tgatgttcag caggaagctc ggcgcaaacg ttgattgttt 5460
   gtctgcgtag aatcctctgt ttgtcatata gcttgtaatc acgacattgt ttcctttcgc 5520
   ttgaggtaca gcgaagtgtg agtaagtaaa ggttacatcg ttaggatcaa gatccatttt 5580
   taacacaagg ccagttttgt tcagcggctt gtatgggcca gttaaagaat tagaaacata 5640
   accaagcatg taaatatcgt tagacgtaat gccgtcaatc gtcatttttg atccgcggga 5700
   gtcagtgaac aggtaccatt tgccgttcat tttaaagacg ttcgcgcgtt caatttcatc 5760
   tgttactgtg ttagatgcaa tcagcggttt catcactttt ttcagtgtgt aatcatcgtt 5820
   tagctcaatc ataccgagag cgccgtttgc taactcagcc gtgcgttttt tatcgctttg 5880
   cagaagtttt tgactttctt gacggaagaa tgatgtgctt ttgccatagt atgctttgtt 5940
   aaataaagat tcttcgcctt ggtagccatc ttcagttcca gtgtttgctt caaatactaa 6000
   gtatttgtgg cctttatctt ctacgtagtg aggatctctc agcgtatggt tgtcgcctga 6060
   gctgtagttg ccttcatcga tgaactgctg tacattttga tacgtttttc cgtcaccgtc 6120
   aaagattgat ttataatcct ctacaccgtt gatgttcaaa gagctgtctg atgctgatac 6180
   gttaacttgt gcagttgtca gtgtttgttt gccgtaatgt ttaccggaga aatcagtgta 6240
   gaataaacgg atttttccgt cagatgtaaa tgtggctgaa cctgaccatt cttgtgtttg 6300
   gtcttttagg atagaatcat ttgcatcgaa tttgtcgctg tctttaaaga cgcggccagc 6360
   gtttttccag ctgtcaatag aagtttcgcc gactttttga tagaacatgt aaatcgatgt 6420
   gtcatccgca tttttaggat ctccggctaa tgcaaagacg atgtggtagc cgtgatagtt 6480
   tgcgacagtg ccgtcagcgt tttgtaatgg ccagctgtcc caaacgtcca ggccttttgc 6540
   agaagagata tttttaattg tggacgaatc aaattcagga acttgatatt tttcattttt 6600
   ttgctgttca gggatttgca gcatatcatg gcgtgtaata tgggaaatgc cgtatgtttc 6660
   cttatatggc ttttggttcg tttctttcgc aaacgcttga gttgcgcctc ctgccagcag 6720
   tgcggtagta aaggttaata ctgttgcttg ttttgcaaac tttttgatgt tcatcgttca 6780
   tgtctccttt tttatgtact gtgttagcgg tctgcttctt ccagccctcc tgtttgaaga 6840
   tggcaagtta gttacgcaca ataaaaaaag acctaaaata tgtaaggggt gacgccaaag 6900
   tatacacttt gccctttaca cattttaggt cttgcctgct ttatcagtaa caaacccgcg 6960
   cgatttactt ttcgacctca ttctattaga ctctcgtttg gattgcaact ggtctatttt 7020
   cctcttttgt ttgatagaaa atcataaaag gatttgcaga ctacgggcct aaagaactaa 7080
   aaaatctatc tgtttctttt cattctctgt attttttata gtttctgttg catgggcata 7140
   aagttgcctt tttaatcaca attcagaaaa tatcataata tctcatttca ctaaataata 7200
   gtgaacggca ggtatatgtg atgggttaaa aaggatcgat cctctagcga accccagagt 7260
   cccgctcaga agaactcgtc aagaaggcga tagaaggcga tgcgctgcga atcgggagcg 7320
   gcgataccgt aaagcacgag gaagcggtca gcccattcgc cgccaagctc ttcagcaata 7380
   tcacgggtag ccaacgctat gtcctgatag cggtccgcca cacccagccg gccacagtcg 7440
   atgaatccag aaaagcggcc attttccacc atgatattcg gcaagcaggc atcgccatgg 7500
   gtcacgacga gatcctcgcc gtcgggcatc cgcgccttga gcctggcgaa cagttcggct 7560
   ggcgcgagcc cctgatgctc ttcgtccaga tcatcctgat cgacaagacc ggcttccatc 7620
   cgagtacgtg ctcgctcgat gcgatgtttc gcttggtggt cgaatgggca ggtagccgga 7680
   tcaagcgtat gcagccgccg cattgcatca gccatgatgg atactttctc ggcaggagca 7740
   aggtgagatg acaggagatc ctgccccggc acttcgccca atagcagcca gtcccttccc 7800
   gcttcagtga caacgtcgag cacagctgcg caaggaacgc ccgtcgtggc cagccacgat 7860
   agccgcgctg cctcgtcttg gagttcattc agggcaccgg acaggtcggt cttgacaaaa 7920
   agaaccgggc gcccctgcgc tgacagccgg aacacggcgg catcagagca gccgattgtc 7980
   tgttgtgccc agtcatagcc gaatagcctc tccacccaag cggccggaga acctgcgtgc 8040
   aatccatctt gttcaatcat gcgaaacgat cctcatcctg tctcttgatc agatcttgat 8100
   cccctgcgcc atcagatcct tggcggcaag aaagccatcc agtttacttt gcagggcttc 8160
   ccaaccttac cagagggcgc cccagctggc aattccggtt cgcttgctgt ccataaaacc 8220
   gcccagtcta gctatcgcca tgtaagccca ctgcaagcta cctgctttct ctttgcgctt 8280
   gcgttttccc ttgtccagat agcccagtag ctgacattca tccggggtca gcaccgtttc 8340
   tgcggactgg ctttctacgt gttccgcttc ctttagcagc ccttgcgccc tgagtgcttg 8400
   cggcagcgtg aagctagtaa cggccgccag tgtgctgg 8438
<210> 19
   <211> 5685
   <212> DNA
   <213> Artificial
<220>
   <223> plasmid pCLIK Int Psod Glucose-6-PDH
<400> 19
   cgcgtttgcc caaatagtgg tcgatgcgga acacagaaga ttctgggaag actgcgttga 60
   ccagctggtt gagctcgtgt gcggattcga ggttgtggcc gaaaggcttc tcgatgatca 120
   cgcggcgcca tgcttcttcg gtggattcag ccatgccgga acgctccagc tggtggcaga 180
   ccgctgtgaa ggaatctggt ggaatggaca ggtagtaagc ccagttgccg gcggtgccgc 240
   gggttttgtc gatgcgcttg agtgttgcag cgaggttgtc gaaagctgca tcatcatcaa 300
   agttgccgcg aacaaattcc ataccctcgg cgaggcgctc ccaaacattt tcacggaatt 360
   ccgtacgagc accagcactt gcggcatcgc gtacgtattt ttcaaagtct tctttggacc 420
   attcgcggcg gccgtaacct accaacgaga atcctggggg cagcaatccg cggtttgcta 480
   gatcataaat ggcggggagc agcttctttc gagccaagtc gccagtgaca ccgaagatca 540
   ccatgccgga agggccagcg atgcggggga gtcgtttatc ctgcgggtcg cgcagtgggt 600
   ttgtccagct ggagggggtc gtgtttgtgc tcacgggtaa aaaatccttt cgtaggtttc 660
   cgcaccgagc atatacatct tttgaaaatc cgtcagatgg cgcttcgcaa aagtacttgg 720
   tgcgacacct cccaatgata ggcctttttg ttgatattgc aacgaaattt ttcagccgac 780
   ctatttatcg ggtagcgggt cacaagcccg gaataattgg cagctagatg gtagtgtcac 840
   gatcctttct ttaatgaaag atgtgtaacg gccacataag atcgaactaa ttcgatttca 900
   tgtcgccgtt actgatgcag cgtgctgatt ctacttcaga cgagcttcca tggactcaag 960
   cagttcgctc caagaagcaa cgaacttgtc cacaccctcg gtctccagga cctggaagac 1020
   atctgccaag tcaacgccca gagcctcaag ctgggagaac acagcgtcag cttctgccgc 1080
   ggagttggac agggtgtcac cgtgcaggtt gccctgctcc agaaccgcgt cgatggtgcc 1140
   ttctggcatg gtgttgacgg tgtttggacc agccagctcg gaaacgtaaa gagttgcagc 1200
   gtacgcaggg ttcttcacgc cggtggatgc ccacagtggg cgctgagtgt tggcaccttc 1260
   aggcagctcg gcggcgtcga aaagctcctt gtacacagcg taagcgcgct gagcgttggc 1320
   aacgcctgcc ttgccgcgca gagccaaagc ctcatcggat ccgattgcct cgaggcgctt 1380
   gtcgatctca acgtcgacat cgatgctctt ctgcgttaat taacaattgg gatcctctag 1440
   acccgggatt taaatgatcc gctagcgggc tgctaaagga agcggaacac gtagaaagcc 1500
   agtccgcaga aacggtgctg accccggatg aatgtcagct actgggctat ctggacaagg 1560
   gaaaacgcaa gcgcaaagag aaagcaggta gcttgcagtg ggcttacatg gcgatagcta 1620
   gactgggcgg ttttatggac agcaagcgaa ccggaattgc cagctggggc gccctctggt 1680
   aaggttggga agccctgcaa agtaaactgg atggctttct tgccgccaag gatctgatgg 1740
   cgcaggggat caagatctga tcaagagaca ggatgaggat cgtttcgcat gattgaacaa 1800
   gatggattgc acgcaggttc tccggccgct tgggtggaga ggctattcgg ctatgactgg 1860
   gcacaacaga caatcggctg ctctgatgcc gccgtgttcc ggctgtcagc gcaggggcgc 1920
   ccggttcttt ttgtcaagac cgacctgtcc ggtgccctga atgaactgca ggacgaggca 1980
   gcgcggctat cgtggctggc cacgacgggc gttccttgcg cagctgtgct cgacgttgtc 2040
   actgaagcgg gaagggactg gctgctattg ggcgaagtgc cggggcagga tctcctgtca 2100
   tctcaccttg ctcctgccga gaaagtatcc atcatggctg atgcaatgcg gcggctgcat 2160
   acgcttgatc cggctacctg cccattcgac caccaagcga aacatcgcat cgagcgagca 2220
   cgtactcgga tggaagccgg tcttgtcgat caggatgatc tggacgaaga gcatcagggg 2280
   ctcgcgccag ccgaactgtt cgccaggctc aaggcgcgca tgcccgacgg cgaggatctc 2340
   gtcgtgaccc atggcgatgc ctgcttgccg aatatcatgg tggaaaatgg ccgcttttct 2400
   ggattcatcg actgtggccg gctgggtgtg gcggaccgct atcaggacat agcgttggct 2460
   acccgtgata ttgctgaaga gcttggcggc gaatgggctg accgcttcct cgtgctttac 2520
   ggtatcgccg ctcccgattc gcagcgcatc gccttctatc gccttcttga cgagttcttc 2580
   tgagcgggac tctggggttc gaaatgaccg accaagcgac gcccaacctg ccatcacgag 2640
   atttcgattc caccgccgcc ttctatgaaa ggttgggctt cggaatcgtt ttccgggacg 2700
   ccggctggat gatcctccag cgcggggatc tcatgctgga gttcttcgcc cacgctagcg 2760
   gcgcgccggc cggcccggtg tgaaataccg cacagatgcg taaggagaaa ataccgcatc 2820
   aggcgctctt ccgcttcctc gctcactgac tcgctgcgct cggtcgttcg gctgcggcga 2880
   gcggtatcag ctcactcaaa ggcggtaata cggttatcca cagaatcagg ggataacgca 2940
   ggaaagaaca tgtgagcaaa aggccagcaa aaggccagga accgtaaaaa ggccgcgttg 3000
   ctggcgtttt tccataggct ccgcccccct gacgagcatc acaaaaatcg acgctcaagt 3060
   cagaggtggc gaaacccgac aggactataa agataccagg cgtttccccc tggaagctcc 3120
   ctcgtgcgct ctcctgttcc gaccctgccg cttaccggat acctgtccgc ctttctccct 3180
   tcgggaagcg tggcgctttc tcatagctca cgctgtaggt atctcagttc ggtgtaggtc 3240
   gttcgctcca agctgggctg tgtgcacgaa ccccccgttc agcccgaccg ctgcgcctta 3300
   tccggtaact atcgtcttga gtccaacccg gtaagacacg acttatcgcc actggcagca 3360
   gccactggta acaggattag cagagcgagg tatgtaggcg gtgctacaga gttcttgaag 3420
   tggtggccta actacggcta cactagaagg acagtatttg gtatctgcgc tctgctgaag 3480
   ccagttacct tcggaaaaag agttggtagc tcttgatccg gcaaacaaac caccgctggt 3540
   agcggtggtt tttttgtttg caagcagcag attacgcgca gaaaaaaagg atctcaagaa 3600
   gatcctttga tcttttctac ggggtctgac gctcagtgga acgaaaactc acgttaaggg 3660
   attttggtca tgagattatc aaaaaggatc ttcacctaga tccttttaaa ggccggccgc 3720
   ggccgccatc ggcattttct tttgcgtttt tatttgttaa ctgttaattg tccttgttca 3780
   aggatgctgt ctttgacaac agatgttttc ttgcctttga tgttcagcag gaagctcggc 3840
   gcaaacgttg attgtttgtc tgcgtagaat cctctgtttg tcatatagct tgtaatcacg 3900
   acattgtttc ctttcgcttg aggtacagcg aagtgtgagt aagtaaaggt tacatcgtta 3960
   ggatcaagat ccatttttaa cacaaggcca gttttgttca gcggcttgta tgggccagtt 4020
   aaagaattag aaacataacc aagcatgtaa atatcgttag acgtaatgcc gtcaatcgtc 4080
   atttttgatc cgcgggagtc agtgaacagg taccatttgc cgttcatttt aaagacgttc 4140
   gcgcgttcaa tttcatctgt tactgtgtta gatgcaatca gcggtttcat cacttttttc 4200
   agtgtgtaat catcgtttag ctcaatcata ccgagagcgc cgtttgctaa ctcagccgtg 4260
   cgttttttat cgctttgcag aagtttttga ctttcttgac ggaagaatga tgtgcttttg 4320
   ccatagtatg ctttgttaaa taaagattct tcgccttggt agccatcttc agttccagtg 4380
   tttgcttcaa atactaagta tttgtggcct ttatcttcta cgtagtgagg atctctcagc 4440
   gtatggttgt cgcctgagct gtagttgcct tcatcgatga actgctgtac attttgatac 4500
   gtttttccgt caccgtcaaa gattgattta taatcctcta caccgttgat gttcaaagag 4560
   ctgtctgatg ctgatacgtt aacttgtgca gttgtcagtg tttgtttgcc gtaatgttta 4620
   ccggagaaat cagtgtagaa taaacggatt tttccgtcag atgtaaatgt ggctgaacct 4680
   gaccattctt gtgtttggtc ttttaggata gaatcatttg catcgaattt gtcgctgtct 4740
   ttaaagacgc ggccagcgtt tttccagctg tcaatagaag tttcgccgac tttttgatag 4800
   aacatgtaaa tcgatgtgtc atccgcattt ttaggatctc cggctaatgc aaagacgatg 4860
   tggtagccgt gatagtttgc gacagtgccg tcagcgtttt gtaatggcca gctgtcccaa 4920
   acgtccaggc cttttgcaga agagatattt ttaattgtgg acgaatcaaa ttcagaaact 4980
   tgatattttt catttttttg ctgttcaggg atttgcagca tatcatggcg tgtaatatgg 5040
   gaaatgccgt atgtttcctt atatggcttt tggttcgttt ctttcgcaaa cgcttgagtt 5100
   gcgcctcctg ccagcagtgc ggtagtaaag gttaatactg ttgcttgttt tgcaaacttt 5160
   ttgatgttca tcgttcatgt ctcctttttt atgtactgtg ttagcggtct gcttcttcca 5220
   gccctcctgt ttgaagatgg caagttagtt acgcacaata aaaaaagacc taaaatatgt 5280
   aaggggtgac gccaaagtat acactttgcc ctttacacat tttaggtctt gcctgcttta 5340
   tcagtaacaa acccgcgcga tttacttttc gacctcattc tattagactc tcgtttggat 5400
   tgcaactggt ctattttcct cttttgtttg atagaaaatc ataaaaggat ttgcagacta 5460
   cgggcctaaa gaactaaaaa atctatctgt ttcttttcat tctctgtatt ttttatagtt 5520
   tctgttgcat gggcataaag ttgccttttt aatcacaatt cagaaaatat cataatatct 5580
   catttcacta aataatagtg aacggcaggt atatgtgatg ggttaaaaag gatcggcggc 5640
   cgctcgattt aaatctcgag aggcctgacg tcgggcccgg tacca 5685
<210> 20
   <211> 7632
   <212> DNA
   <213> Artificial
<220>
   <223> plasmid pK19 sacB Glu-6-P-DH
<400> 20
   cgcccttccc aacagttgcg cagcctgaat ggcgaatggc gataagctag cttcacgctg      60
   ccgcaagcac tcagggcgca agggctgcta aaggaagcgg aacacgtaga aagccagtcc     120
   gcagaaacgg tgctgacccc ggatgaatgt cagctactgg gctatctgga caagggaaaa     180
   cgcaagcgca aagagaaagc aggtagcttg cagtgggctt acatggcgat agctagactg     240
   ggcggtttta tggacagcaa gcgaaccgga attgccagct ggggcgccct ctggtaaggt     300
   tgggaagccc tgcaaagtaa actggatggc tttcttgccg ccaaggatct gatggcgcag     360
   gggatcaaga tctgatcaag agacaggatg aggatcgttt cgcatgattg aacaagatgg     420
   attgcacgca ggttctccgg ccgcttgggt ggagaggcta ttcggctatg actgggcaca     480
   acagacaatc ggctgctctg atgccgccgt gttccggctg tcagcgcagg ggcgcccggt     540
   tctttttgtc aagaccgacc tgtccggtgc cctgaatgaa ctccaagacg aggcagcgcg     600
   gctatcgtgg ctggccacga cgggcgttcc ttgcgcagct gtgctcgacg ttgtcactga     660
   agcgggaagg gactggctgc tattgggcga agtgccgggg caggatctcc tgtcatctca     720
   ccttgctcct gccgagaaag tatccatcat ggctgatgca atgcggcggc tgcatacgct     780
   tgatccggct acctgcccat tcgaccacca agcgaaacat cgcatcgagc gagcacgtac     840
   tcggatggaa gccggtcttg tcgatcagga tgatctggac gaagagcatc aggggctcgc     900
   gccagccgaa ctgttcgcca ggctcaaggc gcggatgccc gacggcgagg atctcgtcgt     960
   gacccatggc gatgcctgct tgccgaatat catggtggaa aatggccgct tttctggatt    1020
   catcgactgt ggccggctgg gtgtggcgga ccgctatcag gacatagcgt tggctacccg    1080
   tgatattgct gaagagcttg gcggcgaatg ggctgaccgc ttcctcgtgc tttacggtat    1140
   cgccgctccc gattcgcagc gcatcgcctt ctatcgcctt cttgacgagt tcttctgagc    1200
   gggactctgg ggttcgctag aggatcgatc ctttttaacc catcacatat acctgccgtt    1260
   cactattatt tagtgaaatg agatattatg atattttctg aattgtgatt aaaaaggcaa    1320
   ctttatgccc atgcaacaga aactataaaa aatacagaga atgaaaagaa acagatagat    1380
   tttttagttc tttaggcccg tagtctgcaa atccttttat gattttctat caaacaaaag    1440
   aggaaaatag accagttgca atccaaacga gagtctaata gaatgaggtc gaaaagtaaa    1500
   tcgcgcgggt ttgttactga taaagcaggc aagacctaaa atgtgtaaag ggcaaagtgt    1560
   atactttggc gtcacccctt acatatttta ggtctttttt tattgtgcgt aactaacttg    1620
   ccatcttcaa acaggagggc tggaagaagc agaccgctaa cacagtacat aaaaaaggag    1680
   acatgaacga tgaacatcaa aaagtttgca aaacaagcaa cagtattaac ctttactacc    1740
   gcactgctgg caggaggcgc aactcaagcg tttgcgaaag aaacgaacca aaagccatat 1800
   aaggaaacat acggcatttc ccatattaca cgccatgata tgctgcaaat ccctgaacag 1860
   caaaaaaatg aaaaatatca agtttctgaa tttgattcgt ccacaattaa aaatatctct 1920
   tctgcaaaag gcctggacgt ttgggacagc tggccattac aaaacgctga cggcactgtc 1980
   gcaaactatc acggctacca catcgtcttt gcattagccg gagatcctaa aaatgcggat 2040
   gacacatcga tttacatgtt ctatcaaaaa gtcggcgaaa cttctattga cagctggaaa 2100
   aacgctggcc gcgtctttaa agacagcgac aaattcgatg caaatgattc tatcctaaaa 2160
   gaccaaacac aagaatggtc aggttcagcc acatttacat ctgacggaaa aatccgttta 2220
   ttctacactg atttctccgg taaacattac ggcaaacaaa cactgacaac tgcacaagtt 2280
   aacgtatcag catcagacag ctctttgaac atcaacggtg tagaggatta taaatcaatc 2340
   tttgacggtg acggaaaaac gtatcaaaat gtacagcagt tcatcgatga aggcaactac 2400
   agctcaggcg acaaccatac gctgagagat cctcactacg tagaagataa aggccacaaa 2460
   tacttagtat ttgaagcaaa cactggaact gaagatggct accaaggcga agaatcttta 2520
   tttaacaaag catactatgg caaaagcaca tcattcttcc gtcaagaaag tcaaaaactt 2580
   ctgcaaagcg ataaaaaacg cacggctgag ttagcaaacg gcgctctcgg tatgattgag 2640
   ctaaacgatg attacacact gaaaaaagtg atgaaaccgc tgattgcatc taacacagta 2700
   acagatgaaa ttgaacgcgc gaacgtcttt aaaatgaacg gcaaatggta cctgttcact 2760
   gactcccgcg gatcaaaaat gacgattgac ggcattacgt ctaacgatat ttacatgctt 2820
   ggttatgttt ctaattcttt aactggccca tacaagccgc tgaacaaaac tggccttgtg 2880
   ttaaaaatgg atcttgatcc taacgatgta acctttactt actcacactt cgctgtacct 2940
   caagcgaaag gaaacaatgt cgtgattaca agctatatga caaacagagg attctacgca 3000
   gacaaacaat caacgtttgc gccgagcttc ctgctgaaca tcaaaggcaa gaaaacatct 3060
   gttgtcaaag acagcatcct tgaacaagga caattaacag ttaacaaata aaaacgcaaa 3120
   agaaaatgcc gatgggtacc gagcgaaatg accgaccaag cgacgcccaa cctgccatca 3180
   cgagatttcg attccaccgc cgccttctat gaaaggttgg gcttcggaat cgttttccgg 3240
   gacgccctcg cggacgtgct catagtccac gacgcccgtg attttgtagc cctggccgac 3300
   ggccagcagg taggccgaca ggctcatgcc ggccgccgcc gccttttcct caatcgctct 3360
   tcgttcgtct ggaaggcagt acaccttgat aggtgggctg cccttcctgg ttggcttggt 3420
   ttcatcagcc atccgcttgc cctcatctgt tacgccggcg gtagccggcc agcctcgcag 3480
   agcaggattc ccgttgagca ccgccaggtg cgaataaggg acagtgaaga aggaacaccc 3540
   gctcgcgggt gggcctactt cacctatcct gcccggctga cgccgttgga tacaccaagg 3600
   aaagtctaca cgaacccttt ggcaaaatcc tgtatatcgt gcgaaaaagg atggatatac 3660
   cgaaaaaatc gctataatga ccccgaagca gggttatgca gcggaaaagc gctgcttccc 3720
   tgctgttttg tggaatatct accgactgga aacaggcaaa tgcaggaaat tactgaactg 3780
   aggggacagg cgagagacga tgccaaagag ctcctgaaaa tctcgataac tcaaaaaata 3840
   cgcccggtag tgatcttatt tcattatggt gaaagttgga acctcttacg tgccgatcaa 3900
   cgtctcattt tcgccaaaag ttggcccagg gcttcccggt atcaacaggg acaccaggat 3960
   ttatttattc tgcgaagtga tcttccgtca caggtattta ttcggcgcaa agtgcgtcgg 4020
   gtgatgctgc caacttactg atttagtgta tgatggtgtt tttgaggtgc tccagtggct 4080
   tctgtttcta tcagctcctg aaaatctcga taactcaaaa aatacgcccg gtagtgatct 4140
   tatttcatta tggtgaaagt tggaacctct tacgtgccga tcaacgtctc attttcgcca 4200
   aaagttggcc cagggcttcc cggtatcaac agggacacca ggatttattt attctgcgaa 4260
   gtgatcttcc gtcacaggta tttattcggc gcaaagtgcg tcgggtgatg ctgccaactt 4320
   actgatttag tgtatgatgg tgtttttgag gtgctccagt ggcttctgtt tctatcaggg 4380
   ctggatgatc ctccagcgcg gggatctcat gctggagttc ttcgcccacc ccaaaaggat 4440
   ctaggtgaag atcctttttg ataatctcat gaccaaaatc ccttaacgtg agttttcgtt 4500
   ccactgagcg tcagaccccg tagaaaagat caaaggatct tcttgagatc ctttttttct 4560
   gcgcgtaatc tgctgcttgc aaacaaaaaa accaccgcta ccagcggtgg tttgtttgcc 4620
   ggatcaagag ctaccaactc tttttccgaa ggtaactggc ttcagcagag cgcagatacc 4680
   aaatactgtt cttctagtgt agccgtagtt aggccaccac ttcaagaact ctgtagcacc 4740
   gcctacatac ctcgctctgc taatcctgtt accagtggct gctgccagtg gcgataagtc 4800
   gtgtcttacc gggttggact caagacgata gttaccggat aaggcgcagc ggtcgggctg 4860
   aacggggggt tcgtgcacac agcccagctt ggagcgaacg acctacaccg aactgagata 4920
   cctacagcgt gagctatgag aaagcgccac gcttcccgaa gggagaaagg cggacaggta 4980
   tccggtaagc ggcagggtcg gaacaggaga gcgcacgagg gagcttccag ggggaaacgc 5040
   ctggtatctt tatagtcctg tcgggtttcg ccacctctga cttgagcgtc gatttttgtg 5100
   atgctcgtca ggggggcgga gcctatggaa aaacgccagc aacgcggcct ttttacggtt 5160
   cctggccttt tgctggcctt ttgctcacat gttctttcct gcgttatccc ctgattctgt 5220
   ggataaccgt attaccgcct ttgagtgagc tgataccgct cgccgcagcc gaacgaccga 5280
   gcgcagcgag tcagtgagcg aggaagcgga agagcgccca atacgcaaac cgcctctccc 5340
   cgcgcgttgg ccgattcatt aatgcagctg gcacgacagg tttcccgact ggaaagcggg 5400
   cagtgagcgc aacgcaatta atgtgagtta gctcactcat taggcacccc aggctttaca 5460
   ctttatgctt ccggctcgta tgttgtgtgg aattgtgagc ggataacaat ttcacacagg 5520
   aaacagctat gaccatgatt acgccaagct tgaaatttgc tgggtggtgg tatccggaag 5580
   ttcaaagatc attttttgcc cctaaattat ggcctgcgcc aggtgtgacc gttgcgggaa 5640
   agcatttcat cagcgctctt tggaccccac gtacccgctg ggtaatcctc tggttctcca 5700
   tcggcatccc atgcttcaag aattggatcc agaatcttcc agctcagttc cacttcctcg 5760
   ttggtaggga agaggctgga ttcatctaac agcgcatcca aaatgaggcg ctcgtatgct 5820
   tcaggtgatt cttcagtgaa ggattctgag taggagaagt ccatgttgac gtcacggact 5880
   tccatggcag aacctggaac cttggaaccg aagcggatga gcacaccttc atcaggctgc 5940
   acgcgaatca cgatggcgtt ttggccaagg gatacagtca tgtcgccgtc gaaaggctgg 6000
   tgtggtgcgt ctttaaacac cacggcaatc tcagtaacac ggcgaccaag acgcttaccg 6060
   gtgcgcaggt agaacggcac accagcccag cgacgagacg tgatctctaa ggtacaagcc 6120
   gcaaaagtct cagtggtgga ctcagggttg aagccatctt cttcgcgaag tcccttgact 6180
   aactcagagc cctgccaacc ggcagcgtac tgaccacgag cggaggtttt atccaatggg 6240
   tagcacggct ttgtcgcaga gagcaccttg atcttttctg cctgcagctg cgctggcacg 6300
   aaagaaattg gttcttccat ggcaaccaga gccaagagct ggatcaggtg gttctggatg 6360
   acgtcgcggg ctgcgccgat gccgtcgtag taaccagcac gtccacccaa gccaatatct 6420
   tcagtcatgg tgatctggac gtggtcaacg tagttggagt tccacagtgg ctcaaacagc 6480
   tggttagcaa aacgcagagc caggatgttt tgaactgttt ccttgcccaa atagtggtcg 6540
   atgcggaaca cagaagattc tgggaagact gcgttgacca gctggttgag ctcgtgtgcg 6600
   gattcgaggt tgtggccgaa aggcttctcg atgatcacgc ggcgccatgc ttcttcggtg 6660
   gattcagcca tgccggaacg ctccagctgg tggcagaccg ctgtgaagga atctggtgga 6720
   atggacaggt agtaagccca gttgccggcg gtgccgcggg ttttgtcgat gcgcttgagt 6780
   gttgcagcga ggttgtcgaa agctgcatca tcatcaaagt tgccgcgaac aaattccata 6840
   ccctcggcga ggcgctccca aacattttca cggaattccg tacgagcacc agcacttgcg 6900
   gcatcgcgta cgtatttttc aaagtcttct ttggaccatt cgcggcggcc gtaacctacc 6960
   aacgagaatc ctgggggcag caatccgcgg tttgctagat cataaatggc ggggagcagc 7020
   ttctttcgag ccaagtcgcc agtgacaccg aagatcacca tgccggaagg gccagcgatg 7080
   cgggggagtc gtttatcctg cgggtcgcgc agtgggtttg tccagctgga gggggtcgtg 7140
   tttgtgctca cgatggtagt gtcacgatcc tttctttaat gaaagatgtg taacggccac 7200
   ataagatcga actaattcga tttcatgtcg ccgttactga tgcagcgtgc tgattctact 7260
   tcagacgagc ttccatggac tcaagcagtt cgctccaaga agcaacgaac ttgtccacac 7320
   cctcggtctc caggacctgg aagacatctg ccaagtcaac gcccagagcc tcaagctggg 7380
   agaacacagc gtcagcttct gccgcggagt tggacagggt gtcaccgtgc aggttgccct 7440
   gctccagaac cgcgtcgatg gtgccttctg gcatggtgtt gacggtgttt ggaccagcca 7500
   gctcggaaac gtaaagagtt gcagcgtacg cagggttctt cacgccggtg gatgcccaca 7560
   gtgggcgctg agtgttggca ccttcaggca gctcggcggc gtcgaaaagc tccttgtaca 7620
   caggaattcc gg 7632
<210> 21
   <211> 7438
   <212> DNA
   <213> Artificial
<220>
   <223> plasmid pK19 PGro PycA
<400> 21
   ttatttgtta actgttaatt gtccttgttc aaggatgctg tctttgacaa cagatgtttt 60
   cttgcctttg atgttcagca ggaagctcgg cgcaaacgtt gattgtttgt ctgcgtagaa 120
   tcctctgttt gtcatatagc ttgtaatcac gacattgttt cctttcgctt gaggtacagc 180
   gaagtgtgag taagtaaagg ttacatcgtt aggatcaaga tccattttta acacaaggcc 240
   agttttgttc agcggcttgt atgggccagt taaagaatta gaaacataac caagcatgta 300
   aatatcgtta gacgtaatgc cgtcaatcgt catttttgat ccgcgggagt cagtgaacag 360
   gtaccatttg ccgttcattt taaagacgtt cgcgcgttca atttcatctg ttactgtgtt 420
   agatgcaatc agcggtttca tcactttttt cagtgtgtaa tcatcgttta gctcaatcat 480
   accgagagcg ccgtttgcta actcagccgt gcgtttttta tcgctttgca gaagtttttg 540
   actttcttga cggaagaatg atgtgctttt gccatagtat gctttgttaa ataaagattc 600
   ttcgccttgg tagccatctt cagttccagt gtttgcttca aatactaagt atttgtggcc 660
   tttatcttct acgtagtgag gatctctcag cgtatggttg tcgcctgagc tgtagttgcc 720
   ttcatcgatg aactgctgta cattttgata cgtttttccg tcaccgtcaa agattgattt 780
   ataatcctct acaccgttga tgttcaaaga gctgtctgat gctgatacgt taacttgtgc 840
   agttgtcagt gtttgtttgc cgtaatgttt accggagaaa tcagtgtaga ataaacggat 900
   ttttccgtca gatgtaaatg tggctgaacc tgaccattct tgtgtttggt cttttaggat 960
   agaatcattt gcatcgaatt tgtcgctgtc tttaaagacg cggccagcgt ttttccagct 1020
   gtcaatagaa gtttcgccga ctttttgata gaacatgtaa atcgatgtgt catccgcatt 1080
   tttaggatct ccggctaatg caaagacgat gtggtagccg tgatagtttg cgacagtgcc 1140
   gtcagcgttt tgtaatggcc agctgtccca aacgtccagg ccttttgcag aagagatatt 1200
   tttaattgtg gacgaatcaa attcaggaac ttgatatttt tcattttttt gctgttcagg 1260
   gatttgcagc atatcatggc gtgtaatatg ggaaatgccg tatgtttcct tatatggctt 1320
   ttggttcgtt tctttcgcaa acgcttgagt tgcgcctcct gccagcagtg cggtagtaaa 1380
   ggttaatact gttgcttgtt ttgcaaactt tttgatgttc atcgttcatg tctccttttt 1440
   tatgtactgt gttagcggtc tgcttcttcc agccctcctg tttgaagatg gcaagttagt 1500
   tacgcacaat aaaaaaagac ctaaaatatg taaggggtga cgccaaagta tacactttgc 1560
   cctttacaca ttttaggtct tgcctgcttt atcagtaaca aacccgcgcg atttactttt 1620
   cgacctcatt ctattagact ctcgtttgga ttgcaactgg tctattttcc tcttttgttt 1680
   gatagaaaat cataaaagga tttgcagact acgggcctaa agaactaaaa aatctatctg 1740
   tttcttttca ttctctgtat tttttatagt ttctgttgca tgggcataaa gttgcctttt 1800
   taatcacaat tcagaaaata tcataatatc tcatttcact aaataatagt gaacggcagg 1860
   tatatgtgat gggttaaaaa ggatcgatcc tctagcgaac cccagagtcc cgctcagaag 1920
   aactcgtcaa gaaggcgata gaaggcgatg cgctgcgaat cgggagcggc gataccgtaa 1980
   agcacgagga agcggtcagc ccattcgccg ccaagctctt cagcaatatc acgggtagcc 2040
   aacgctatgt cctgatagcg gtccgccaca cccagccggc cacagtcgat gaatccagaa 2100
   aagcggccat tttccaccat gatattcggc aagcaggcat cgccatgggt cacgacgaga 2160
   tcctcgccgt cgggcatccg cgccttgagc ctggcgaaca gttcggctgg cgcgagcccc 2220
   tgatgctctt cgtccagatc atcctgatcg acaagaccgg cttccatccg agtacgtgct 2280
   cgctcgatgc gatgtttcgc ttggtggtcg aatgggcagg tagccggatc aagcgtatgc 2340
   agccgccgca ttgcatcagc catgatggat actttctcgg caggagcaag gtgagatgac 2400
   aggagatcct gccccggcac ttcgcccaat agcagccagt cccttcccgc ttcagtgaca 2460
   acgtcgagca cagctgcgca aggaacgccc gtcgtggcca gccacgatag ccgcgctgcc 2520
   tcgtcttgga gttcattcag ggcaccggac aggtcggtct tgacaaaaag aaccgggcgc 2580
   ccctgcgctg acagccggaa cacggcggca tcagagcagc cgattgtctg ttgtgcccag 2640
   tcatagccga atagcctctc cacccaagcg gccggagaac ctgcgtgcaa tccatcttgt 2700
   tcaatcatgc gaaacgatcc tcatcctgtc tcttgatcag atcttgatcc cctgcgccat 2760
   cagatccttg gcggcaagaa agccatccag tttactttgc agggcttccc aaccttacca 2820
   gagggcgccc cagctggcaa ttccggttcg cttgctgtcc ataaaaccgc ccagtctagc 2880
   tatcgccatg taagcccact gcaagctacc tgctttctct ttgcgcttgc gttttccctt 2940
   gtccagatag cccagtagct gacattcatc cggggtcagc accgtttctg cggactggct 3000
   ttctacgtgt tccgcttcct ttagcagccc ttgcgccctg agtgcttgcg gcagcgtgaa 3060
   gctagatgca tgctcgagcg gccgccagtg tgatggatat ctgcagaatt cgcccttccg 3120
   gcgaagtgtc tgctcgcgtg attgtgcttc ctttggctac taacccacgc gccaagatgc 3180
   gttccctgcg ccacggtttt gtgaagctgt tctgccgccg taactctggc ctgatcatcg 3240
   gtggtgtcgt ggtggcaccg accgcgtctg agctgatcct accgatcgct gtggcagtga 3300
   ccaaccgtct gacagttgct gatctggctg ataccttcgc ggtgtaccca tcattgtcag 3360
   gttcgattac tgaagcagca cgtcagctgg ttcaacatga tgatctaggc taatttttct 3420
   gagtcttaga ttttgagaaa acccaggatt gctttgtgca ctcctgggtt ttcactttgt 3480
   taagcagttt tggggaaaag tgcaaagttt gcaaagttta gaaatatttt aagaggtaag 3540
   atgtctgcag gtggaagcgt ttaaatgcgt taaacttggc caaatgtggc aacctttgca 3600
   aggtgaaaaa ctggggcggg gtaagggcga attccagcac actggcggcc gttactagct 3660
   tatcgccatt cgccattcag gctgcgcaac tgttgggaag ggcgatcggt gcgggcctct 3720
   tcgctattac gccagctggc gaaaggggga tgtgctgcaa ggcgattaag ttgggtaacg 3780
   ccagggtttt cccagtcacg acgttgtaaa acgacggcca gtgaattcaa cctgtggcgc 3840
   aacgctgtat ataacctgcg tacggcttaa agtttggctg ccatgtgaat ttttagcacc 3900
   ctcaacagtt gagtgctggc actctcgggg gtagagtgcc aaataggttg tttgacacac 3960
   agttgttcac ccgcgacgac ggctgtgctg gaaacccaca accggcacac acaaaatttt 4020
   tctcatggag ggattcatcg tgtcgactca cacatcttca acgcttccag cattcaaaaa 4080
   gatcttggta gcaaaccgcg gcgaaatcgc ggtccgtgct ttccgtgcag cactcgaaac 4140
   cggtgcagcc acggtagcta tttacccccg tgaagatcgg ggatcattcc accgctcttt 4200
   tgcttctgaa gctgtccgca ttggtaccga aggctcacca gtcaaggcgt acctggacat 4260
   cgatgaaatt atcggtgcag ctaaaaaagt taaagcagat gccatttacc cgggatacgg 4320
   cttcctgtct gaaaatgccc agcttgcccg cgagtgtgcg gaaaacggca ttacttttat 4380
   tggcccaacc ccagaggttc ttgatctcac cggtgataag tctcgcgcgg taaccgccgc 4440
   gaagaaggct ggtctgccag ttttggcgga atccaccccg agcaaaaaca tcgatgagat 4500
   cgttaaaagc gctgaaggcc agacttaccc catctttgtg aaggcagttg ccggtggtgg 4560
   cggacgcggt atgcgttttg ttgcttcacc tgatgagctt cgcaaattag caacagaagc 4620
   atctcgtgaa gctgaagcgg ctttcggcga tggcgcggta tatgtcgaac gtgctgtgat 4680
   taaccctcag catattgaag tgcagatcct tggcgatcac actggagaag ttgtacacct 4740
   ttatgaacgt gactgctcac tgcagcgtcg tcaccaaaaa gttgtcgaaa ttgcgccagc 4800
   acagcatttg gatccagaac tgcgtgatcg catttgtgcg gatgcagtaa agttctgccg 4860
   ctccattggt taccagggcg cgggaaccaa gggcgaattc ctctggataa tcatcgcggt 4920
   agttacgagc ggcgcgaatg caagggcgaa ttcgagctcg gtacccgggg atcctctaga 4980
   gtcgacctgc aggcatgcaa gcttggcgta atcatggtca tagctgtttc ctgtgtgaaa 5040
   ttgttatccg ctcacaattc cacacaacat acgagccgga agcataaagt gtaaagcctg 5100
   gggtgcctaa tgagtgagct aactcacatt aattgcgttg cgctcactgc ccgctttcca 5160
   gtcgggaaac ctgtcgtgcc agctgcatta atgaatcggc caacgcgcgg ggagaggcgg 5220
   tttgcgtatt gggcgctctt ccgcttcctc gctcactgac tcgctgcgct cggtcgttcg 5280
   gctgcggcga gcggtatcag ctcactcaaa ggcggtaata cggttatcca cagaatcagg 5340
   ggataacgca ggaaagaaca tgtgagcaaa aggccagcaa aaggccagga accgtaaaaa 5400
   ggccgcgttg ctggcgtttt tccataggct ccgcccccct gacgagcatc acaaaaatcg 5460
   acgctcaagt cagaggtggc gaaacccgac aggactataa agataccagg cgtttccccc 5520
   tggaagctcc ctcgtgcgct ctcctgttcc gaccctgccg cttaccggat acctgtccgc 5580
   ctttctccct tcgggaagcg tggcgctttc tcatagctca cgctgtaggt atctcagttc 5640
   ggtgtaggtc gttcgctcca agctgggctg tgtgcacgaa ccccccgttc agcccgaccg 5700
   ctgcgcctta tccggtaact atcgtcttga gtccaacccg gtaagacacg acttatcgcc 5760
   actggcagca gccactggta acaggattag cagagcgagg tatgtaggcg gtgctacaga 5820
   gttcttgaag tggtggccta actacggcta cactagaaga acagtatttg gtatctgcgc 5880
   tctgctgaag ccagttacct tcggaaaaag agttggtagc tcttgatccg gcaaacaaac 5940
   caccgctggt agcggtggtt tttttgtttg caagcagcag attacgcgca gaaaaaaagg 6000
   atctcaagaa gatcctttga tcttttctac ggggtctgac gctcagtgga acgaaaactc 6060
   acgttaaggg attttggtca tgagattatc aaaaaggatc ttcacctaga tccttttggg 6120
   gtgggcgaag aactccagca tgagatcccc gcgctggagg atcatccagc cctgatagaa 6180
   acagaagcca ctggagcacc tcaaaaacac catcatacac taaatcagta agttggcagc 6240
   atcacccgac gcactttgcg ccgaataaat acctgtgacg gaagatcact tcgcagaata 6300
   aataaatcct ggtgtccctg ttgataccgg gaagccctgg gccaactttt ggcgaaaatg 6360
   agacgttgat cggcacgtaa gaggttccaa ctttcaccat aatgaaataa gatcactacc 6420
   gggcgtattt tttgagttat cgagattttc aggagctgat agaaacagaa gccactggag 6480
   cacctcaaaa acaccatcat acactaaatc agtaagttgg cagcatcacc cgacgcactt 6540
   tgcgccgaat aaatacctgt gacggaagat cacttcgcag aataaataaa tcctggtgtc 6600
   cctgttgata ccgggaagcc ctgggccaac ttttggcgaa aatgagacgt tgatcggcac 6660
   gtaagaggtt ccaactttca ccataatgaa ataagatcac taccgggcgt attttttgag 6720
   ttatcgagat tttcaggagc tctttggcat cgtctctcgc ctgtcccctc agttcagtaa 6780
   tttcctgcat ttgcctgttt ccagtcggta gatattccac aaaacagcag ggaagcagcg 6840
   cttttccgct gcataaccct gcttcggggt cattatagcg attttttcgg tatatccatc 6900
   ctttttcgca cgatatacag gattttgcca aagggttcgt gtagactttc cttggtgtat 6960
   ccaacggcgt cagccgggca ggataggtga agtaggccca cccgcgagcg ggtgttcctt 7020
   cttcactgtc ccttattcgc acctggcggt gctcaacggg aatcctgctc tgcgaggctg 7080
   gccggctacc gccggcgtaa cagatgaggg caagcggatg gctgatgaaa ccaagccaac 7140
   caggaagggc agcccaccta tcaaggtgta ctgccttcca gacgaacgaa gagcgattga 7200
   ggaaaaggcg gcggcggccg gcatgagcct gtcggcctac ctgctggccg tcggccaggg 7260
   ctacaaaatc acgggcgtcg tggactatga gcacgtccgc gagggcgtcc cggaaaacga 7320
   ttccgaagcc caacctttca tagaaggcgg cggtggaatc gaaatctcgt gatggcaggt 7380
   tgggcgtcgc ttggtcggtc atttcgctcg gtacccatcg gcattttctt ttgcgttt 7438
<210> 22
   <211> 4980
   <212> DNA
   <213> Artificial
<220>
   <223> plasmid pCLIK int sacB dapA codon low
<400> 22
   acgcgtgcgg tagaatgttt aagaaagctg caggtagcag cgccaactgt tttcggtgat 60
   tttgagattg aaactttggc agacggatcg caaatggcaa caagcccgta tgtcatggac 120
   ttttaacgca aagctcacac ccacgagcta aaaattcata tagttaagac aacatttttg 180
   gctgtaaaag acagccgtaa aaacctcttg ctcgtgtcaa ttgttcttat cggaatgtgg 240
   cttgggcgat tgttatgcaa aagttgttag gttttttgcg gggttgttta acccccaaat 300
   gagggaagaa ggtaaccttg aactctatga gcacagggtt aacagctaag acaggggtag 360
   agcacttcgg gaccgttggg gtagcaatgg ttactccatt cacggaatcc ggagacatcg 420
   atatcgctgc tggccgcgaa gtcgcggctt atttggttga taagggcttg gattctttgg 480
   ttctcgcggg caccactggt gaatccccaa cgacaaccgc cgctgaaaaa ctagaactgc 540
   tcaaggccgt tcgtgaggaa gttggggatc gggcgaagct catcgccggt gtcggaacca 600
   acaacacgcg gacatctgtg gaacttgcgg aagctgctgc ttctgctggc gcagacggcc 660
   ttttagttgt aactccttat tactccaagc cggtcgacat cgatgctctt ctgcgttaat 720
   taacaattgg gatcctctag acccgggatt taaatcgcta gcgggctgct aaaggaagcg 780
   gaacacgtag aaagccagtc cgcagaaacg gtgctgaccc cggatgaatg tcagctactg 840
   ggctatctgg acaagggaaa acgcaagcgc aaagagaaag caggtagctt gcagtgggct 900
   tacatggcga tagctagact gggcggtttt atggacagca agcgaaccgg aattgccagc 960
   tggggcgccc tctggtaagg ttgggaagcc ctgcaaagta aactggatgg ctttcttgcc 1020
   gccaaggatc tgatggcgca ggggatcaag atctgatcaa gagacaggat gaggatcgtt 1080
   tcgcatgatt gaacaagatg gattgcacgc aggttctccg gccgcttggg tggagaggct 1140
   attcggctat gactgggcac aacagacaat cggctgctct gatgccgccg tgttccggct 1200
   gtcagcgcag gggcgcccgg ttctttttgt caagaccgac ctgtccggtg ccctgaatga 1260
   actgcaggac gaggcagcgc ggctatcgtg gctggccacg acgggcgttc cttgcgcagc 1320
   tgtgctcgac gttgtcactg aagcgggaag ggactggctg ctattgggcg aagtgccggg 1380
   gcaggatctc ctgtcatctc accttgctcc tgccgagaaa gtatccatca tggctgatgc 1440
   aatgcggcgg ctgcatacgc ttgatccggc tacctgccca ttcgaccacc aagcgaaaca 1500
   tcgcatcgag cgagcacgta ctcggatgga agccggtctt gtcgatcagg atgatctgga 1560
   cgaagagcat caggggctcg cgccagccga actgttcgcc aggctcaagg cgcgcatgcc 1620
   cgacggcgag gatctcgtcg tgacccatgg cgatgcctgc ttgccgaata tcatggtgga 1680
   aaatggccgc ttttctggat tcatcgactg tggccggctg ggtgtggcgg accgctatca 1740
   ggacatagcg ttggctaccc gtgatattgc tgaagagctt ggcggcgaat gggctgaccg 1800
   cttcctcgtg ctttacggta tcgccgctcc cgattcgcag cgcatcgcct tctatcgcct 1860
   tcttgacgag ttcttctgag cgggactctg gggttcgaaa tgaccgacca agcgacgccc 1920
   aacctgccat cacgagattt cgattccacc gccgccttct atgaaaggtt gggcttcgga 1980
   atcgttttcc gggacgccgg ctggatgatc ctccagcgcg gggatctcat gctggagttc 2040
   ttcgcccacg ctagcggcgc gccggccggc ccggtgtgaa ataccgcaca gatgcgtaag 2100
   gagaaaatac cgcatcaggc gctcttccgc ttcctcgctc actgactcgc tgcgctcggt 2160
   cgttcggctg cggcgagcgg tatcagctca ctcaaaggcg gtaatacggt tatccacaga 2220
   atcaggggat aacgcaggaa agaacatgtg agcaaaaggc cagcaaaagg ccaggaaccg 2280
   taaaaaggcc gcgttgctgg cgtttttcca taggctccgc ccccctgacg agcatcacaa 2340
   aaatcgacgc tcaagtcaga ggtggcgaaa cccgacagga ctataaagat accaggcgtt    2400
   tccccctgga agctccctcg tgcgctctcc tgttccgacc ctgccgctta ccggatacct    2460
   gtccgccttt ctcccttcgg gaagcgtggc gctttctcat agctcacgct gtaggtatct    2520
   cagttcggtg taggtcgttc gctccaagct gggctgtgtg cacgaacccc ccgttcagcc    2580
   cgaccgctgc gccttatccg gtaactatcg tcttgagtcc aacccggtaa gacacgactt    2640
   atcgccactg gcagcagcca ctggtaacag gattagcaga gcgaggtatg taggcggtgc    2700
   tacagagttc ttgaagtggt ggcctaacta cggctacact agaaggacag tatttggtat    2760
   ctgcgctctg ctgaagccag ttaccttcgg aaaaagagtt ggtagctctt gatccggcaa    2820
   acaaaccacc gctggtagcg gtggtttttt tgtttgcaag cagcagatta cgcgcagaaa    2880
   aaaaggatct caagaagatc ctttgatctt ttctacgggg tctgacgctc agtggaacga    2940
   aaactcacgt taagggattt tggtcatgag attatcaaaa aggatcttca cctagatcct    3000
   tttaaaggcc ggccgcggcc gccatcggca ttttcttttg cgtttttatt tgttaactgt    3060
   taattgtcct tgttcaagga tgctgtcttt gacaacagat gttttcttgc ctttgatgtt    3120
   cagcaggaag ctcggcgcaa acgttgattg tttgtctgcg tagaatcctc tgtttgtcat    3180
   atagcttgta atcacgacat tgtttccttt cgcttgaggt acagcgaagt gtgagtaagt    3240
   aaaggttaca tcgttaggat caagatccat ttttaacaca aggccagttt tgttcagcgg    3300
   cttgtatggg ccagttaaag aattagaaac ataaccaagc atgtaaatat cgttagacgt    3360
   aatgccgtca atcgtcattt ttgatccgcg ggagtcagtg aacaggtacc atttgccgtt    3420
   cattttaaag acgttcgcgc gttcaatttc atctgttact gtgttagatg caatcagcgg    3480
   tttcatcact tttttcagtg tgtaatcatc gtttagctca atcataccga gagcgccgtt    3540
   tgctaactca gccgtgcgtt ttttatcgct ttgcagaagt ttttgacttt cttgacggaa    3600
   gaatgatgtg cttttgccat agtatgcttt gttaaataaa gattcttcgc cttggtagcc    3660
   atcttcagtt ccagtgtttg cttcaaatac taagtatttg tggcctttat cttctacgta    3720
   gtgaggatct ctcagcgtat ggttgtcgcc tgagctgtag ttgccttcat cgatgaactg    3780
   ctgtacattt tgatacgttt ttccgtcacc gtcaaagatt gatttataat cctctacacc    3840
   gttgatgttc aaagagctgt ctgatgctga tacgttaact tgtgcagttg tcagtgtttg    3900
   tttgccgtaa tgtttaccgg agaaatcagt gtagaataaa cggatttttc cgtcagatgt    3960
   aaatgtggct gaacctgacc attcttgtgt ttggtctttt aggatagaat catttgcatc    4020
   gaatttgtcg ctgtctttaa agacgcggcc agcgtttttc cagctgtcaa tagaagtttc    4080
   gccgactttt tgatagaaca tgtaaatcga tgtgtcatcc gcatttttag gatctccggc    4140
   taatgcaaag acgatgtggt agccgtgata gtttgcgaca gtgccgtcag cgttttgtaa    4200
   tggccagctg tcccaaacgt ccaggccttt tgcagaagag atatttttaa ttgtggacga    4260
   atcaaattca gaaacttgat atttttcatt tttttgctgt tcagggattt gcagcatatc    4320
   atggcgtgta atatgggaaa tgccgtatgt ttccttatat ggcttttggt tcgtttcttt    4380
   cgcaaacgct tgagttgcgc ctcctgccag cagtgcggta gtaaaggtta atactgttgc   4440
   ttgttttgca aactttttga tgttcatcgt tcatgtctcc ttttttatgt actgtgttag    4500
   cggtctgctt cttccagccc tcctgtttga agatggcaag ttagttacgc acaataaaaa   4560
   aagacctaaa atatgtaagg ggtgacgcca aagtatacac tttgcccttt acacatttta   4620
   ggtcttgcct gctttatcag taacaaaccc gcgcgattta cttttcgacc tcattctatt   4680
   agactctcgt ttggattgca actggtctat tttcctcttt tgtttgatag aaaatcataa   4740
   aaggatttgc agactacggg cctaaagaac taaaaaatct atctgtttct tttcattctc   4800
   tgtatttttt atagtttctg ttgcatgggc ataaagttgc ctttttaatc acaattcaga   4860
   aaatatcata atatctcatt tcactaaata atagtgaacg gcaggtatat gtgatgggtt   4920
   aaaaaggatc ggcggccgct cgatttaaat ctcgagaggc ctgacgtcgg gcccggtacc   4980